(19) Europäisches Patentamt / European Patent Office / Office européen des brevets

(11) **EP 4 337 780 B1**

(12) **EUROPEAN PATENT SPECIFICATION**

(45) Date of publication and mention
of the grant of the patent:
**01.04.2026 Bulletin 2026/14**

(21) Application number: **22729147.3**

(22) Date of filing: **12.05.2022**

(51) International Patent Classification (IPC):
*C12N 15/86* (2006.01)    *C12N 15/861* (2006.01)
*A61K 48/00* (2006.01)

(52) Cooperative Patent Classification (CPC):
**A61K 48/005; A61K 48/0058; C12N 9/1276;**
**C12N 15/86; C12Y 207/07049;** C12N 2750/14143;
C12N 2830/008

(86) International application number:
**PCT/EP2022/062990**

(87) International publication number:
**WO 2022/238557 (17.11.2022 Gazette 2022/46)**

(54) **RECOMBINANT TERT-ENCODING VIRAL GENOMES AND VECTORS**

REKOMBINANTE TERT-CODIERENDE VIRALE GENOME UND VEKTOREN

GÉNOMES ET VECTEURS VIRAUX RECOMBINANTS À ENCODAGE TERT

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **12.05.2021 EP 21382441**

(43) Date of publication of application:
**20.03.2024 Bulletin 2024/12**

(73) Proprietors:
• **Fundación del Sector Público Estatal Centro**
 **Nacional de Investigaciones Oncológicas Carlos**
 **III**
 **(F.S.P. CNIO)**
 **28029 Madrid (ES)**
• **Universitat Autònoma de Barcelona**
 **08193 Cerdanyola del Vallès (ES)**
• **Telomere Therapeutics SL**
 **08007 Barcelona (ES)**

(72) Inventors:
• **BLASCO, María**
 **28029 Madrid (ES)**
• **MARTINEZ, Paula**
 **28029 Madrid (ES)**
• **BOSCH TUBERT, Maria Fàtima**
 **08193 Cerdanyola del Vallès (Barcelona) (ES)**
• **JIMENEZ CENZANO, Verónica**
 **08193 Cerdanyola del Vallès (Barcelona) (ES)**
• **GARCIA MARTINEZ, Miquel**
 **08193 Cerdanyola del Vallès (Barcelona) (ES)**
• **CASANA LORENTE, Estefanía**
 **08193 Cerdanyola del Vallès (Barcelona) (ES)**

(74) Representative: **Hoffmann Eitle**
 **Hoffmann Eitle S.L.U.**
 **Paseo de la Castellana 140, 3a planta**
 **Edificio LIMA**
 **28046 Madrid (ES)**

(56) References cited:
**EP-A1- 2 402 038**    **US-A1- 2001 016 352**
**US-A1- 2014 088 175**

Remarks:
The complete document including Reference
Table(s) and the Sequence Listing(s) can be
downloaded from the EPO website

## Description

### Field

Aspects and embodiments described herein relate to the field of medicine, particularly gene therapy.

### Background

Telomeres are specialized structures at the ends of chromosomes, which have a role in protecting the chromosome ends from DNA repair and degrading activities (Blackburn et al. Cell 2001; 106(6):661-673). Mammalian telomeres consist of TTAGGG repeats bound by a multi-protein complex known as shelterin. The presence of a minimum length of TTAGGG repeats and the integrity of the shelterin complex are necessary for telomere protection. When telomeres become critically short, they lose their protective function and a persistent DNA damage response at the telomeres is triggered which subsequently leads to a cellular senescence response.

Telomerase is a cellular reverse transcriptase (TERT, telomerase reverse transcriptase; also known as TP2; TRT; EST2; TCSI) capable of compensating telomere attrition through de novo addition of TTAGGG repeats onto the chromosome ends by using an associated ncRNA component as template (Terc, telomerase RNA component) (Greider & Blackburn. Cell 1985; 43:405-413). Telomerase is expressed in most adult stem cell compartments; however, in most somatic cells telomerase is silenced after birth leading to the gradual shortening of telomeres. This is evidenced by the fact that telomere shortening occurs with age in most human and mouse tissues (Blasco, Nat Chem Biol. 2007; 3:640-649).

Shortened telomeres have been associated with numerous diseases, such as dyskeratosis congenita, aplastic anaemia, myelodysplastic syndrome, Fanconi anaemia, pulmonary fibrosis and cardiovascular diseases (CVD). Given the severity of these diseases and the poor prognosis of the patients suffering from them, there is a need for improved therapies to treat diseases associated with shortened telomere length.

Therapeutic intervention with telomerase, aimed at preventing telomere loss beyond a critically short length, represents a potential treatment for conditions associated with shortened telomeres. AAV-mediated TERT gene therapy such as using AAV9 vectors encoding telomerase under the control of the CMV promoter (AAV9-CMV-mTert), was previously shown to be effective in extending health span of mice (Bernardes de Jesus et al. EMBO Mol Med 2012; 4:691-704) and treating conditions associated with shortened telomere length such as aplastic anemia and pulmonary fibrosis (WO2016/20345, Beier et al. Blood 2012; 120(15):2990-3000, Povedano et al. eLife 2018; 7: e31299) and cardiovascular diseases (WO2016/020346, Bär et al. Nat Comm 2014; 5:5863), Chatterjee et al. Mol Ther 2021; 29(4): 1-16), demonstrated amelioration of doxorubicin toxicity in mice hearts and in human cardiomyocytes utilizing AAV9 vectors encoding telomerase under the control of the chicken troponin promoter. Existing AAV vectors are associated with drawbacks which affect their effectiveness in TERT gene therapy, such as low gene expression in target tissues, off-target gene expression in tissues other than the ones being treated and the occurrence of intermediate vector species containing truncated viral genomes. In view of the above, there is a need for the provision of improved recombinant viral genomes and vectors for gene therapy utilizing TERT.

US 2001/0016352 relates to an oligonucleotide which includes at least one nucleic acid sequence which binds to at least one nuclear protein found in lung cells, such as TTF-1.

US 2014/0088175 relates to methods of transcytosing barrier epithelial cells using adeno-associated virus-4 (AAV4), adeno-associated virus-5 (AAV5), adeno-associated virus-7 (AAV7), bovine adeno-associated virus (BAAV), and vectors and particles derived therefrom.

EP 2402038 relates to expression vectors of the telomerase reverse transcriptase, especially non integrative vectors such as those derived from adeno-associated viruses (AAV), preferably derived from AAV9, as well as to its use for the amelioration of markers of ageing such as insulin insensitivity, osteoporosis, loss of neuromuscular coordination, loss of memory, and the longevity itself.

However, none of said documents describe the subject matter claimed herein.

### Summary

The invention is as defined in the claims.

### Description

The present invention provides recombinant adeno-associated viral genomes and vectors useful in gene therapy utilizing telomerase reverse transcriptase (TERT). Recombinant viral genomes and vectors described herein exhibit at least one, at least two, at least three, or all of the following benefits over known viral genomes and vectors:

- Increased TERT expression in target tissues
- Decreased TERT expression in off-target tissues
- Prevention of formation of intermediate species comprising truncated viral genomes associated with the utilization of viral vectors with low encapsidation capacity
- Increased therapeutic efficacy

As also demonstrated in the Examples section herein, a significant efficacy improvement over existing therapeutic strategies is expected from the application of the recombinant viral genomes and vectors of the invention in gene therapy utilizing TERT. Accordingly, the aspects and embodiments of the present invention as described herein solve at least some of the problems and needs as discussed herein.

Recombinant viral genome

In a first aspect, there is provided a recombinant adeno-associated viral genome comprising a nucleotide sequence encoding a telomerase reverse transcriptase (TERT) operably linked to a tissue-specific promoter, wherein the total length of the viral genome is less than 4700 nucleotides, and wherein the tissue-specific promoter consists of SEQ ID NOs: 12.

A viral genome may consist of DNA or RNA molecules. Said molecules can be single or double stranded. Said molecules can be linear, circular, segmented (composed of multiple pieces of nucleic acids) or nonsegmented. Viral genome ends may comprise repeated sequences, chemical modifications and/or secondary structures, which may have regulatory functions such as assisting with viral genome replication in the respective host. In the context of the invention, any adeno-associated viral genome may be contemplated as long as it allows for expression of its comprised TERT-encoding nucleotide sequence upon introduction of said genome in a cell.

The recombinant adeno-associated viral genome originates from an adeno-associated virus (AAV). Accordingly, there is provided a recombinant AAV genome comprising a nucleotide sequence encoding a telomerase reverse transcriptase (TERT) operably linked to a tissue-specific and/or organ-specific promoter consisting of SEQ ID NO: 12, wherein the total length of the viral genome is less than 4700 nucleotides.

The term "adeno-associated viral genome" (AAV genome) refers to a genome of a virus that belongs to the genus *Dependoparvovirus* (also referred to as *Dependovirus*) of the family *Parvoviridae*. The naturally occurring (wild-type) AAV genome is approximately 4.7 kb in length. The genome includes inverted terminal repeats (ITRs) at both ends of the DNA strand. A "recombinant", alternatively referred to as "chimeric" or "engineered", viral genome is a viral genome not normally found in nature (wild-type viral genome), such as a viral genome wherein native regulatory sequences and/or transcribed DNA regions have been removed, modified or otherwise juxtaposed in a manner not naturally occurring, and/or wherein non-native regulatory sequences and/or transcribed DNA regions have been incorporated.

The recombinant adeno-associated viral genome according to the invention optionally comprises inverted terminal repeats (ITRs) originating from an AAV genome. Said sequences may enhance the maintenance of the recombinant adeno-associated viral genome in a respective host. Inverted terminal repeats may be included at the 5' and the 3' flanks of the recombinant adeno-associated viral genome (5' ITR and 3' ITR, respectively). Suitable inverted terminal repeat sequences may be derived from any adeno-associated virus serotype, such as but not limited to AAV1, AAV2, AAV3, AAV4, AAV5, and others. Preferred ITRs are those of AAV2 which are represented by sequences comprising, consisting essentially of, or consisting of SEQ ID NO: 19 (5' ITR) and SEQ ID NO: 20 (3' ITR). The invention also preferably encompasses the use of a sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with SEQ ID NO: 19 as 5' ITR and a sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with SEQ ID NO: 20 as 3' ITR.

The viral genome is a recombinant adeno-associated viral genome (AAV genome). Said genome may or may not comprise its natively associated inverted terminal repeats (ITRs). Preferably, said genome comprises the ITRs of adeno-associated virus serotype 2 (AAV2), more preferably it comprises SEQ ID NO: 19 (5' ITR) and SEQ ID NO: 20 (3' ITR).

A recombinant adeno-associated viral genome according to the invention has a total length of less than 4700 nucleotides. The present inventors have surprisingly found that not exceeding the abovementioned length allows for the prevention of the formation of intermediate species comprising truncated viral genomes when viruses with low encapsidation capacity, such as but not limited to adeno-associated viruses, are utilized as expression vectors, resulting in improved therapeutic efficacy of said vectors. In some embodiments, the recombinant adeno-associated viral genome has a total length of less than 4680 nucleotides, less than 4660 nucleotides, less than 4640 nucleotides, less than 4620 nucleotides, less than 4600 nucleotides, less than 4580 nucleotides, less than 4560 nucleotides, less than 4540 nucleotides, less than 4520 nucleotides, or less than 4500 nucleotides, preferably less than 4680 nucleotides.

In some embodiments, the recombinant adeno-associated viral genome has a length of 4400-4700 nucleotides, 4420-4700 nucleotides, 4440-4700 nucleotides, 4460-4700 nucleotides, 4480-4700 nucleotides, 4500-4700 nucleotides, 4520-4700 nucleotides, 4540-4700 nucleotides, 4560-4700 nucleotides, 4580-4700 nucleotides, 4600-4700 nucleotides, 4620-4700 nucleotides, 4640-4700 nucleotides, 4660-4700 nucleotides, or 4680-4700 nucleotides, preferably it has a length of 4400-4700 nucleotides.

In some embodiments, the recombinant adeno-associated viral genome has a length of 4400-4700 +/- 10 nucleotides, 4420-4700 +/- 10 nucleotides, 4440-4700 +/- 10 nucleotides, 4460-4700 +/- 10 nucleotides, 4480-4700 +/- 10 nucleotides, 4500-4700 +/- 10 nucleotides, 4520-4700 +/- 10 nucleotides, 4540-4700 +/- 10 nucleotides, 4560-4700 +/- 10 nucleotides, 4580-4700 +/- 10 nucleotides, 4600-4700 +/- 10 nucleotides, 4620-4700 +/- 10 nucleotides, 4640-4700 +/- 10 nucleotides, 4660-4700 +/- 10 nucleotides, or 4680-4700 +/- 10 nucleotides, preferably it has a length of 4400-4700 +/- 10 nucleotides.

A "telomerase reverse transcriptase" (TERT), alternatively known as TP2, TRT, EST2, or TCSI (EC 2.7.7.49) is a catalytic component of the telomerase holoenzyme complex, whose main activity is the elongation of telomeres by its acting as a reverse transcriptase that adds simple sequence repeats to chromosome ends by copying a template sequence within the ncRNA component of the enzyme (Terc, telomerase RNA component). TERT catalyzes the RNA-dependent extension of 3'-chromosomal termini with the 6-nucleotide telomeric repeat unit 5'-TTAGGG-3'. The skilled person understands that the term also encompasses modified versions of TERT, such as but not limited to TERT fragments, as long as said modified versions remain functional. In this context, functionality refers to the modified protein exhibiting at least 20%, at least 30%, at least 40%, at least 50%, at least 60%, at least 70%, at least 80%, at least 90%, or at least 95%, of the catalytic activity as compared to the unmodified protein. A modified version of TERT may also exhibit increased functionality as compared to the unmodified protein. In this context, increased functionality refers to the modified protein exhibiting at least 105%, at least 110%, at least 120%, at least 130%, at least 140%, at least 150%, at least 160%, at least 170%, at least 180%, at least 190%, or at least 200% of the catalytic activity as compared to the unmodified protein. Catalytic activity of TERT may be measured according to standard methods in the art as discussed elsewhere herein.

A nucleotide sequence encoding a TERT according to the invention preferably comprises, essentially consists of, or consists of, the coding sequence (CDS) of a TERT gene. "Coding sequence" or "coding region" refers to the part of a gene that codes for a protein. Said nucleotide sequence may be modified or non-modified compared to a naturally-occurring, alternatively referred to as "wild-type", sequence. Suitable non-limiting modifications may be selected from nucleotide insertions, deletions, mutations and/or substitutions. The skilled person understands that modifications resulting in the expression of modified versions of TERT as discussed above are also encompassed. The nucleotide sequence encoding a TERT may or may not comprise non-coding regions of a TERT gene, such as introns and/or 5' and/or 3' untranslated regions, preferably it does not comprise non-coding regions. Accordingly, in some embodiments the nucleotide sequence encoding a TERT comprises, essentially consists of, or consists of coding regions of a TERT gene. In some embodiments, the nucleotide sequence encoding a TERT consists of the coding sequence of a TERT gene. In some embodiments, a preferred nucleotide sequence encoding a TERT does not comprise the natively associated 3' UTR region of a TERT gene.

Modification of a nucleotide sequence may be performed using any recombinant DNA technique as known in the art, such as for example described in standard handbooks like Ausubel et al., Current Protocols in Molecular Biology, 3rd edition (2003), John Wiley & Sons Inc and in Sambrook and Green, Molecular Cloning. A Laboratory Manual, 4th Edition (2012), Cold Spring Harbor Laboratory Press; both of which are incorporated herein by reference in their entirety. Also see, Kunkel (1985) Proc. Natl. Acad. Sci. 82:488 (describing site directed mutagenesis) and Roberts et al. (1987) Nature 328:731-734 or Wells, J.A., et al. (1985) Gene 34: 315 (describing cassette mutagenesis).

A nucleotide sequence encoding a TERT that is comprised in a recombinant adeno-associated viral genome according to the invention may or may not correspond to a nucleotide sequence that is native to a cell wherein the recombinant adeno-associated viral genome comprising said gene is to be introduced in. Said nucleotide sequence may be derived from any TERT-encoding sequence. In some embodiments, the nucleotide sequence encoding a TERT is of mammalian origin. In some embodiments, the nucleotide sequence encoding a TERT is of murine, leporid, swine, equine, sheep, bovine, feline, canine, or human origin. In humans, two TERT isoforms exist which differ in length; a long isoform (NCBI CCDS ID: 3861.2) and a short isoform (NCBI CCDS ID:54831.1). In some embodiments, the nucleotide sequence encoding a TERT is of murine (such as mouse or rat) or human origin, preferably of human origin, more preferably it is the long or short isoform of human TERT or a modified version thereof.

Modification of a nucleotide sequence encoding TERT may comprise codon optimization. Accordingly, in some embodiments, the nucleotide sequence encoding a TERT is codon optimized, preferably for expression in a human cell or a mouse cell, preferably a human cell. A description of "codon optimization" is provided later herein in the section entitled "general information".

In some embodiments, a preferred nucleotide sequence encoding a TERT comprises, essentially consists of, or consists of a nucleotide sequence encoding a polypeptide represented by the amino acid sequence of SEQ ID NO: 4, or an amino acid sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity or similarity with SEQ ID NO: 4. SEQ ID NO: 4 represents an amino acid sequence of mouse TERT (Uniprot accession number: 070372-1).

In some embodiments, a preferred nucleotide sequence encoding a TERT comprises, essentially consists of, or consists of a nucleotide sequence encoding a polypeptide represented by the amino acid sequence of SEQ ID NOs: 7 or 10, or an amino acid sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity or similarity with SEQ ID NO: 7 or 10. SEQ ID NO: 7 represents an amino acid sequence of the long isoform of human TERT (Uniprot accession number: 014746-1). SEQ ID NO: 10 represents an amino acid sequence of the short isoform of human TERT (Uniprot accession number: 014746-3). In some embodiments, a preferred nucleotide sequence encoding a TERT comprises, essentially consists of, or consists of a nucleotide sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with any one of SEQ ID NOs: 1, 2, 3, 5, 6, 8, or 9, preferably with any one of SEQ ID NOs: 3, 6, or 9, more preferably with SEQ ID NO: 6 or 9. Accordingly, in preferred embodiments the recombinant adeno-associated viral genome according to the invention comprises a nucleotide sequence encoding a telomerase reverse transcriptase (TERT), wherein:

a) the TERT encoding sequence comprises, essentially consists of, or consists of a nucleotide sequence encoding a polypeptide represented by the amino acid sequence of any one of SEQ ID NOs: 4, 7, or 10, preferably SEQ ID NO: 7 or 10, or an amino acid sequence having at least 60%, 70%, 80%, 90%, 95%, or 99% identity or similarity with any one of SEQ ID NOs: 4, 7, or 10, preferably with SEQ ID NO: 7 or 10, or;

b) the TERT encoding sequence comprises, essentially consists of, or consists of a nucleotide sequence having at least 60%, 70%, 80%, 90%, 95%, or 99% identity with any one of SEQ ID NOs: 1, 2, 3, 5, 6, 8, or 9, preferably with any one of SEQ ID NOs: 3, 6, or 9, more preferably with SEQ ID NO: 6 or 9, or;

c) the TERT encoding sequence comprises, essentially consists of, or consists of a nucleotide sequence the sequence of which differs from the sequence of a nucleotide sequence of a) or b) due to the degeneracy of the genetic code.

The nucleotide sequence encoding a telomerase reverse transcriptase (TERT) according to the invention is operably linked to a tissue-specific and/or an organ-specific promoter consisting of SEQ ID NO: 12. A description of "operably linked" is provided later herein in the section entitled "general information". Throughout this disclosure, the terms "promoter" and "promoter sequence" are used interchangeably and may be replaced by "transcription regulatory sequence" or "regulatory sequence". Definitions of the terms are provided in the "general information" section.

A "tissue-specific" or "organ-specific" promoter is a promoter that is active in a specific type of tissue or organ, respectively. Said promoter may be constitutive or inducible. Definitions of the terms are provided in the "general information" section. The term also encompasses promoters that are active in specific cells of said tissues or organs (cell-specific promoters). In this context, the term "promoter activity" refers to the capability of a promoter to initiate transcription of a coding nucleotide sequence operably linked to said promoter. Tissue-specific and organ-specific promoters regulate expression of one or more genes primarily in one tissue or organ and/or in specific cells of a tissue or organ. In some embodiments, they may still allow detectable level ("leaky") expression in other tissues or organs. Leaky expression in other tissues or organs means at least one-fold, at least two-fold, at least three-fold, at least fourfold or at least five-fold lower, but still detectable expression as compared to the tissue-specific or organ-specific expression, as evaluated on the level of the mRNA or the protein by standard assays known to a person of skill in the art (e.g. qPCR, Western blot analysis, ELISA). Assessment of the tissue-specific and/or organ-specific nature of a promoter can be performed by standard molecular toolbox techniques, such as, for example, described in Sambrook and Green (supra). As a non-limiting example, any expression vector comprising any of the recombinant adeno-associated viral genomes as described herein, wherein the nucleotide sequence encoding a TERT has been replaced by a nucleotide sequence encoding a GFP, can be produced. Cells, tissues and organs transduced as described herein can then be assessed for expression based on RNA or protein levels or immunostaining and/or microscopy methods (e.g. fluorescence microscopy, light microscopy, bright-field microscopy, and the like) according to standard protocols, for example as described in the Examples section of this disclosure.

As demonstrated in the Examples, the present inventors have surprisingly found that the tissue- and organ-specific promoters described herein allow for increased gene expression in target tissues and decreased gene expression in off-target tissues.

The tissue-specific and/or organ-specific promoter according to the invention is a lung-specific promoter and consists of SEQ ID NO: 12.

A "lung-specific promoter" is a promoter that is capable of initiating transcription in the lung and/or a lung tissue and/or a lung cell. Said promoter may or may not still allow leaky expression in other organs and parts of the body. "Lung-specific" also encompasses promoters that drive the preferential or predominant expression of a nucleotide sequence in the lung and/or a lung tissue and/or a lung cell as compared to other organs, tissues or cells as described later herein. Transcription in the lung can be detected in relevant areas, such as the trachea, bronchi, pleura, diaphragm, bronchioles, alveoli or epithelium. Promoters that are capable of initiating transcription in cells of the lung epithelium are advantageous. Promoters that are capable of initiating transcription in alveolar type II epithelial cells (ATII cells) are particularly advantageous. Accordingly, the lung-specific promoter is an alveolar type II epithelial cell specific (ATII-cell specific) promoter consisting of SEQ ID NO: 12.

A "heart-specific promoter", alternatively known as "cardiac-specific promoter', is a promoter that is capable of initiating transcription in the heart and/or a heart tissue and/or a heart cell. Said promoter may or may not still allow leaky expression in other organs and parts of the body. "Heart-specific" also encompasses promoters that drive the preferential or predominant expression of a nucleotide sequence in the heart and/or a heart tissue and/or a heart cell as compared to other organs, tissues or cells as described later herein. Transcription in the heart can be detected in relevant areas, such as the tricuspid valve, pulmonary valve, mitral valve, aortic valve, left atrium, right atrium, left ventricle, right ventricle, epithelium or myocardium. Promoters that are capable of initiating transcription in cells of the myocardium are advantageous. Promoters that are capable of initiating transcription in cardiac myocytes are particularly advantageous.

In the context of the invention, tissue-specific and/or organ-specific promoters may be promoters that are capable of driving the preferential or predominant (at least 10% higher, at least 20% higher, at least 30% higher, at least 40% higher, at least 50% higher, at least 60% higher, at least 70% higher, at least 80% higher, at least 90% higher, at least 100% higher, at least 150% higher, at least 200% higher or more) expression of a nucleotide sequence in the specific tissues and/or organs as compared to other tissues or organs. Other organs or tissues may be the liver, CNS, brain, adipose tissue (white and/or brown), skeletal muscle, heart, kidney, colon, hematopoietic tissue, lung, ovary, spleen, stomach, pancreas, testis, epididymis, intestine, and others. Preferably, other organs or tissues are the liver and adipose tissue (white and/or brown).

A lung-specific promoter is a promoter that is capable of driving the preferential or predominant expression of a nucleotide sequence in the lungs as compared to one or more tissues and/or organs selected from the brain, adipose tissue (white and/or brown), heart and/or liver, preferably brain, brown adipose tissue and/or heart. A heart-specific promoter may preferably be a promoter that is capable of driving the preferential or predominant expression of a nucleotide sequence in the heart as compared to one or more tissues and/or organs selected from the adipose tissue (white and/or brown), liver, intestine, testis and/or skeletal muscle. Expression may be assessed as described in the "general information" section.

The lung-specific promoter is a shortened version of the human SpB promoter, and it consists of SEQ ID NO: 12.

A recombinant adeno-associated viral genome according to the invention may further comprise additional nucleotide sequences that are operably linked to the nucleotide sequence encoding TERT, such as, but not limited to, signal sequences, nuclear localization signals, expression enhancers, polyadenylation signals, kozak sequences and the like. Said sequences may or may not correspond to sequences that are native to a cell wherein the recombinant adeno-associated viral genome comprising said promoter is to be introduced in.

An "enhancer" is a sequence that can stimulate the transcription of the sequence it is operably linked to. Generally, DNA sequences that are operably linked are contiguous, and, in the case of a signal sequence, both contiguous and sharing a reading frame. However, enhancers need not be contiguous with a coding sequence whose transcription they control. Linking is accomplished by ligation at convenient restriction sites or at adapters or linkers inserted in lieu thereof, or by gene synthesis. Enhancers may be used as single sequences or may be comprised in a fusion nucleotide sequence with other enhancers and/or any of the tissue-specific and/or organ-specific promoters described herein. Enhancer-promoter fusions may be particularly advantageous in enhancing transcription-driving capability when shortened promoter derivatives, as compared to naturally-occurring promoters, are utilized to drive expression of a nucleotide sequence encoding a TERT.

In some embodiments, the recombinant adeno-associated viral genome of the invention comprises a CMV enhancer (SEQ ID NO: 14).

A kozak sequence, alternatively referred to herein as a kozak consensus sequence, is a nucleic acid motif that functions as the protein translation initiation site and can enhance the expression of nucleotide sequences it is operably linked to. In some embodiments, a recombinant viral adeno-associated genome according to the invention comprises a kozak consensus sequence operably linked to the nucleotide sequence encoding a TERT. Preferably, said kozak consensus sequence is of human origin. In some embodiments, a preferred kozak consensus sequence comprises, essentially consists of, or consists of a nucleotide sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with SEQ ID NO: 17.

A polyadenylation sequence, alternatively referred to herein as a "polyA sequence" or "polyA tail", is a nucleotide sequence consisting of a stretch of multiple adenosine monophosphates that can enhance the expression of nucleotide sequences it is operably linked to by enhancing nuclear export, translation and/or stability of mRNA sequences. In some embodiments, a recombinant adeno-associated viral genome according to the invention comprises a polyA sequence operably linked to the nucleotide sequence encoding a TERT. Preferably, said polyA sequence is selected from the SV40 polyA sequence, the rabbit β-globin polyA sequence, or the bovine growth hormone polyA sequence, more preferably it is the bovine growth hormone polyA sequence. In some embodiments, a preferred polyA sequence comprises, essentially consists of, or consists of a nucleotide sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with SEQ ID NO: 18.

The recombinant adeno-associated viral genome is a recombinant AAV genome comprising a nucleotide sequence encoding a telomerase reverse transcriptase (TERT) operably linked to a lung-specific promoter consisting of SEQ ID NO: 12, wherein the total length of the viral genome is less than 4700 nucleotides. Optionally, the gene construct further includes 5' and 3' flanks of inverted terminal repeats (ITRs) derived from the genome of an AAV, preferably from AAV2. In some embodiments, such a recombinant adeno-associated viral genome comprises, essentially consists of, or consists of, the nucleotide sequence of SEQ ID NO: 21, or a sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity therewith.

Expression vector

A recombinant adeno-associated viral genome as described herein can be placed in expression vectors.

The expression vector comprising a recombinant adeno-associated viral genome, is an adeno-associated viral vector. An adeno-associated viral vector may alternatively be referred to herein as an adeno-associated vector or adeno-associated virus derived vector or AAV vector. A description of "adeno-associated viral vector" has been provided under the section entitled "general information". Said vectors typically have a nucleotide sequence encapsidation capacity of approximately 4700 nucleotides (4.7 kb). As demonstrated in the Examples, the present inventors have surprisingly found that the recombinant adeno-associated viral genome of the invention exhibits significant unexpected advantages when AAV vectors are utilized as expression vectors, as the formation of intermediate species containing truncated viral genomes is prevented and the therapeutic efficacy of said vectors is increased. A preferred AAV vector may be selected from the list consisting of AAV of serotype 1 (AAV1), AAV of serotype 2 (AAV2), AAV of serotype 3 (AAV3), AAV of serotype 4 (AAV4), AAV of serotype 5 (AAV5), AAV of serotype 6 (AAV6), AAV of serotype 7 (AAV7), AAV of serotype 8 (AAV8), AAV of serotype 9 (AAV9), AAV of serotype rh10 (AAVrh10), AAV of serotype rh8 (AAVrh8), AAV of serotype Cb4 (AAVCb4), AAV of serotype rh74 (AAVrh74), AAV of serotype DJ (AAVDJ), AAV of serotype 2/5 (AAV2/5), AAV of serotype 2/1 (AAV2/1), AAV of serotype 1/2 (AAV1/2) and AAV of serotype Anc80 (AAVAnc80), preferably it is selected from the list consisting of AAV of serotype 6, 8, or 9 (AAV6, AAV8, or AAV9), more preferably it is selected from AAV of serotype 6 (AAV6) or 9 (AAV9), even more preferably it is of serotype 9 (AAV9)..

In some embodiments, a preferred expression vector is an AAV6 or AAV9, preferably an AAV9, and comprises a recombinant AAV genome comprising a nucleotide sequence encoding a telomerase reverse transcriptase (TERT) operably linked to a lung-specific promoter, wherein the total length of the viral genome is less than 4700 nucleotides as described earlier herein. Optionally, the recombinant AAV genome further includes 5' and 3' flanks of inverted terminal repeats (ITRs) derived from the genome of an AAV, preferably from AAV2. Said expression vector preferably comprises a nucleotide sequence encoding a TERT that comprises, essentially consists of, or consists of a nucleotide sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with any one of SEQ ID NOs:1, 2, 3, 5, 6, 8, or 9,, preferably with any one of SEQ ID NOs: 3, 6, or 9, more preferably with SEQ ID NO: 6 or 9.

In some embodiments, a preferred expression vector is an AAV6 or AAV9 and comprises a recombinant AAV viral genome comprising a nucleotide sequence encoding a telomerase reverse transcriptase (TERT) operably linked to a SpB promoter consisting of SEQ ID NO: 12, wherein the total length of the viral genome is less than 4700 nucleotides as described earlier herein.. Optionally, the recombinant AAV viral genome further includes 5' and 3' flanks of inverted terminal repeats (ITRs) derived from the genome of an AAV, preferably from AAV2. Said expression vector preferably comprises a nucleotide sequence encoding a TERT that comprises, essentially consists of, or consists of a nucleotide sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with any one of SEQ ID NOs: 1, 2, 3, 5, 6, 8, or 9, preferably with any one of SEQ ID NOs: 3, 6, or 9, more preferably with SEQ ID NO: 6 or 9.

The production of a recombinant AAV vector (also referred to as "rAAV") for vectorizing nucleotide sequences has been described previously, see Ayuso E, et al., Curr. Gene Ther. 2010; 10:423-436, Okada T, et al., Hum. Gene Ther. 2009; 20:1013-1021, Zhang H, et al., Hum. Gene Ther. 2009; 20:922-929, and Virag T, et al., Hum. Gene Ther. 2009;

20:807-817; all of which incorporated herein by reference in their entirety. These protocols can be used or adapted to generate an AAV vector according to the invention.

In short, the methods generally involve (a) the introduction of the recombinant AAV genome comprising the nucleotide sequence to be expressed into a cell, (b) the presence or introduction of an AAV helper construct in the cell, wherein the helper construct comprises the viral functions missing from the recombinant AAV genome and, optionally, (c) the introduction of a helper virus and/or helper virus plasmid into the host cell. All components for AAV vector replication and packaging need to be present, to achieve replication and packaging of the genome into AAV vectors. These typically include AAV cap proteins, AAV rep proteins and, optionally, viral proteins upon which AAV is dependent for replication. Rep and cap regions are well known in the art, see e.g. Chiorini et al. (1999, J. of Virology, Vol 73(2): 1309-1319, incorporated herein by reference in its entirety) or US 5,139,941 (incorporated herein by reference in its entirety). The AAV cap and rep proteins may derive from the same AAV serotype or they can derive from a combination of different serotypes, preferably they derive from an AAV6 or AAV9 serotype. The viral proteins upon which AAV is dependent for replication may derive from any virus, such as a herpes simplex viruses (such as HSV types 1 and 2), a vaccinia virus, an adeno-associated virus or an adenovirus, preferably from an adenovirus.

In some embodiments, the producer cell line is transfected transiently with a recombinant AAV genome according to the invention (comprising the TERT expression cassette flanked by ITRs) and with construct(s) that encode(s) rep and cap proteins and provide(s) helper functions (helper construct(s)). In some embodiments, the cell line supplies stably the helper functions and is transfected transiently with the recombinant AAV genome according to the invention (comprising the TERT expression cassette flanked by ITRs) and with construct(s) that encode(s) rep and cap proteins. In some embodiments, the cell line supplies stably the rep and cap proteins and the helper functions and is transiently transfected with the recombinant AAV genome according to the invention. In another embodiment, the cell line supplies stably the rep and cap proteins and is transfected transiently with the recombinant AAV viral genome according to the invention and a polynucleotide encoding the helper functions. In some embodiments, the cell line supplies stably the recombinant AAV genome according to the invention, the rep and cap proteins and the helper functions. Methods of making and using these and other AAV production systems have been described in the art. See Muzyczka N, et al., US 5,139,941, Zhou X, et al., US 5,741,683, Samulski R, et al., US 6,057,152, Samulski R, et al., US 6,204,059, Samulski R, et al., US 6,268,213, Rabinowitz J, et al., US 6,491,907, Zolotukhin S, et al., US 6,660,514, Shenk T, et al., US 6,951,753, Snyder R, et al., US 7,094,604, Rabinowitz J, et al., US 7,172,893, Monahan P, et al., US 7,201,898, Samulski R, et al., US 7,229,823, and Ferrari F, et al., US 7,439,065, all of which are incorporated herein by reference in their entirety.

The recombinant AAV (rAAV) genome present in a rAAV vector typically comprises at least the nucleotide sequences of the inverted terminal repeat regions (ITRs) of one of the AAV serotypes (preferably the ones of serotype AAV2 as disclosed herein), or nucleotide sequences substantially identical thereto or nucleotide sequences having at least 60%, 70%, 80%, 90%, 95% or 99% identity thereto, and a nucleotide sequence encoding a TERT operably linked to a tissue-specific and/or organ-specific promoter consisting of SEQ ID NO: 12 as described herein, inserted between the two ITRs. A vector genome generally requires the use of flanking 5' and a 3' ITR sequences to allow for efficient packaging of the vector genome into the rAAV capsid. Said ITR sequences do not necessarily have to be derived from the same AAV serotype as the rAAV vector.

The complete genome of several AAV serotypes and corresponding ITRs has been sequenced (Chiorini et al. 1999, J. of Virology Vol. 73, No.2, p1309-1319, incorporated herein by reference in its entirety). They can be either cloned or made by chemical synthesis as known in the art, using for example an oligonucleotide synthesizer as supplied e.g. by Applied Biosystems Inc. (Fosters, CA, USA) or by standard molecular biology techniques. The ITRs can be cloned from an AAV genome or excised from a vector comprising the AAV ITRs. The ITR nucleotide sequences can be either ligated at either end of a nucleotide sequence encoding a TERT operably linked to a tissue-specific and/or organ-specific promoter as described herein using standard molecular biology techniques, or alternatively the AAV sequence between the ITRs can be replaced with the desired nucleotide sequence.

Preferably, the rAAV genome as present in a rAAV vector does not comprise any nucleotide sequences encoding viral proteins, such as the rep (replication) or cap (capsid) genes of AAV. This rAAV genome may further comprise a marker or reporter gene, such as a gene for example encoding an antibiotic resistance gene, a fluorescent protein (e.g. GFP) or a gene encoding a chemically, enzymatically or otherwise detectable and/or selectable product (e.g. lacZ, aph, etc.) known in the art.

The introduction into a producer cell can be carried out using standard virological techniques, such as transformation, transduction and transfection. Most vectors do not replicate in the producer cells infected with the vector. Examples of workable combinations of cell lines and expression vectors are described in Sambrook and Green (supra), and in Metzger et al (1988) Nature 334: 31-36 (incorporated herein by reference in its entirety). For example, suitable expression vectors can be expressed in, yeast, e.g. *S. cerevisiae,* e.g., insect cells, e.g., Sf9 cells, mammalian cells, e.g., CHO cells and bacterial cells, e.g., *E. coli.* A cell may thus be a prokaryotic or eukaryotic producer cell. A cell may be a cell that is suitable for culture in liquid or on solid media. Finally, the producer cells are cultured under standard conditions known in the art to produce the assembled AAV vectors which are then purified using standard techniques such as polyethylene glycol precipitation or CsCl gradients (Xiao et al. 1996, J. Virol. 70: 8098-8108, incorporated herein by reference in its entirety). Residual helper virus activity can be inactivated using known methods, such as for example heat inactivation.

The recombinant adeno-associated viral genomes and expression vectors as described herein may then be introduced into a host cell using standard molecular techniques, as discussed in standard handbooks such as Current Protocols in Molecular Biology (Ausubel et al., supra) and Sambrook and Green (supra). In the case of viral vectors, transduction is preferably used. The transduced host cell may or may not comprise the protein shell of the viral vectors. "Host cell" or "target cell", alternatively referred to as "cell" or "engineered cell", refers to the cell into which the DNA delivery takes place, such as the lung or heart cells of a mammalian subject as described elsewhere herein. AAV vectors in particular are able to transduce both dividing and non-dividing cells.

Accordingly, the disclosure further provides a host cell transduced with any of the recombinant viral genomes or expression vectors described herein. In some embodiments, a host cell transduced with any of the recombinant viral genomes or expression vectors described herein is lung cell, such as a lung cell of a vertebrate, preferably a lung cell of a mammal, more preferably an alveolar type II epithelial cell (ATII cell), of a mammal. In some embodiments, a host cell transduced with any of the recombinant viral genomes or expression vectors described herein is a lung cell, preferably an alveolar type II epithelial cell (ATII cell), of a rat, mouse, dog or a human, preferably of a mouse or a human, more preferably a human.

The provided host cells need not necessarily be present in an individual. The skilled person understands that introduction of the recombinant viral genomes and expression vectors as described herein may be performed in cell cultures. In some embodiments, the provided host cells are present in an artificial organ, preferably an artificial lung or heart. In some embodiments, the provided host cells are present in an organoid, preferably a lung or heart organoid. An "organoid" as defined herein is a miniaturized and simplified version of an organ produced in vitro in three dimensions that shows realistic micro-anatomy. The skilled person is able to arrive at such artificial organs and/or organoids using the host cells of the invention by applying generally known procedures in the art. The transduced host cells present in an artificial organ and/or organoid may be implanted to a vertebrate, preferably a mammal, more preferably a mouse, rat, dog or human, more preferably a mouse or human, most preferably a human, using generally known procedures in the art.

Composition

In a further aspect there is provided a pharmaceutical composition comprising a recombinant adeno-associated viral genome as described herein and/or an adeno-associated viral vector, as described herein, optionally further comprising one or more pharmaceutically acceptable ingredients. Such a composition may be called a gene therapy composition. A composition such as a pharmaceutical composition may be formulated to be suitable for a particular mode of administration. Suitable administration modes are described elsewhere herein. The types of formulations, as well as formulation methods, that will be the most appropriate for a composition as described herein for it to be suitable for a particular mode of administration are well within the capabilities of the skilled person and are described in standard handbooks, such as Remington: The Science and Practice of Pharmacy, 23rd edition, Elsevier (2020), incorporated herein by reference in its entirety.

As used herein, "pharmaceutically acceptable ingredients" include pharmaceutically acceptable carriers, fillers, stabilizers, preservatives, solubilizers, vehicles, diluents and/or excipients. Accordingly, the one or more pharmaceutically acceptable ingredients may be selected from the group consisting of pharmaceutically acceptable carriers, fillers, stabilizers, preservatives, solubilizers, vehicles, diluents and/or excipients. Such pharmaceutically acceptable carriers, fillers, stabilizers, preservatives, solubilizers, vehicles, diluents and/or excipients may for instance be found in Remington: The Science and Practice of Pharmacy (supra).

A further compound may be present in a composition of the invention. Said compound may help in delivery of the composition. Examples of suitable compounds in this context are: compounds capable of forming complexes, nanoparticles, micelles and/or liposomes that deliver each constituent as described herein, complexed or trapped in a vesicle or liposome through a cell membrane. Many of these compounds are known in the art. Suitable compounds may comprise polyethylenimine (PEI), or similar cationic polymers, including polypropyleneimine or polyethylenimine copolymers (PECs) and derivatives; synthetic amphiphiles (SAINT-18); lipofectin™, DOTAP. Additional examples of suitable compounds are inert carriers (such as calcium carbonate and sugars, for example sucrose, mannitol, lactose, or dextrose, and the like), solvents (such as water, saline, and the like), and propellant gases (such as chlorofluorocarbon (CFC), fluorocarban (FC), or hydrofluroalkane (HFA, and the like).

In some embodiments, a pharmaceutical composition is formulated to be suitable for a specific mode of administration of a recombinant adeno-associated viral genome as described herein and/or an adeno-associated viral vector, as described herein, such as, but not limited to, intranasal, intratracheal, intrapulmonary administration or administration via inhalation. Non-limiting examples of exemplary formulations are aerosols, solutions and/or suspensions of particles in carriers which are suitable for respiratory tract and/or lung delivery, and dry powders.

In some embodiments, a pharmaceutical composition, is comprised within a delivery device. In some embodiments, a composition, preferably a pharmaceutical composition is comprised within a delivery device suitable for delivery via inhalation (an inhalation device). Such devices are commonly known in the art, many being commercially available, and include inhalers (such as fixed dose inhalers, metered dose inhalers, dry powder inhalers, and the like) and nebulizers (such as jet nebulisers, ultrasonic nebulisers, mesh nebulisers, and the like). Nebulizers typically operate by aerosol particle generation. Exemplary nebulizers for delivering an aerosolized composition as described herein include the AERx™ (Aradigm), the Ultravent® (Mallinkrodt), the Pari LC Plus™ or the Pari LC Star™ (Pari GmbH, Germany), the DeVilbiss Pulmo-Aide, and the Acorn II® (Marquest Medical Products).

Method and use

Also provided herein are recombinant adeno-associated viral genomes, adeno-associated viral vectors and compositions as described herein for use in therapy. In some embodiments, recombinant adeno-associated viral genomes, adeno-associated viral vectors and compositions as described herein are for use as a medicament.

The recombinant adeno-associated viral genomes, adeno-associated viral vectors and compositions described herein are particularly effective in treating and/or preventing conditions associated with shortened telomere length. A definition of "shortened telomere length" has been provided in the section entitled "general information". Accordingly, in a further aspect, the recombinant adeno-associated viral genomes, adeno-associated viral vectors and/or compositions described herein are provided for use in the treatment and/or prevention of a condition associated with shortened telomere length. In some embodiments, the recombinant adeno-associated viral genomes, adeno-associated viral vectors and/or compositions described herein are provided for use in the treatment and/or prevention of a lung condition associated with shortened telomere length. Non-limiting examples of such conditions are pulmonary fibrosis, chronic obstructive pulmonary disease (COPD), emphysema syndrome and conditions associated therewith. In preferred embodiments, the recombinant adeno-associated viral genomes adeno-associated viral vectors and/or compositions described herein are provided for use in the treatment and/or prevention of pulmonary fibrosis.

Pulmonary fibrosis refers to a condition characterised by scarring of the lung tissue. Pulmonary fibrosis can be caused by many factors, including, but not limited to, lung cell telomere shortening, chronic inflammatory processes, infections, environmental compounds, ionizing radiation (for example radiation therapy to treat tumours of the chest), genetic predisposition, or chronic medical conditions (lupus, rheumatoid arthritis and others). Pulmonary fibrosis includes idiopathic pulmonary fibrosis (IPF), which refers to pulmonary fibrosis without an identifiable cause. Symptoms of pulmonary fibrosis include, but are not limited to, lung scarring, development of fibrotic volume, loss of lung volume, shortness of breath, dry cough, fatigue, weight loss, and nail clubbing. Conditions associated with pulmonary fibrosis include, but are not limited to, pulmonary hypertension, respiratory failure, pneumothorax, and lung cancer. In some embodiments, pulmonary fibrosis may be idiopathic pulmonary fibrosis (IPF).

Myocardial infarction refers to a condition characterized by tissue death (infarction) of the heart muscle (myocardium). Myocardial infarction can be caused by many factors, including, but not limited to, heart cell telomere shortening, atherosclerosis, high blood pressure (hypertension), hypercholesterolemia, diabetes, coronary heart disease, smoking, obesity, physical inactivity, environmental compounds, infections, genetic predisposition, or chronic inflammatory and other medical conditions. Symptoms of myocardial infarction include, but are not limited to, heart scarring, heart failure, an irregular heartbeat, fatigue, heart pain, cardiogenic shock or cardiac arrest. Conditions associated with myocardial infarction include, but are not limited to, myocardial infarction events (heart attack), tissue damage resulting from myocardial infarction (including loss of cardiomyocytes), fibrosis of the myocardium resulting from myocardial infarction, and reduced cardiac function resulting from myocardial infarction.

In some embodiments, recombinant adeno-associated viral genomes comprising a nucleotide sequence encoding a telomerase reverse transcriptase (TERT) operably linked to a lung-specific promoter consisting of SEQ ID NO. 12, adeno-associated viral vectors comprising said genomes and/or compositions comprising said genomes and/or vectors as described herein are provided for use in the treatment and/or prevention of a lung condition associated with shortened telomere length, preferably of pulmonary fibrosis or a condition associated therewith.

As used herein, an "effective amount" is an amount sufficient to exert beneficial or desired results. Accordingly, a "therapeutically effective amount" is an amount that, when administered to a subject in need thereof, is sufficient to exert some therapeutic effect as described herein, such as, but not limited to, a reduction in the magnitude of at least one symptom and/or the improvement of at least one parameter of a condition associated with shortened telomere length, preferably of pulmonary fibrosis, myocardial infarction or a condition associated therewith as described elsewhere herein. An amount that is "therapeutically effective" will vary from subject to subject, depending on the age, the disease progression and overall general condition of the individual. An appropriate "therapeutically effective" amount in any individual case may be determined by the skilled person using routine experimentation, such as the methods described later herein, and/or the methods of the experimental part herein. A "subject in need" may be any individual affected by, and/or at risk of developing, a condition associated with shortened telomere length

In a further aspect there is provided a use of a recombinant adeno-associated viral genome, an adeno-associated viral vector or a composition as described herein, for the manufacture of a medicament for the treatment and/or prevention of a condition associated with shortened telomere length. In some embodiments, the condition associated with shortened telomere length is a lung condition, preferably it is pulmonary fibrosis or a condition associated therewith.

In some embodiments, there is provided a use of a recombinant adeno-associated viral genome comprising a nucleotide sequence encoding a telomerase reverse transcriptase (TERT) operably linked to a lung-specific promoter consisting of SEQ ID NO: 12, an adeno-associated viral vector comprising said genome and/or a composition comprising said genome and/or vector as described herein for the manufacture of a medicament for the treatment and/or prevention of a lung condition associated with shortened telomere length, preferably of pulmonary fibrosis or a condition associated therewith.

In a further aspect there is provided a use of a recombinant adeno-associated viral genome, an adeno-associated viral vector, or a composition as described herein, for the treatment and/or prevention of a condition associated with shortened telomere length. In some embodiments, the condition associated with shortened telomere length is a lung condition, preferably it is pulmonary fibrosis or a condition associated therewith.

In some embodiments, there is provided a use of a recombinant adeno-associated viral genome comprising a nucleotide sequence encoding a telomerase reverse transcriptase (TERT) operably linked to a lung-specific promoter consisting of SEQ ID NO: 12, an expression adeno-associated viral vector comprising said genome and/or a composition comprising said genome and/or vector as described herein for the treatment and/or prevention of a lung condition associated with shortened telomere length, preferably of pulmonary fibrosis or a condition associated therewith.

Within the context of recombinant adeno-associated viral genome for use, adeno-associated viral vectors for use, compositions for use, methods and uses according to the invention, the therapy and/or medicament may involve expression, preferably specific expression, of TERT in the lungs. A description of expression has been provided in the section entitled "general information". In some embodiments, expression of TERT does not involve expression in at least one, at least two, at least three, at least four organs selected from the group consisting of liver, CNS, brain, adipose tissue (white and/or brown), skeletal muscle, heart, kidney, colon, hematopoietic tissue, lung, ovary, spleen, stomach, pancreas, testis, epididymis, intestine, and others.

In preferred embodiments, a treatment or a therapy or a use or the administration of a medicament as described herein does not have to be repeated. In some embodiments, a treatment or a therapy or a use or the administration of a medicament as described herein may be repeated each year or each 2, 3, 4, 5, 6, 7, 8, 9 or 10 years, including intervals between any two of the listed values.

The subject treated may be a vertebrate, preferably a mammal such as a cat, a mouse, a rat, a dog, or a human. In preferred embodiments, the subject treated is a human. Treatment administration may be performed to an individual, a cell, tissue, and/or an organ of an individual affected and/or at risk of developing a condition associated with shortened telomere length, preferably pulmonary fibrosis, myocardial infarction or conditions associated therewith. Treatment administration may be performed directly or indirectly *in vivo, ex vivo* or *in vitro,* using suitable means known in the art. Improvements in means for providing an individual or a cell, tissue, and/or organ of said individual with a recombinant viral genome and/or an expression vector (preferably an adeno-associated viral vector) and/or a composition of the invention are anticipated, considering the progress that has already thus far been achieved. Such future improvements may of course be incorporated to achieve the mentioned effect of the invention. Depending on the disease or condition, a cell, tissue and/or organ of said individual may be as earlier described herein. When administering a recombinant viral genome and/or an expression vector, preferably an adeno-associated viral vector, and/or a composition of the invention, it is preferred that such gene construct and/or an expression vector and/or a composition is dissolved in a solution that is compatible with the delivery method. Such solutions are generally known in the art, see for example Remington: The Science and Practice of Pharmacy (supra).

Treatment administration may be performed using different administration modes which are generally known in the art. An administration mode may be intravenous, intranasal, intramuscular, intraperitoneal, via inhalation, intraparenchymal, subcutaneous, intraarticular, intra-adipose tissue, oral, intrahepatic, intrasplanchnic, intra-ear, intrathoracic, intrapulmonary, intracardial, transendocardial or intratracheal administration. Preferred administration modes for lung-specific expression are via inhalation, intranasal, intratracheal, intrapulmonary, and intravenous administration. "Intratracheal administration" or "intratracheal instillation" refers to direct administration into the trachea. "Intravenous administration" refers to direct administration into a vein, typically by injection. A preferred administration mode for heart-specific administration is intracardial, transendocardial or intravenous administration. "Intracardial administration" refers to direct administration into the heart muscle or ventricles "Transendocardial administration" refers to direct administration into the endocardium.

In some embodiments, a treatment or a therapy or a use or the administration of a medicament for a treatment as described herein exhibit at least one, at least two, at least three, or all of the following benefits over known treatments, therapies, uses or administration of medicaments:

- Increased TERT expression in target tissues
- Decreased TERT expression in off-target tissues
- Prevention of formation of intermediate species comprising truncated viral genomes associated with the utilization of viral vectors with low encapsidation capacity
- Increased therapeutic efficacy

In some embodiments, a treatment or a therapy or a use or the administration of a medicament for a treatment as described herein alleviates, removes, prevents and/or reverses shortening of telomeres.

"Shortened telomeres" or "short telomeres" generally refers to telomeres below a certain length, e.g. 8 kb, 7 kb, 6kb, 5kb, or shorter. The skilled person understands that telomere length depends not only on whether an individual is healthy, but also on the individual's age. Typically, shortened telomeres have a length that is below the 20th or 10th percentile of the telomere length of a population of healthy individuals belonging to a certain age group, which indicates the telomere length below which 20% or 10% of the telomeres fall. Conversely, long telomeres typically have a length that is above the 80th or 90th percentile of the telomere length of a population of healthy individuals belonging to a certain age group. Telomere length may be assessed in samples taken directed from a treated individual, or a cell, tissue, and/or organ of a treated individual, according to generally known methods in the art. For example, standard hybridization techniques, such as fluorescence *in situ* hybridization (FISH), quantitative fluorescent *in situ* hybridization (Q-FISH), or high throughput quantitative fluorescent *in situ* hybridization (HT Q-FISH) as described in Gonzalez-Suarez et al. (2001) EMBO J 20(11): 2619-30, incorporated by reference herein in its entirety, may be used. Alternatively, telomere length may be measured as described in any one of the disclosures of WO2016/020346, Canela et al. (2007) Methods Mol Biol 371: 45-72, or Bär et al. (2018) Nat Comm 5: 5863, incorporated by reference herein in their entireties. Telomere length may also be inferred by monitoring the deterioration of or the improvement in a symptom and/or a parameter of a condition associated with shortened telomere length as discussed elsewhere herein using standard procedures in the art, as for example provided in the Examples section herein.

Samples may be taken throughout the course of the treatment so that both absolute telomere length and the rate of telomere lengthening or shortening over the course of treatment can be determined. Samples may be taken every day during the course of treatment, or at longer intervals. In some embodiments, samples are taken once a week, once every two weeks, once every three weeks, once every four weeks, once every five weeks, once every six weeks or at longer intervals. Comparisons of telomere length can be made by comparing the proportion of shortened telomeres in the taken samples. The proportion of shortened telomeres may be assessed by measuring the fraction of telomeres presenting an intensity below the mean intensity of the sample as measured by an *in situ* hybridization technique, such as FISH or Q-FISH. In some embodiments, the proportion of shortened telomeres is the fraction of telomeres presenting an intensity 75%, 70%, 65%, 60%, 55%, 50%, 45%, 40% or lower as compared to the mean intensity of the sample. In some embodiments, the proportion of shortened telomeres in a sample taken directed from a treated individual, or a cell, tissue, and/or organ of a treated individual, is decreased by 10%, 20%, 30%, 40%, 50%, 60%, 70%, or greater as compared to a control sample. A control sample may be taken from the individual to be treated, or a cell, tissue, and/or organ of an individual to be treated prior to commencement of the treatment and/or from an untreated individual suffering from the same condition, or a cell, tissue, and/or organ of an untreated individual suffering from the same condition.

In some embodiments, a treatment or a therapy or a use or the administration of a medicament for the treatment of a pulmonary fibrosis or a condition associated therewith alleviates, removes and/or prevents a symptom and/or improves a parameter associated therewith selected from the group consisting of lung scarring, development of fibrotic volume, loss of lung volume, shortness of breath, dry cough, fatigue, weight loss, and nail clubbing, preferably development of fibrotic volume and/or loss of lung volume.

Alleviating a symptom of a condition as discussed herein may mean that said symptom is improved or decreased or that the progression of a typical symptom has been slowed down in an individual, in a cell, tissue or organ of said individual as assessed by a physician. A decrease or improvement of a typical symptom may mean a slowdown in progression of symptom development or a complete disappearance of symptoms. Symptoms, and thus also a decrease in symptoms, can be assessed using a variety of methods, to a large extent the same methods as used in diagnosis of the relevant conditions, including clinical examination and routine laboratory tests. Laboratory tests may include both macroscopic and microscopic methods, molecular methods, radiographic methods such as X-rays, CT-scans, spirometry, biochemical methods, immunohistochemical methods and others. In this context, "decrease" (respectively "improvement") means at least a detectable decrease (respectively a detectable improvement) using an assay known to a person of skill in the art, such as assays as carried out in the experimental part. The decrease may be a decrease of at least 5%, at least 10%, at least 20%, at least 30%, at least 40%, at least 50%, at least 60%, at least 70%, at least 80%, at least 90% or at least 100%. The decrease may be seen after at least one week, one month, six months, one year or more of treatment using a recombinant viral genome and/or an expression vector (preferably an adeno-associated viral vector) and/or a composition of the invention. Preferably, the decrease is observed after a single administration. In some embodiments, the decrease is observed for a duration of at least one week, one month, six months, 1 year, 2 years, 3 years, 4 years, 5 years, 6 years, 7 years, 8 years, 9 years, 10 years, 12 years, 15 years, 20 years or more, preferably after a single administration.

Improving a parameter may mean that the value of a typical parameter associated with the relevant disease (e.g. lung volume in the case of pulmonary fibrosis) is improved in an individual, in a cell, tissue or organ of said individual as assessed by a physician. In this context, improvement of a parameter may be interpreted as to mean that said parameter assumes a value closer to the value displayed by a healthy individual. The improvement of a parameter may be seen after at least one week, one month, six months, one year or more of treatment using a recombinant viral vector and/or an expression vector, preferably an adeno-associated viral vector, and/or a composition of the invention. Preferably, the improvement is observed after a single administration. In some embodiments, the improvement is observed for a duration of at least one week, one month, six months, 1 year, 2 years, 3 years, 4 years, 5 years, 6 years, 7 years, 8 years, 9 years, 10 years, 12 years, 15 years, 20 years or more, preferably after a single administration.

In some embodiments, a treatment or a therapy or a use or the administration of a medicament for treatment as described herein results in an increase in overall physical fitness of a treated individual as compared to the same individual prior to commencement of the treatment or an untreated individual suffering from the same condition. Overall physical fitness may be determined by evaluating and/or measuring physical attributes associated with the relevant condition as assessed by a physician and an increase in overall physical fitness may mean that at least one such attribute is improved. Examples of physical attributes include dry cough, fatigue, and others.

In some embodiments, a treatment or a therapy or a use or the administration of a medicament for treatment as described herein results in an increase in the lifespan of a treated individual as compared to an untreated individual suffering from the same condition. The extension of said lifespan may depend on the relevant condition to be treated and preferably is 5%, 10%, 15%, 20% or more.

General information

Unless stated otherwise, all technical and scientific terms used herein have the same meaning as customarily and ordinarily understood by a person of ordinary skill in the art to which this invention belongs, and read in view of this disclosure.

*Lung and heart*

The terms "lung" and "heart" as used herein refer to the organs of the respiratory and cardiovascular system, respectively, as customarily and ordinarily understood by the skilled person. "Epithelial" cell refers to a cell of the epithelial tissue, as customarily and ordinarily understood by the skilled person. "Alveolar epithelial" refers to cell of the epithelial tissue of the alveoli, as customarily and ordinarily understood by the skilled person. A "myocardial cell" refers to a cell of the myocardium, as customarily and ordinarily understood by the skilled person.

*Sequence identity*

In the context of the invention, a nucleotide sequence such as a nucleotide sequence encoding a TERT is represented by a nucleic acid or nucleotide sequence which encodes a protein fragment or a polypeptide or a peptide or a derived peptide. In the context of the invention, a TERT, a TERT fragment, fragment or a polypeptide or a peptide or a derived peptide is represented by an amino acid sequence.

It is to be understood that each nucleic acid molecule or protein fragment or polypeptide or peptide or derived peptide or construct as identified herein by a given sequence identity number (SEQ ID NO) is not limited to this specific sequence as disclosed. Each coding sequence as identified herein encodes a given protein fragment or polypeptide or peptide or derived peptide or construct or is itself a protein fragment or polypeptide or construct or peptide or derived peptide.

Throughout this application, each time one refers to a specific nucleotide sequence SEQ ID NO (take SEQ ID NO: X as example) encoding a given protein fragment or polypeptide or peptide or derived peptide, one may replace it by:

i. a nucleotide sequence comprising a nucleotide sequence that has at least 60%, 70%, 80%, 90%, 95% or 99% sequence identity with SEQ ID NO: X;

ii. a nucleotide sequence the sequence of which differs from the sequence of a nucleic acid molecule of (i) due to the degeneracy of the genetic code; or

iii. a nucleotide sequence that encodes an amino acid sequence that has at least 60%, 70%, 80%, 90%, 95% or 99% amino acid identity or similarity with an amino acid sequence encoded by a nucleotide sequence SEQ ID NO: X.

Another preferred level of sequence identity or similarity is 70%. Another preferred level of sequence identity or similarity is 80%. Another preferred level of sequence identity or similarity is 90%. Another preferred level of sequence identity or similarity is 95%. Another preferred level of sequence identity or similarity is 99%.

Throughout this application, each time one refers to a specific amino acid sequence SEQ ID NO (take SEQ ID NO: Y as example), one may replace it by: a polypeptide represented by an amino acid sequence comprising a sequence that has at least 60%, 70%, 80%, 90%, 95% or 99% sequence identity or similarity with amino acid sequence SEQ ID NO: Y. Another preferred level of sequence identity or similarity is 70%. Another preferred level of sequence identity or similarity is 80%. Another preferred level of sequence identity or similarity is 90%. Another preferred level of sequence identity or similarity is 95%. Another preferred level of sequence identity or similarity is 99%.

Each nucleotide sequence or amino acid sequence described herein by virtue of its identity or similarity percentage with a given nucleotide sequence or amino acid sequence respectively has in a further preferred embodiment an identity or a similarity of at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% with the given nucleotide or amino acid sequence, respectively.

Each non-coding nucleotide sequence (i.e. of a promoter or of another regulatory region) could be replaced by a nucleotide sequence comprising a nucleotide sequence that has at least 60% sequence identity or similarity with a specific nucleotide sequence SEQ ID NO (take SEQ ID NO: A as example). A preferred nucleotide sequence has at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO: A. In a preferred embodiment, such non-coding nucleotide sequence such as a promoter exhibits or exerts at least an activity of such a non-coding nucleotide sequence such as an activity of a promoter as known to a person of skill in the art.

The terms "homology", "sequence identity" and the like are used interchangeably herein. Sequence identity is described herein as a relationship between two or more amino acid (polypeptide or protein) sequences or two or more nucleic acid (polynucleotide) sequences, as determined by comparing the sequences. In a preferred embodiment, sequence identity is calculated based on the full length of two given SEQ ID NO's or on a part thereof. Part thereof preferably means at least 50%, 60%, 70%, 80%, 90%, or 100% of both SEQ ID NO's. In the art, "identity" also refers to the degree of sequence relatedness between amino acid or nucleic acid sequences, as the case may be, as determined by the match between strings of such sequences. "Similarity" between two amino acid sequences is determined by comparing the amino acid sequence and its conserved amino acid substitutes of one polypeptide to the sequence of a second polypeptide. "Identity" and "similarity" can be readily calculated by known methods, including but not limited to those described in Bioinformatics and the Cell: Modern Computational Approaches in Genomics, Proteomics and transcriptomics, Xia X., Springer International Publishing, New York, 2018; and Bioinformatics: Sequence and Genome Analysis, Mount D., Cold Spring Harbor Laboratory Press, New York, 2004, each incorporated by reference herein in its entirety.

"Sequence identity" and "sequence similarity" can be determined by alignment of two peptide or two nucleotide sequences using global or local alignment algorithms, depending on the length of the two sequences. Sequences of similar lengths are preferably aligned using a global alignment algorithm (e.g. Needleman-Wunsch) which aligns the sequences optimally over the entire length, while sequences of substantially different lengths are preferably aligned using a local alignment algorithm (e.g. Smith-Waterman). Sequences may then be referred to as "substantially identical" or "essentially similar" when they (when optimally aligned by for example the program EMBOSS needle or EMBOSS water using default parameters) share at least a certain minimal percentage of sequence identity (as described below).

A global alignment is suitably used to determine sequence identity when the two sequences have similar lengths. When sequences have a substantially different overall length, local alignments, such as those using the Smith-Waterman algorithm, are preferred. EMBOSS needle uses the Needleman-Wunsch global alignment algorithm to align two sequences over their entire length (full length), maximizing the number of matches and minimizing the number of gaps. EMBOSS water uses the Smith-Waterman local alignment algorithm. Generally, the EMBOSS needle and EMBOSS water default parameters are used, with a gap open penalty = 10 (nucleotide sequences) / 10 (proteins) and gap extension penalty = 0.5 (nucleotide sequences) / 0.5 (proteins). For nucleotide sequences the default scoring matrix used is DNAfull and for proteins the default scoring matrix is Blosum62 (Henikoff & Henikoff, 1992, PNAS 89, 915-919, incorporated herein by reference in its entirety).

Alternatively, percentage similarity or identity may be determined by searching against public databases, using algorithms such as FASTA, BLAST, etc. Thus, the nucleic acid and protein sequences of some embodiments of the present invention can further be used as a "query sequence" to perform a search against public databases to, for example, identify other family members or related sequences. Such searches can be performed using the BLASTn and BLASTx programs (version 2.0) of Altschul, et al. (1990) J. Mol. Biol. 215:403-10, incorporated herein by reference in its entirety. BLAST nucleotide searches can be performed with the NBLAST program, score = 100, wordlength = 12 to obtain nucleotide sequences homologous to oxidoreductase nucleic acid molecules of the invention. BLAST protein searches can be performed with the BLASTx program, score = 50, wordlength = 3 to obtain amino acid sequences homologous to protein molecules of the invention. To obtain gapped alignments for comparison purposes, Gapped BLAST can be utilized as described in Altschul et al., (1997) Nucleic Acids Res. 25(17): 3389-3402, incorporated herein by reference in its entirety. When utilizing BLAST and Gapped BLAST programs, the default parameters of the respective programs (e.g., BLASTx and BLASTn) can be used. See the homepage of the National Center for Biotechnology Information accessible on the world wide web at www.ncbi.nlm.nih.gov/. Optionally, in determining the degree of amino acid similarity, the skilled person may also take into account so-called conservative amino acid substitutions. As used herein, "conservative" amino acid substitutions refer to the interchangeability of residues having similar side chains. Examples of classes of amino acid residues for conservative substitutions are given in the Tables below (incorporated herein as Table 1).

Table 1.

| Acidic Residues | Asp (D) and Glu (E) |
|---|---|
| Basic Residues | Lys (K), Arg (R), and His (H) |
| Hydrophilic Uncharged Residues | Ser (S), Thr (T), Asn (N), and Gln (Q) |
| Aliphatic Uncharged Residues | Gly (G), Ala (A), Val (V), Leu (L), and Ile (I) |
| Non-polar Uncharged Residues | Cys (C), Met (M), and Pro (P) |
| Aromatic Residues | Phe (F), Tyr (Y), and Trp (W) |

Alternative conservative amino acid residue substitution classes :

| 1 | A | S | T |
|---|---|---|---|
| 2 | D | E | |
| 3 | N | Q | |
| 4 | R | K | |
| 5 | I | L | M |
| 6 | F | Y | W |

Alternative physical and functional classifications of amino acid residues:

| Alcohol group-containing residues | S and T |
|---|---|
| Aliphatic residues | I, L, V, and M |
| Cycloalkenyl-associated residues | F, H, W, and Y |
| Hydrophobic residues | A, C, F, G, H, I, L, M, R, T, V, W, and Y |
| Negatively charged residues | D and E |
| Polar residues | C, D, E, H, K, N, Q, R, S, and T |
| Positively charged residues | H, K, and R |
| Small residues | A, C, D, G, N, P, S, T, and V |
| Very small residues | A, G, and S |
| Residues involved in turn formation | A, C, D, E, G, H, K, N, Q, R, S, P and T |
| Flexible residues | Q, T, K, S, G, P, D, E, and R |

For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to Ser; Arg to Lys; Asn to Gln or His; Asp to Glu; Cys to Ser or Ala; Gln to Asn; Glu to Asp; Gly to Pro; His to Asn or Gln; Ile to Leu or Val; Leu to Ile or Val; Lys to Arg; Gln or Glu; Met to Leu or Ile; Phe to Met, Leu or Tyr; Ser to Thr; Thr to Ser; Trp to Tyr; Tyr to Trp or Phe; and, Val to Ile or Leu.

*Gene or coding nucleotide sequence*

The term "gene" means a DNA fragment comprising a region (transcribed region), which is transcribed into an RNA molecule (e.g. an mRNA) in a cell, operably linked to suitable regulatory regions (e.g. a promoter). A gene will usually comprise several operably linked fragments, such as a promoter, a 5' leader sequence, a coding region and a 3'-untranslated region (3'-end) e.g. comprising a polyadenylation- and/or transcription termination site. A chimeric or recombinant gene (such as a TERT gene) is a gene not normally found in nature, such as a gene in which for example the promoter is not associated in nature with part or all of the transcribed DNA region. "Expression of a gene" refers to the process wherein a DNA region which is operably linked to appropriate regulatory regions, particularly a promoter, is transcribed into an RNA, which is biologically active, i.e. which is capable of being translated into a biologically active protein or peptide.

In the context of the invention, a TERT gene may normally not be expressed or expressed at an insufficient level in a cell. In this context, "insufficient" means that although said TERT gene is expressed in a cell, a condition and/or disease as described herein could still be developed. In this case, the invention allows the over-expression of TERT. Coding nucleotide sequences may comprise sequences that are native to the cell, sequences that naturally do not occur in the cell and it may comprise combinations of both. A gene may contain sequences encoding a TERT and/or additional proteins as earlier identified herein that may be operably linked to appropriate regulatory sequences for expression of the sequences encoding a TERT in the cell. Preferably, the introduced gene is not integrated into the host cell's genome.

*Promoter*

As used herein, the term "promoter" or "transcription regulatory sequence" refers to a nucleic acid fragment that functions to control the transcription of one or more coding sequences, and is located upstream with respect to the direction of transcription of the transcription initiation site of the coding sequence, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active in most tissues under most physiological and developmental conditions. An "inducible" promoter is a promoter that is physiologically or developmentally regulated, e.g. by the application of a chemical inducer. As used herein, a "regulator" or "transcriptional regulator" is a protein that controls the rate of transcription of genetic information from DNA to messenger RNA, by binding to a specific DNA sequence. Expression may be assessed as described elsewhere in this section. "Transcriptional initiation capability" or "promoter strength" refers to the extent of the capability of a promoter to initiate transcription of a coding nucleotide sequence to which it is operably linked.

*Operably linked*

As used herein, the term "operably linked" refers to a linkage of polynucleotide elements in a functional relationship. A nucleic acid is "operably linked" when it is placed into a functional relationship with another nucleic acid sequence. For instance, a transcription regulatory sequence is operably linked to a coding sequence if it affects the transcription of the coding sequence. Operably linked means that the DNA sequences being linked are typically contiguous and, where necessary to join two protein encoding regions, contiguous and in reading frame. Linking can be accomplished by ligation at convenient restriction sites or at adapters or linkers inserted in lieu thereof, or by gene synthesis.

*Proteins and amino acids*

The terms "protein" or "polypeptide" or "amino acid sequence" are used interchangeably and refer to molecules consisting of a chain of amino acids, without reference to a specific mode of action, size, 3-dimensional structure or origin. In amino acid sequences as described herein, amino acids or "residues" are denoted by three-letter symbols. These three-letter symbols as well as the corresponding one-letter symbols are well known to a person of skill in the art and have the following meaning: A (Ala) is alanine, C (Cys) is cysteine, D (Asp) is aspartic acid, E (Glu) is glutamic acid, F (Phe) is phenylalanine, G (Gly) is glycine, H (His) is histidine, I (Ile) is isoleucine, K (Lys) is lysine, L (Leu) is leucine, M (Met) is methionine, N (Asn) is asparagine, P (Pro) is proline, Q (Gln) is glutamine, R (Arg) is arginine, S (Ser) is serine, T (Thr) is threonine, V (Val) is valine, W (Trp) is tryptophan, Y (Tyr) is tyrosine. A residue may be any proteinogenic amino acid, but also any non-proteinogenic amino acid such as D-amino acids and modified amino acids formed by post-translational modifications, and also any non-natural amino acid.

*Expression vector*

The phrase "expression vector" or "vector" or "delivery vector" generally refers to a tool in molecular biology used to obtain gene expression in a cell, for example by introducing a nucleotide sequence that is capable of effecting expression of a gene or a coding sequence in a host compatible with such sequences. An expression vector may carry a genome that is able to stabilize and remain episomal in a cell. Within the context of the invention, a cell may mean to encompass a cell used to make the vector or a cell wherein the vector will be administered. Alternatively, a vector is capable of integrating into a cell's genome, for example through homologous recombination or otherwise.

*Viral vector*

A viral vector or a viral expression vector a viral gene therapy vector generally refers to a virus-derived vector used in molecular biology to obtain gene expression in a cell, for example by introducing a nucleotide sequence that is capable of effecting expression of a gene or a coding sequence in a host compatible with such sequences.

A viral vector or a viral gene therapy vector is a vector that is suitable for gene therapy. Vectors that are suitable for gene therapy are described in Anderson 1998, Nature 392: 25-30; Walther and Stein, 2000, Drugs 60: 249-71; Kay et al., 2001, Nat. Med. 7: 33-40; Russell, 2000, J. Gen. Virol. 81: 2573-604; Amado and Chen, 1999, Science 285: 674-6; Federico, 1999, Curr. Opin. Biotechnol.10: 448-53; Vigna and Naldini, 2000, J. Gene Med. 2: 308-16; Marin et al., 1997, Mol. Med. Today 3: 396-403; Peng and Russell, 1999, Curr. Opin. Biotechnol. 10: 454-7; Sommerfelt, 1999, J. Gen. Virol. 80: 3049-64; Reiser, 2000, Gene Ther. 7: 910-3; WO2016/020346, WO2016/020345, Bär et al, 2018, Nat. Comm. 5: 5863, van Lieshout et al, 2018, Mol. Ther. Meth. Clin. Dev. 9, and references cited therein; all of which are incorporated herein by reference in their entirety.

A particularly suitable gene therapy vector includes an adenoviral and adeno-associated virus (AAV) vector. These vectors infect a wide number of dividing and non-dividing cell types including synovial cells and liver cells. The episomal nature of the adenoviral and AAV vectors after cell entry makes these vectors suited for therapeutic applications, (Russell, 2000, J. Gen. Virol. 81: 2573-2604; Goncalves, 2005, Virol J. 2(1):43; WO2016/020346; WO2016/020345; Bär et al, 2018, Nat. Comm. 5: 5863; van Lieshout et al, 2018, Mol. Ther. Meth. Clin. Dev. 9, incorporated herein by reference in their entirety) as indicated above. AAV vectors are even more preferred since they are known to result in very stable long-term expression of transgene expression (up to 9 years in dog (Niemeyer et al, Blood. 2009 Jan 22;113(4):797-806) and ~10 years in human (Buchlis, G. et al., Blood. 2012 Mar 29;119(13):3038-41). Preferred adenoviral vectors are modified to reduce the host response as reviewed by Russell (2000, supra). Methods for gene therapy using AAV vectors are described by Wang et al., 2005, J Gene Med. March 9 (Epub ahead of print), Mandel et al., 2004, Curr Opin Mol Ther. 6(5):482-90, and Martin et al., 2004, Eye 18(11):1049-55, Nathwani et al, N Engl J Med. 2011 Dec 22;365(25):2357-65, Apparailly et al, Hum Gene Ther. 2005 Apr;16(4):426-34; WO2016/020346; WO2016/020345; Bär et al, 2018, Nat. Comm. 5: 5863; van Lieshout et al, 2018, Mol. Ther. Meth. Clin. Dev. 9, all of which are incorporated herein by reference in their entirety.

Another suitable gene therapy vector includes a retroviral vector. A preferred retroviral vector for application in the present invention is a lentiviral based expression construct. Lentiviral vectors have the ability to infect and to stably integrate into the genome of dividing and non-dividing cells (Amado and Chen, 1999 Science 285: 674-6, incorporated herein by reference in their entirety). Methods for the construction and use of lentiviral based expression constructs are described in U.S. Patent No.'s 6,165,782, 6,207,455, 6,218,181, 6,277,633 and 6,323,031 and in Federico (1999, Curr Opin Biotechnol 10: 448-53) and Vigna et al. (2000, J Gene Med 2000; 2: 308-16); all of which are incorporated herein by reference in their entirety. Other suitable gene therapy vectors include an adenovirus vector, a herpes virus vector, a polyoma virus vector or a vaccinia virus vector.

*Adeno-associated virus vector (AAV vector)*

The terms "adeno-associated virus", "AAV virus", "AAV virion", "AAV viral particle" and "AAV particle", used as synonyms herein, refer to a viral particle composed of at least one capsid protein of AAV (preferably composed of all capsid protein of a particular AAV serotype) and an encapsulated polynucleotide of the AAV genome. If the particle comprises a heterologous polynucleotide (i.e. a polynucleotide different from a wild-type AAV genome, such as a gene or a recombinant viral genome to be delivered to a mammalian cell) flanked by AAV inverted terminal repeats, then they are typically known as a "AAV vector particle" or "AAV viral vector" or "AAV vector". AAV refers to a virus that belongs to the genus *Dependoparvovirus* (also referred to as *Dependovirus*) of the family *Parvoviridae.* The AAV genome is approximately 4.7 kb in length and it consists of single strand deoxyribonucleic acid (ssDNA) that can be positive or negative detected. The invention also encompasses the use of double stranded AAV also called dsAAV or scAAV. The genome includes inverted terminal repeats (ITR) at both ends of the DNA strand, and two open reading frames (ORFs): rep and cap. The frame rep is made of four overlapping genes that encode proteins Rep necessary for AAV lifecycle. The frame cap contains nucleotide sequences overlapping with capsid proteins: VP1, VP2 and VP3, which interact to form a capsid of icosahedral symmetry (see Carter and Samulski ., 2000, and Gao et al, 2004, incorporated for reference herein in their entirety).

A preferred viral vector or a preferred gene therapy vector is an AAV vector. An AAV vector as used herein preferably comprises a recombinant AAV vector (rAAV vector). A "rAAV vector" as used herein refers to a recombinant vector comprising part of an AAV genome encapsidated in a protein shell of capsid protein derived from an AAV serotype as explained herein. Part of an AAV genome may contain the inverted terminal repeats (ITR) derived from an adeno-associated virus serotype, such as AAV1, AAV2, AAV3, AAV4, AAV5 and others, as described elsewhere herein.

Protein shell comprised of capsid protein may be derived from any AAV serotype. A protein shell may also be named a capsid protein shell. rAAV vector may have one or preferably all wild type AAV genes deleted, but may still comprise functional ITR nucleic acid sequences. Functional ITR sequences are necessary for the replication, rescue and packaging of AAV virions. The ITR sequences may be wild type sequences or may have at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with wild type sequences or may be altered by for example by insertion, mutation, deletion or substitution of nucleotides, as long as they remain functional. In this context, functionality refers to the ability to direct packaging of the genome into the capsid shell and then allow for expression in the host cell to be infected or target cell. In the context of the present invention a capsid protein shell may be of a different serotype than the rAAV vector genome ITR.

A nucleic acid molecule represented by a nucleic acid sequence of choice is preferably inserted between the rAAV genome or ITR sequences as identified above, for example an expression construct comprising an expression regulatory element operably linked to a coding sequence and a 3' termination sequence. Said nucleic acid molecule may also be called a transgene.

"AAV helper functions" generally refers to the corresponding AAV functions required for rAAV replication and packaging supplied to the rAAV vector *in trans.* AAV helper functions complement the AAV functions which are missing in the rAAV vector, but they lack AAV ITRs (which are provided by the rAAV vector genome). AAV helper functions include the two major ORFs of AAV, namely the *rep* coding region and the *cap* coding region or functional substantially identical sequences thereof. Rep and Cap regions are well known in the art, see *e.g.* Chiorini et al. (1999, J. of Virology, Vol 73(2): 1309-1319) or US 5,139,941, incorporated herein by reference in its entirety. The AAV helper functions can be supplied on an AAV helper construct. Introduction of the helper construct into the host cell can occur e.g. by transformation, transfection, or transduction prior to or concurrently with the introduction of the rAAV genome present in the rAAV vector as identified herein. The AAV helper constructs of the invention may thus be chosen such that they produce the desired combination of serotypes for the rAAV vector's capsid protein shell on the one hand and for the rAAV genome present in said rAAV vector replication and packaging on the other hand.

"AAV helper virus" provides additional functions required for AAV replication and packaging. Suitable AAV helper viruses include adenoviruses, herpes simplex viruses (such as HSV types 1 and 2) and vaccinia viruses. The additional functions provided by the helper virus can also be introduced into the host cell via plasmids, as described in US 6,531,456 incorporated herein by reference in its entirety.

"Transduction" refers to the delivery of a nucleic acid molecule into a recipient host cell by a viral vector. For example, transduction of a target cell by an AAV vector according to the invention leads to transfer of the recombinant viral genome contained in that vector into the transduced cell.

*Expression*

Expression may be assessed by any method known to a person of skill in the art. For example, expression may be assessed by measuring the levels of gene expression in the transduced tissue on the level of the mRNA or the protein by standard assays known to a person of skill in the art, such as qPCR, RNA sequencing, Northern blot analysis, Western blot analysis, mass spectrometry analysis of protein-derived peptides or ELISA.

Expression may be assessed at any time after administration of the recombinant viral genome, expression vector or composition as described herein. In some embodiments herein, expression may be assessed after 1 week, 2 weeks, 3 weeks, 4, weeks, 5 weeks, 6 weeks, 7 weeks, 8 weeks, 9, weeks, 10 weeks, 11 weeks, 12 weeks, 14 weeks, 16 weeks, 18 weeks, 20 weeks, 22 weeks, 24 weeks, 28 weeks, 32 weeks, 36 weeks, 40 weeks, or more.

*Codon optimization*

"Codon optimization", as used herein, refers to the processes employed to modify an existing coding sequence, or to design a coding sequence, for example, to improve translation in an expression host cell or organism of a transcript RNA molecule transcribed from the coding sequence, or to improve transcription of a coding sequence. Codon optimization includes, but is not limited to, processes including selecting codons for the coding sequence to suit the codon preference of the expression host organism. For example, to suit the codon preference of mammalian, preferably of murine, canine or human expression hosts. Codon optimization also eliminates elements that potentially impact negatively RNA stability and/or translation (e. g. termination sequences, TATA boxes, splice sites, ribosomal entry sites, repetitive and/or GC rich sequences and RNA secondary structures or instability motifs). In some embodiments, codon-optimized sequences show at least 3%, 5%, 10%, 15%, 20%, 25%, 30%, 40%, 50%, 60%, 70%, 80%, 90%, 100% or more increase in gene expression, transcription, RNA stability and/or translation compared to the original, non-codon-optimized sequence.

*General terms*

In this document and in its claims, the verb "to comprise" and its conjugations is used in its non-limiting sense to mean that items following the word are included, but items not specifically mentioned are not excluded. In addition, the verb "to consist" may be replaced by "to consist essentially of" meaning that a composition as described herein may comprise additional component(s) than the ones specifically identified, said additional component(s) not altering the unique characteristics of the invention. In addition, the verb "to consist" may be replaced by "to consist essentially of" meaning that a method or use as described herein may comprise additional step(s) than the ones specifically identified, said additional step(s) not altering the unique characteristic of the invention. In addition, the verb "to consist" may be replaced by "to consist essentially of" meaning that a nucleotide or amino acid sequence as described herein may comprise additional nucleotides or amino acids than the ones specifically identified, said additional nucleotides or amino acids not altering the unique characteristics of the invention. Reference to an element by the indefinite article "a" or "an" does not exclude the possibility that more than one of the element is present, unless the context clearly requires that there be one and only one of the elements. The indefinite article "a" or "an" thus usually means "at least one".

As used herein, with "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The word "about" or "approximately" when used in association with a numerical value (e.g. about 10) preferably means that the value may be the given value (of 10) more or less 1% of the value.

As used herein, the term "and/or" indicates that one or more of the stated cases may occur, alone or in combination with at least one of the stated cases, up to with all of the stated cases.

Various embodiments are described herein. Each embodiment as identified herein may be combined together unless otherwise indicated.

One skilled in the art will recognize many methods and materials similar or equivalent to those described herein, which could be used in the practice of the present invention. Indeed, the present invention is in no way limited to the methods and materials described.

The present invention is further described by the following examples which should not be construed as limiting the scope of the invention.

Description of the figures

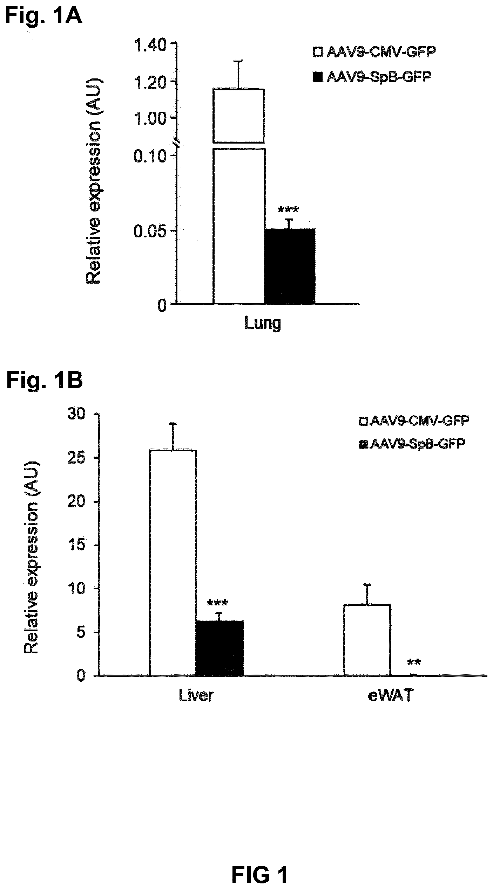

**Figure 1. Comparison of lung-specific expression between AAV9-SpB-GFP and AAV9-CMV-GFP vectors.**

Mice were treated with AAV9 vectors encoding the marker protein GFP under the control of the shortened SpB promoter (AAV9-SpB-GFP) or under the control of the ubiquitous CMV promoter (AAV9-CMV-GFP). A dose of $1.75 \times 10^{12}$ viral genomes (vg) of AAV9-SpB-GFP or AAV9-CMV-GFP vectors was administered intravenously (IV) via tail vein. GFP expression levels were evaluated by quantitative PCR four weeks post-AAV administration in lung (**Fig. 1A**), liver and epididymal white adipose tissue (eWAT) (**Fig. 1B**). Results are expressed as mean relative expression (AU) + SEM. \*\*$p<0.01$, \*\*\*$p<0.001$ vs AA V9-CMV-GFP.

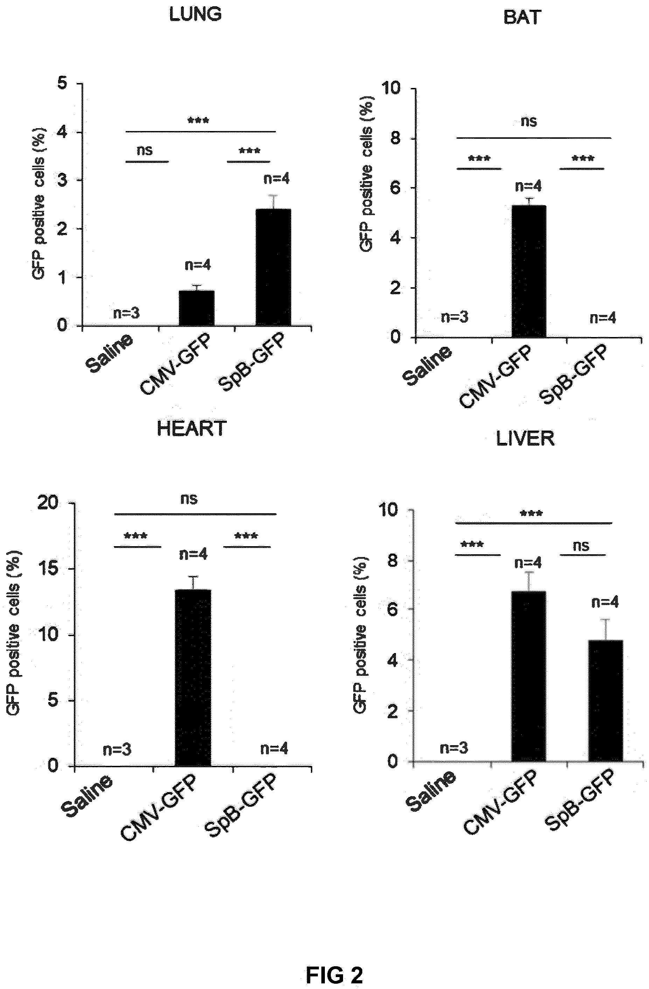

**Figure 2. Quantification of GFP positive cells in tissues of mice treated with AAV9-SpB-GFP and AAV9-CMV-GFP vectors.** Mice were treated with AAV9 vectors encoding the marker protein GFP under the control of the shortened SpB promoter (AAV9-SpB-GFP) or under the control of the ubiquitous CMV promoter (AAV9-CMV-GFP). A dose of $1.75 \times 10^{12}$ viral genomes (vg) of AAV9-SpB-GFP or AAV9-CMV-GFP vectors was administered intravenously (IV) via tail vein. Saline solution was used as control. The percentage of GFP positive cells in lung, liver, heart, and brown adipose tissue (BAT) in mice treated with AAV9-SpB-GFP, AAV9-CMV-GFP, or saline was quantified. Results were evaluated by quantitative PCR four weeks post-AAV administration. Results are expressed as mean percentage + SEM. n= 3-4. \*\*\*$p< 0.05$, ns not significant. Straight lines connecting bars indicate the comparisons.

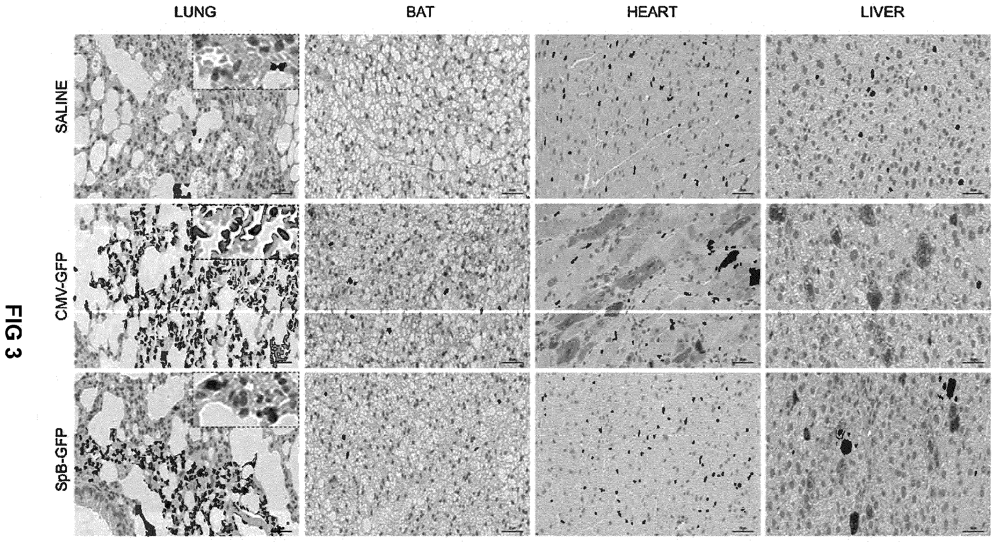

**Figure 3. Visualisation of GFP positive cells in tissues of mice treated with AAV9-SpB-GFP and AAV9-CMV-GFP vectors.** Mice were treated with AAV9 vectors encoding the marker protein GFP under the control of the shortened SpB promoter (AAV9-SpB-GFP) or under the control of the ubiquitous CMV promoter (AAV9-CMV-GFP). A dose of $1.75 \times 10^{12}$ viral genomes (vg) of AAV9-SpB-GFP or AAV9-CMV-GFP vectors was administered intravenously (IV) via tail vein. Results were evaluated four weeks post-AAV administration. Saline solution was used as control. GFP positive cells in lung, brown adipose tissue (BAT), heart, and liver were detected by immunohistochemistry staining using a rabbit monoclonal antibody against GFP and visualised by light microscopy.

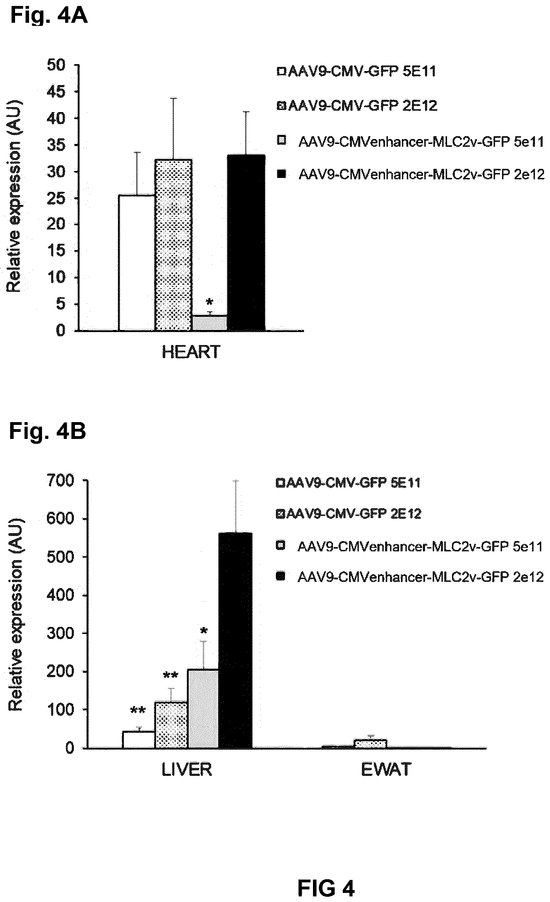

**Figure 4. Comparison of heart-specific expression between AAV9-CMVenh-MLC2v-GFP and AAV9-CMV-GFP vectors.** Mice were treated with two different doses ($5 \times 10^{11}$ or $2 \times 10^{12}$ viral genomes (vg)/mouse) of AAV9 vectors encoding the marker protein GFP under the control of the CMVenh-MLC2v promoter (AAV9-CMVenhancer-MLC2v-GFP) or under the control of the ubiquitous CMV promoter (AAV9-CMV-GFP), administered intravenously (IV) via tail vein. GFP expression levels were evaluated by quantitative PCR three weeks post-AAV administration in heart **(Fig. 4A)**, liver and epididymal white adipose tissue (eWAT) **(Fig. 4B)**. Results are expressed as mean relative expression (AU) + SEM. \*$p<0.05$, \*\*$p<0.01$ vs AAV9-CMVenhancer-MLC2v-GFP $2 \times 10^{12}$ vg/mouse.

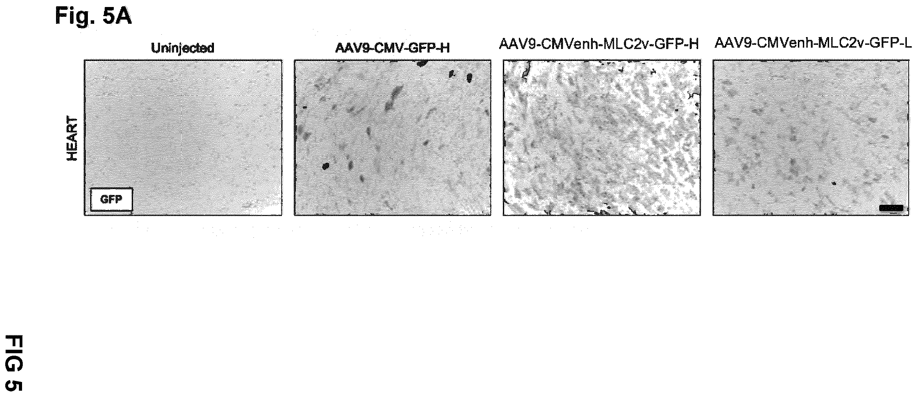

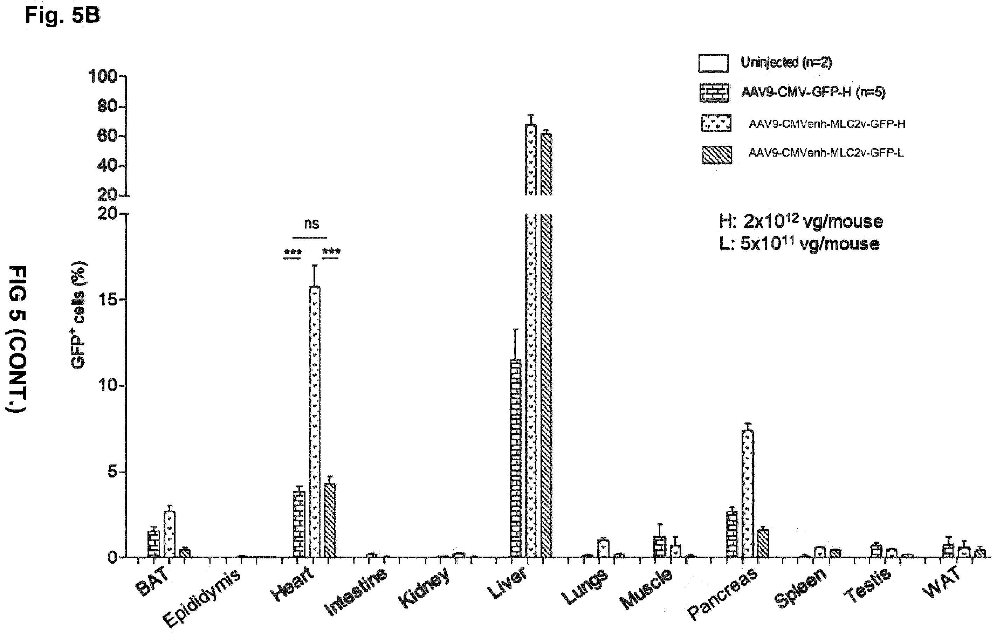

**Figure 5. Visualisation and quantification of GFP positive cells in tissues of mice treated with AAV9-CMVenh-MLC2v-GFP and AAV9-CMV-GFP vectors.** Mice were treated with two different doses ($5 \times 10^{11}$ (low (L) dose) or $2 \times 10^{12}$ (high (H) dose) viral genomes (vg)/mouse) of AAV9 vectors encoding the marker protein GFP under the control of the CMVenh-MLC2v promoter (AAV9-CMVenh-MLC2v-GFP) or under the control of the ubiquitous CMV promoter (AAV9-CMV-GFP), administered intravenously via tail vein. Results were evaluated three weeks post-AAV administration. Uninjected mice were used as control. GFP positive cells in heart, brown adipose tissue (BAT), epididymis, intestine, kidney, liver, lungs, muscle, pancreas, spleen, testis and white adipose tissue (WAT) were detected by immunohistochemistry staining using a rabbit monoclonal antibody against GFP and visualised by light microscopy. In **Fig. 5A** GFP positive cells in the heart are visualised . In **Fig. 5B** the percentage of GFP positive cells in brown adipose tissue (BAT), epididymis, heart, intestine, kidney, liver, lungs, muscle, pancreas, spleen, testis, and white adipose tissue (WAT) is quantified. Results are expressed as mean percentage + SEM. n= 2-6. \*\*\*$p< 0.05$, ns not significant. Straight lines connecting bars indicate the comparisons.

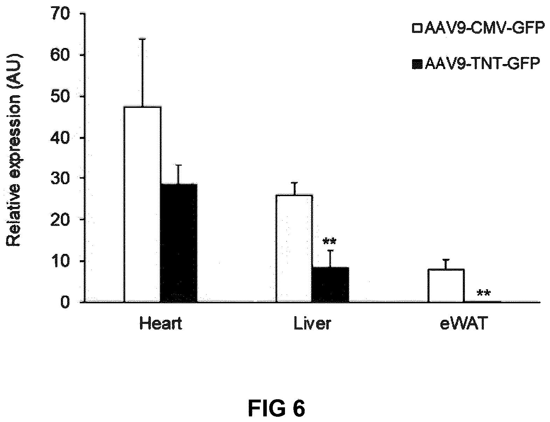

**Figure 6. Comparison of heart-specific expression between AAV9-TNT-GFP and AAV9-CMV-GFP vectors.** 7-week-old male C57Bl6 mice were treated with AAV9 vectors encoding the marker protein GFP under the control of the shortened TNT promoter (AAV9-TNT-GFP) or under the control of the ubiquitous CMV promoter (AAV9-CMV-GFP). A dose of $1.75 \times 10^{12}$ viral genomes (vg) of AAV9-TNT-GFP or AAV9-CMV-GFP vectors was administered intravenously (IV) via tail vein. GFP expression levels were evaluated by quantitative PCR four weeks post-AAV administration in heart, liver and epididymal white adipose tissue (eWAT). Results are expressed as mean relative expression (AU) + SEM. \*\*$p<0.01$ vs AAV9-CMV-GFP.

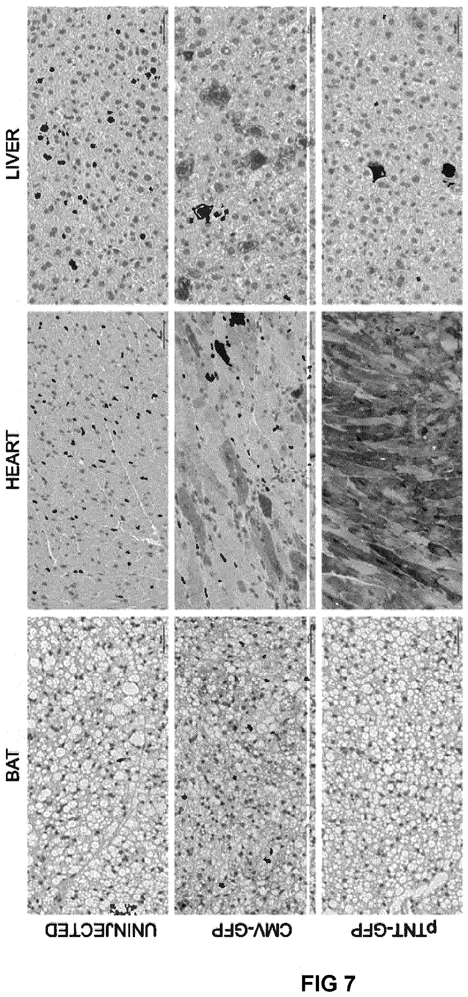

**Figure 7. Visualisation of GFP positive cells in tissues of mice treated with AAV9-TNT-GFP and AAV9-CMV-GFP vectors.** 7-week-old male C57Bl6 mice were treated with AAV9 vectors encoding the marker protein GFP under the control of the shortened TNT promoter (AAV9-TNT-GFP) or under the control of the ubiquitous CMV promoter (AAV9-CMV-GFP). A dose of $1.75 \times 10^{12}$ viral genomes (vg) of AAV9-TNT-GFP or AAV9-CMV-GFP vectors was administered intravenously (IV) via tail vein. Uninjected mice were used as control. Results were evaluated four weeks post-AAV administration. GFP positive cells in brown adipose tissue (BAT), heart, and liver were detected by immunohistochemistry staining using a rabbit monoclonal antibody against GFP and visualised by light microscopy.

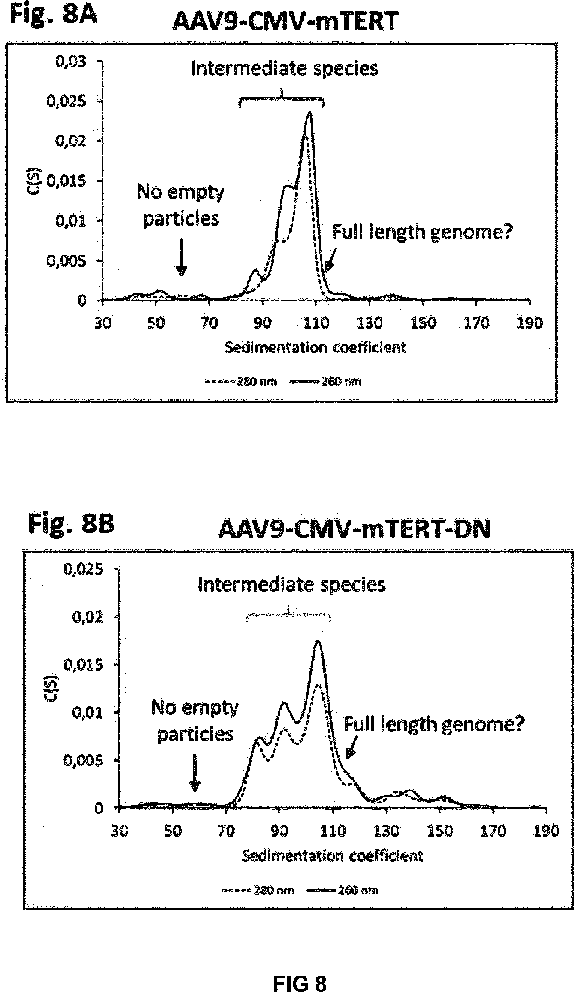

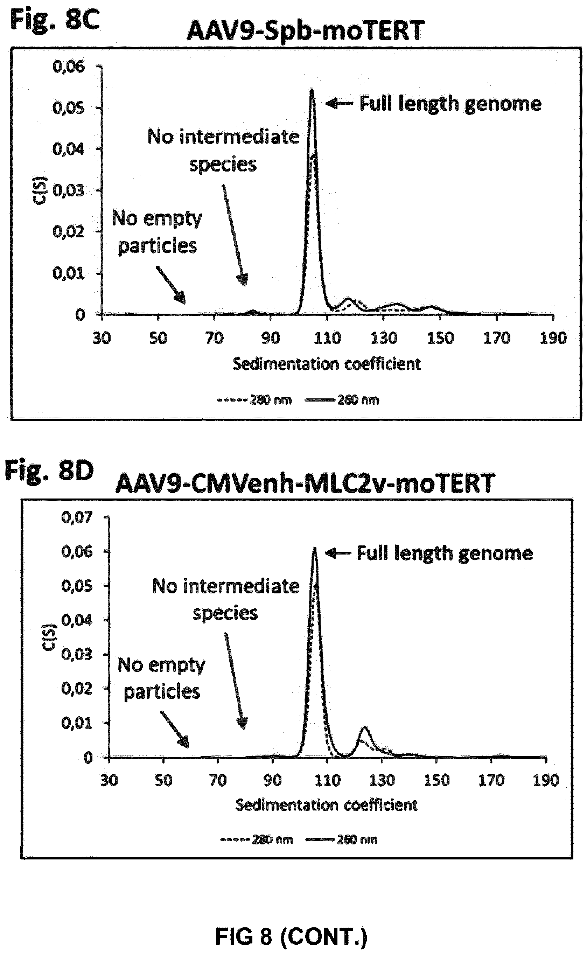

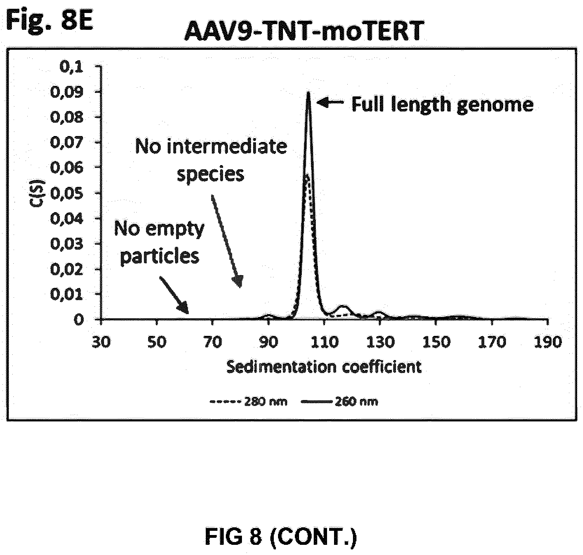

**Figure 8. Characterization of AAV vectors by sedimentation velocity analytical ultracentrifugation.** AAV9-CMV-mTert (**Fig. 8A**, 5291 nucleotides), AAV9-CMV-mTERT-DN (**Fig. 8B,** 5291 nucleotides), AAV9-SpB-moTERT (**Fig. 8C**, 4638 nucleotides), AAV9-CMVenh-MLC2v-moTERT (**Fig. 8D**, 4676 nucleotides), AA9-TNT-moTERT (**Fig. 8E**, 4536 nucleotides). Sedimentation velocity centrifugation was performed at 20,000 rpm. Particle sedimentation was recorded at 260 nm and 280 nm in the same run. The relative percentage of each peak in the C(S) distribution was calculated based on the molar concentration of each species in relation to the sum of the molar concentration of all species in the distribution. Peaks corresponding to empty vectors, intermediate species and vectors containing full length genomes are indicated with arrows.

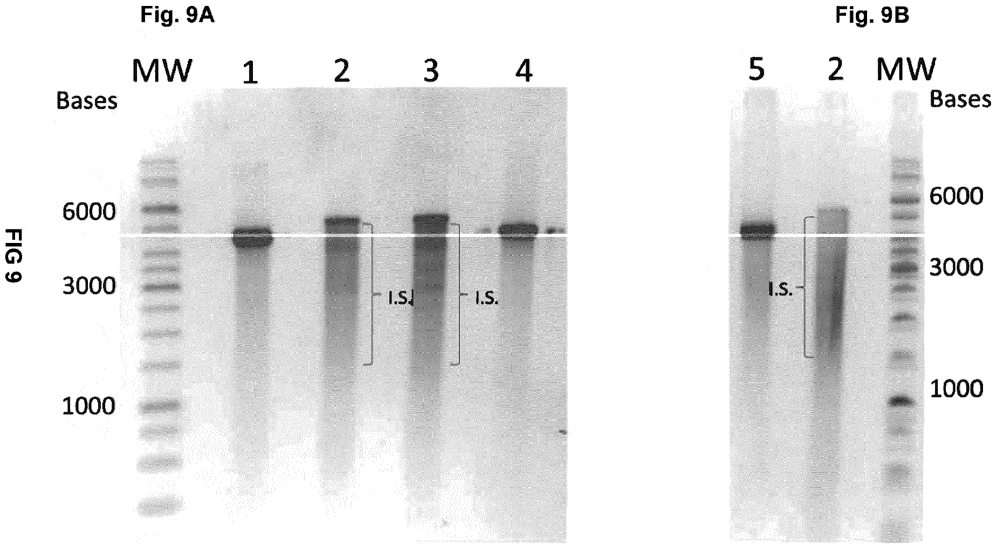

**Figure 9. Characterization of AAV vectors by alkaline agarose gel electrophoresis.** Purified vector DNA was subjected to agarose gel electrophoresis under denaturing conditions. Band size and quality (presence of smear) were examined to assess genome integrity. MW denotes the DNA ladder. I.S. denotes the presence of intermediate species. **(Fig. 9A)** AAV9-SpB-moTERT (well "1", 4638 nucleotides), AAV9-CMV-mTert (well "2", 5291 nucleotides), AAV9-CMV-mTERT-DN (well "3", 5291 nucleotides), AAV9-CMVenh-MLC2v-moTERT (well "4", 4676 nucleotides). **(Fig. 9B)** AA9-TNT-moTERT (well "5", 4536 nucleotides), AAV9-CMV-mTert (well "2", 5291 nucleotides).

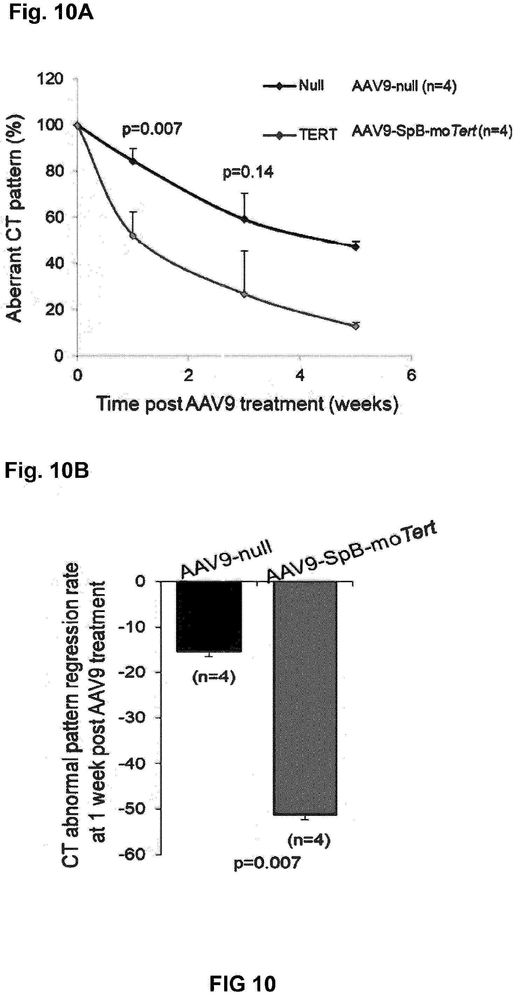

**Figure 10. Evaluation of AAV9-SpB-moTERT therapeutic benefit in a pulmonary fibrosis mouse model.** Bleomycin (0.5 mg/kg body weight) was instilled intratracheally in 8-week-old G3-Tert-/- male mice. Two weeks post-Bleomycin insult, a computerized tomography (CT) scan was performed for each mouse to diagnose bona-fide pulmonary fibrotic pattern. Mice diagnosed with pulmonary fibrosis were intravenously (IV) administered either with $2x10^{12}$ vg of AAV9-SpB-moTERT or AAV9-null vector (negative control). One, 3 and 5 weeks post-viral treatment a CT scan was performed for measurement of the decrease in the affected lung volume showing an aberrant CT pattern relative to the start of the treatment. **(Fig. 10A)** Time series of decrease in aberrant fibrotic pattern (%) as measured by CT scan. **(Fig. 10B)** CT abnormal pattern regression rate at 1 week post-treatment. Results are expressed as mean percentage of initial fibrotic pattern + SEM. n = 4.

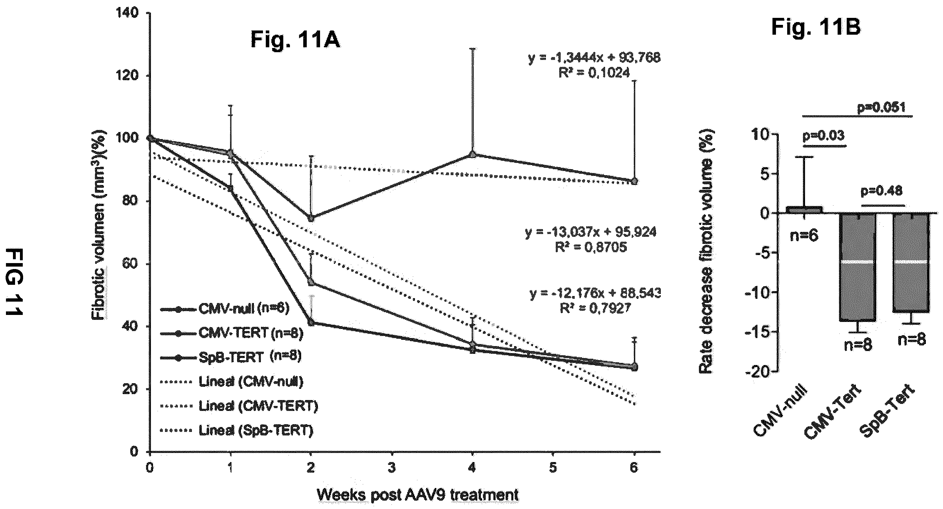

**Figure 11. Evaluation of therapeutic efficacy of by AAV9-SpB-moTERT-mediated pulmonary fibrosis treatment in a mouse model.** Bleomycin (0.5-0.7 mg/kg body weight) was instilled intratracheally in 8-week-old G3-Tert-/- male mice. Mice diagnosed with pulmonary fibrosis were intravenously (IV) administered either with $2x10^{12}$ vg of AAV-CMV-null, with $2x10^{12}$ vg of AAV9-CMV-mTERT or with $2x10^{12}$ vg of AAV9-SpB-moTERT. Disease progression was followed by weekly CT-scans. A weekly CT scan was performed for measurement of the decrease in the fibrotic lung volume relative to the start of the treatment. **(Fig. 11A)** Time series of decrease in aberrant fibrotic volume (%) as measured by CT scan. **(Fig. 11B)** Rate of decrease of affected volume calculated during the first six weeks of treatment.

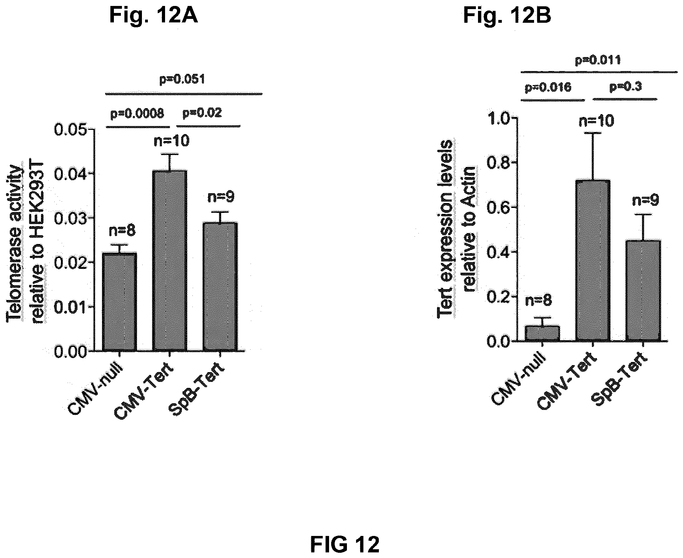

**Figure 12. TERT expression levels and activity in lung samples of mice with pulmonary fibrosis treated with AAV9-SpB-moTERT.** Bleomycin (0.5-0.7 mg/kg body weight) was instilled intratracheally in 8-week-old G3-Tert-/- male mice. Mice diagnosed with pulmonary fibrosis were intravenously (IV) administered either with $2x10^{12}$ vg of AAV-CMV-null, with $2x10^{12}$ vg of AAV9-CMV-mTERT or with $2x10^{12}$ vg of AAV9-SpB-moTERT. Mice were sacrificed at 8 weeks post-treatment and TERT expression levels and activity were measured in lung samples. **(Fig. 12A)** TERT activity (relative to HEK293T) were measured in lung samples. **(Fig. 12B)** TERT expression levels (relative to actin). Results are expressed as mean percentage + SEM. n=8-10.

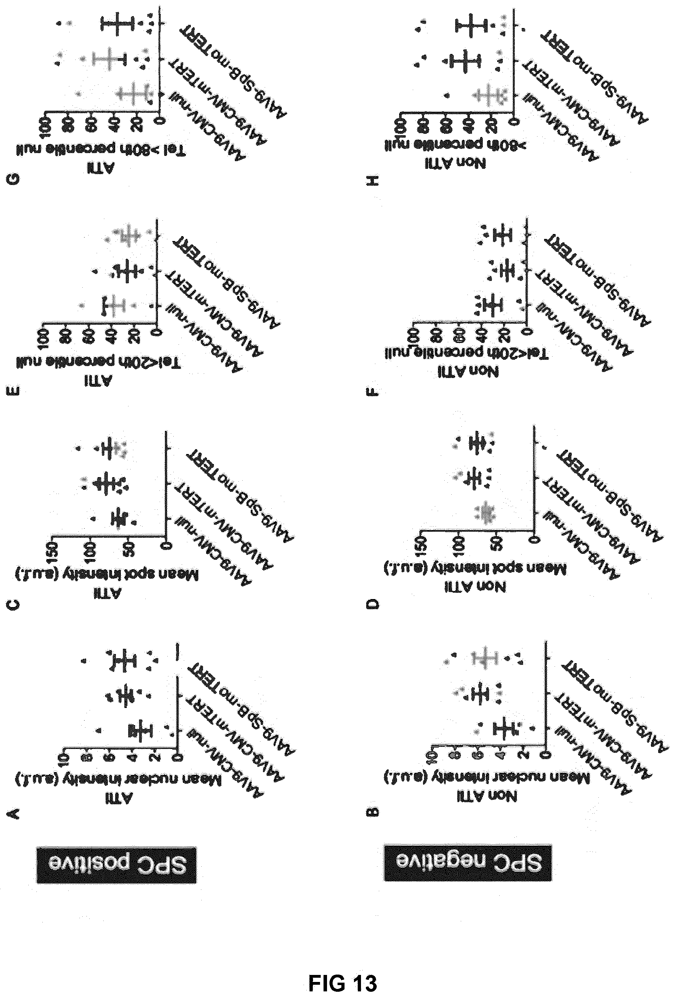

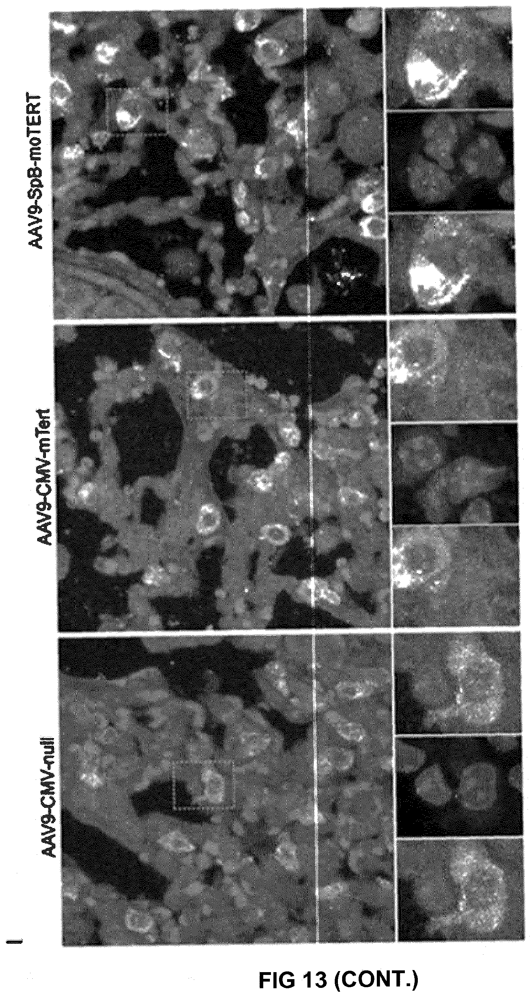

**Figure 13. Telomere length analysis in lung samples of AAV9-CMV-null, AAV9-CMV-mTERT and AAV9-SpB-moTERT treated mice.** A-B. Mean nuclear intensity in ATII cells SPC positive (A) and SPC negative cells (B). C-D. Mean telomere intensity in ATII cells SPC positive (C) and SPC negative cells (D). E-F. Percentage of short telomeres below 20th percentile of AAV9-CMV-null control samples in ATII cells SPC positive (E) and SPC negative cells (F). G-H. Percentage of short telomeres below 20th percentile of AAV9-CMV-null control samples in ATII cells SPC positive (E) and SPC negative cells (F). G-H. Percentage of long telomeres above 80th percentile of AAV9-CMV-null control samples in ATII cells SPC positive (G) and SPC negative cells (H). I. Representative images of SPC immunofluorescence and telomeric Fish in lung samples.

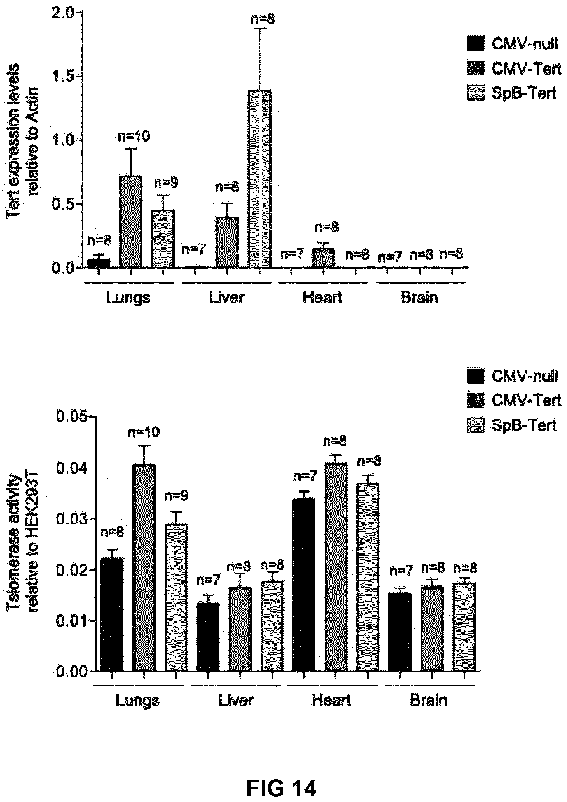

**Figure 14. Quantification of Tert expression levels and telomerase activity (TRAP) in lung, liver, heart and brain samples.**
Bleomycin (0.5-0.7 mg/kg body weight) was instilled intratracheally in 8-week-old G3-Tert-/- male mice. Mice diagnosed with pulmonary fibrosis were intravenously (IV) administered either with $2x10^{12}$ vg of AAV-CMV-null, with $2x10^{12}$ vg of AAV9-CMV-mTERT or with $2x10^{12}$ vg of AAV9-SpB-moTERT. Mice were sacrificed at 8 weeks post-treatment and TERT expression levels and activity were measured in lung samples, liver, heart and brain.

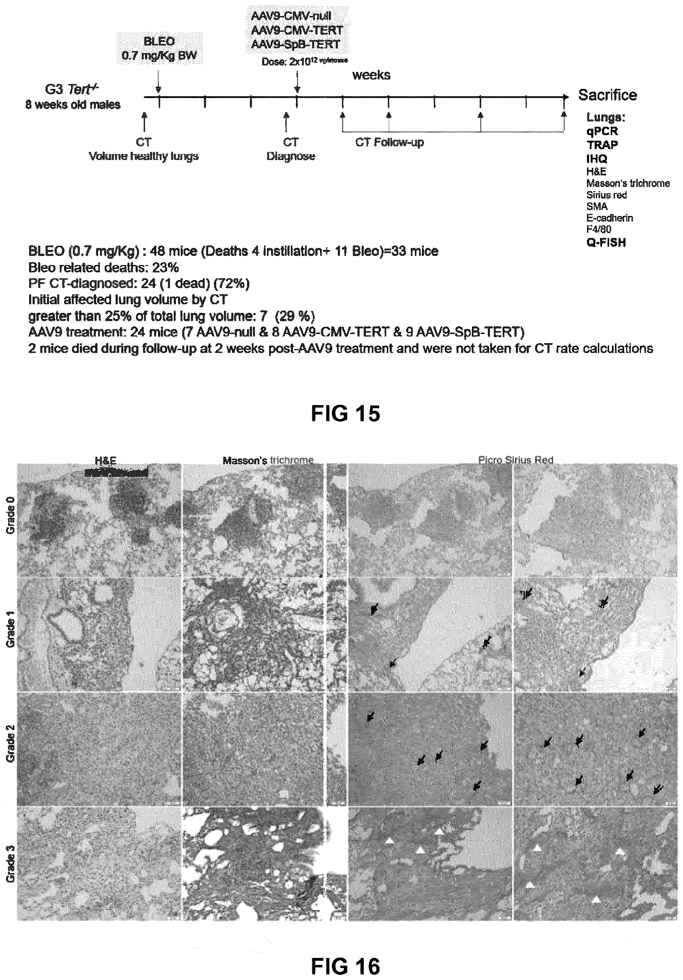

**Figure 15. Evaluation of therapeutic efficacy in PF regression of AAV9-SpB-moTERT and AAV9-CMV-mTERT. Experimental lay-out**

A mouse model of pulmonary fibrosis induced by short telomeres is used. This model is based on the intratracheal instillation of a low dose of bleomycin (0.7 mg/Kg body weight) in 8 weeks old G3-*Tert*$^{-/-}$ male mice, a dose that does not induce fibrosis in wild type mice with normal length telomeres. Before bleomycin (Bleo) insult, an initial CT is performed for each mouse to calculate the volume of healthy lungs for further calculation of disease progression. Two weeks post-Bleo insult a computerized tomography (CT) will be performed for each mouse to diagnosed *bona-fide* pulmonary fibrotic pattern. Mice diagnosed with pulmonary fibrosis were intravenously (IV) administered either with $2x10^{12}$ vg of AAV-CMV-null, with $2x10^{12}$ vg of AAV9-CMV-mTERT or with $2x10^{12}$ vg of AAV9-SpB-moTERT. Disease progression was followed by CT-scans. CT scans were performed at 1, 2, 4, 6 weeks post-infection to measure the decrease in the fibrotic lung volume relative to the start of the treatment. Mice are sacrificed either at 8 weeks post-infections.

**Figure 16. Representative images of lung fibrotic pathologies with different PF scores.**

Histopathological representative images of fibrosis score. Fibrotic thickening of interalveolar septa in Grades 1 and 2 were showed with Masson's Trichrome (see arrows) and Picro Sirius Red (see arrows). In Grade 3, isolated fibrotic areas with thickening of interalveolar septa (see arrowheads).

**Figure 17. Lung histopathological analysis at the end point (8 weeks post AAV9 treatment) and correlation with CT molecular imaging.**

Quantification of PF score (A) and CT value (B) at 8 weeks post AAV9 treatment. A correlation analysis was performed between PF score and CT values (C).

Pulmonary Fibrosis (PF) histological score: Semiquantitative scoring of pulmonary fibrosis based on the analysis of 10 visual fields (200x) stained with Sirius red. (Lawson et al, 2005, 2011; Degryse AL et al 2012)

Grade 0: normal lung architecture
Grade 1: Increased thickness of some (≤50%) of interalveolar septa
Grade 2: Thickening of >50% of interalveolar septa without formation of fibrotic lesions
Grade 3: Thickening of the interalveolar septa with formation of isolated fibrotic lesions
Grade 4: Multiple fibrotic lesions with total or subtotal distortion of parenchymal architecture

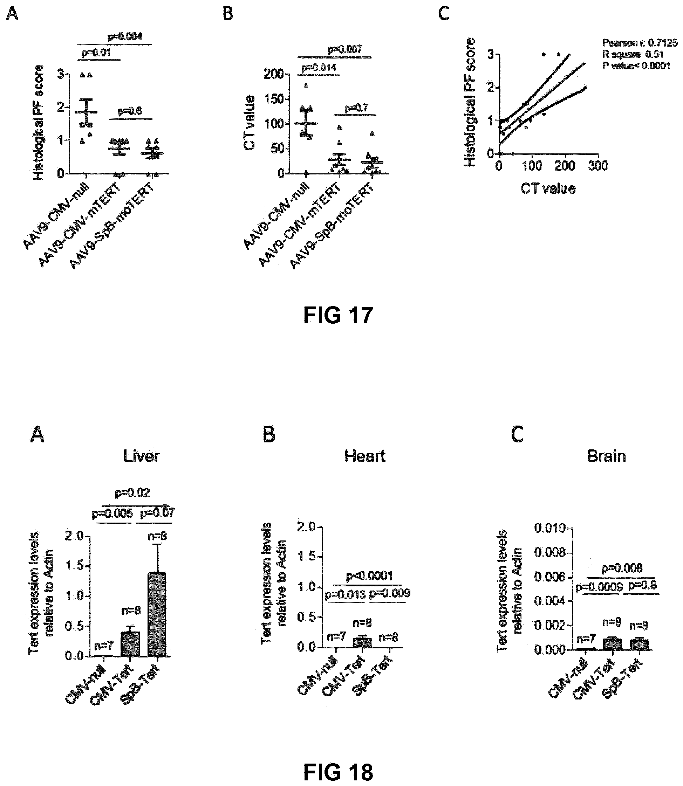

**Figure 18. Telomerase expression (qPCR) in liver, heart and brain samples**

Bleomycin (0.7 mg/kg body weight) was instilled intratracheally in 8-week-old G3-Tert-/- male mice. Mice diagnosed with pulmonary fibrosis were intravenously (IV) administered either with $2x10^{12}$ vg of AAV-CMV-null, with $2x10^{12}$ vg of AAV9-CMV-mTERT or with $2x10^{12}$ vg of AAV9-SpB-moTERT. Mice were sacrificed at 8 weeks post-treatment and TERT expression levels was measured in liver (A), heart (B) and brain (C) samples.

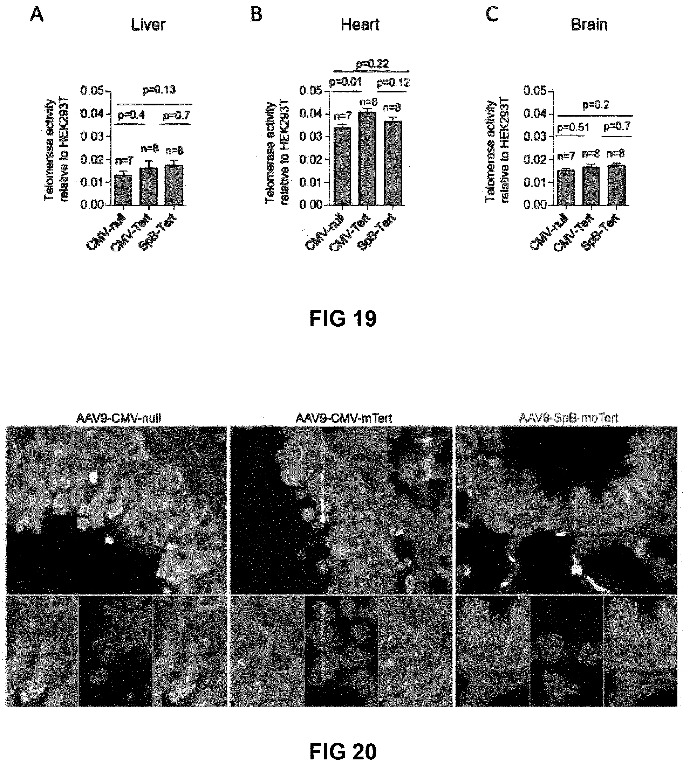

**Figure 19. Telomerase activity (TRAP) in liver, heart and brain samples**

Bleomycin (0.7 mg/kg body weight) was instilled intratracheally in 8-week-old G3-Tert-/- male mice. Mice diagnosed with pulmonary fibrosis were intravenously (IV) administered either with $2x10^{12}$ vg of AAV-CMV-null, with $2x10^{12}$ vg of AAV9-CMV-mTERT or with $2x10^{12}$ vg of AAV9-SpB-moTERT. Mice were sacrificed at 8 weeks post-treatment and telomerase activity was measured in liver (A), heart (B) and brain (C) samples.

Figure 20. Representative images of Immuno qFISH CC10 & Tel probe & DAPI

Representative images of CC10 immunofluorescence and telomeric Fish in lung samples of AAV9-CMV-null, AAV9-CMV-mTERT and AAV9-SpB-moTERT treated mice.

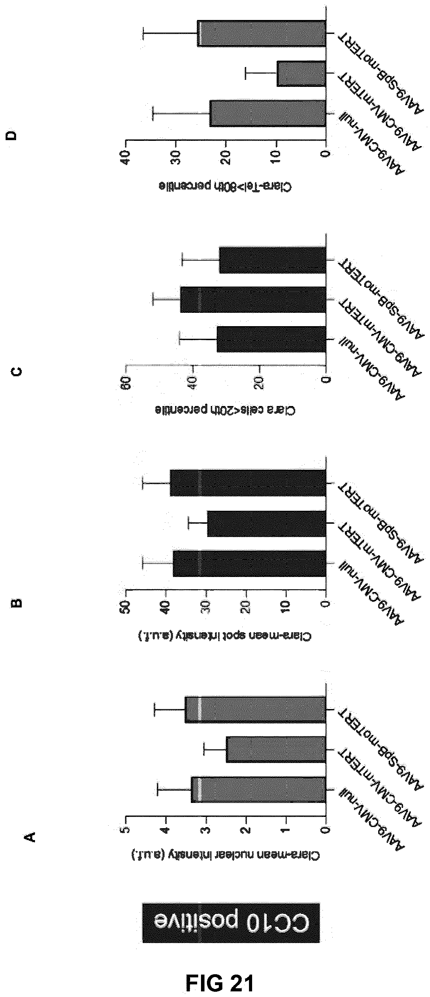

Figure 21. Quantification of Immuno-qFISH CC10-Tel (Clara cells specific)

Telomere length analysis in Clara cells in lung samples of AAV9-CMV-null, AAV9-CMV-mTERT and AAV9-SpB-moTERT treated mice. Mean nuclear intensity in Clara cells CC10 positive (A), mean telomere intensity in Clara cells CC10 positive (B), percentage of short telomeres below 20th percentile of AAV9-CMV-null control samples in Clara cells CC10 positive (C) and percentage of long telomeres above 80th percentile of AAV9-CMV-null control samples Clara cells CC10 positive (D)

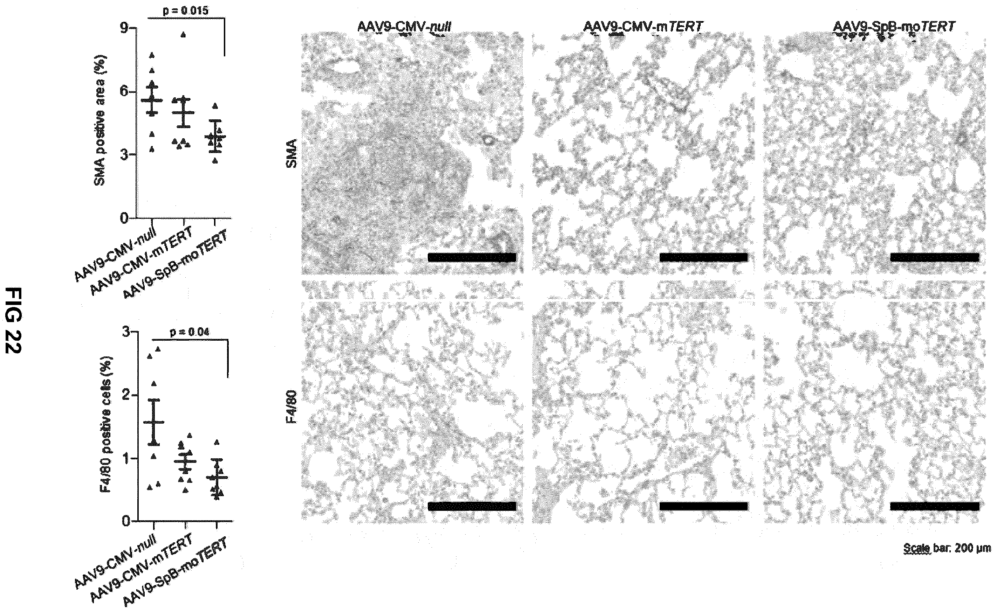

**Figure 22. Quantification of SMA and F4/80.**
Mice diagnosed with pulmonary fibrosis were intravenously (IV) administered either with $2\times10^{12}$ vg of AAV-CMV-null, with $2\times10^{12}$ vg of AAV9-CMV-mTERT or with $2\times10^{12}$ vg of AAV9-SpB-moTERT. Mice were sacrificed at 8 weeks post-treatment and smooth muscle actin (SMA) positive area (upper panel) and macrophage F4/80 positive cells (lower panel) were analyzed in lung samples. Representative IHQ images of SMA and F4/80 staining are shown to the right.

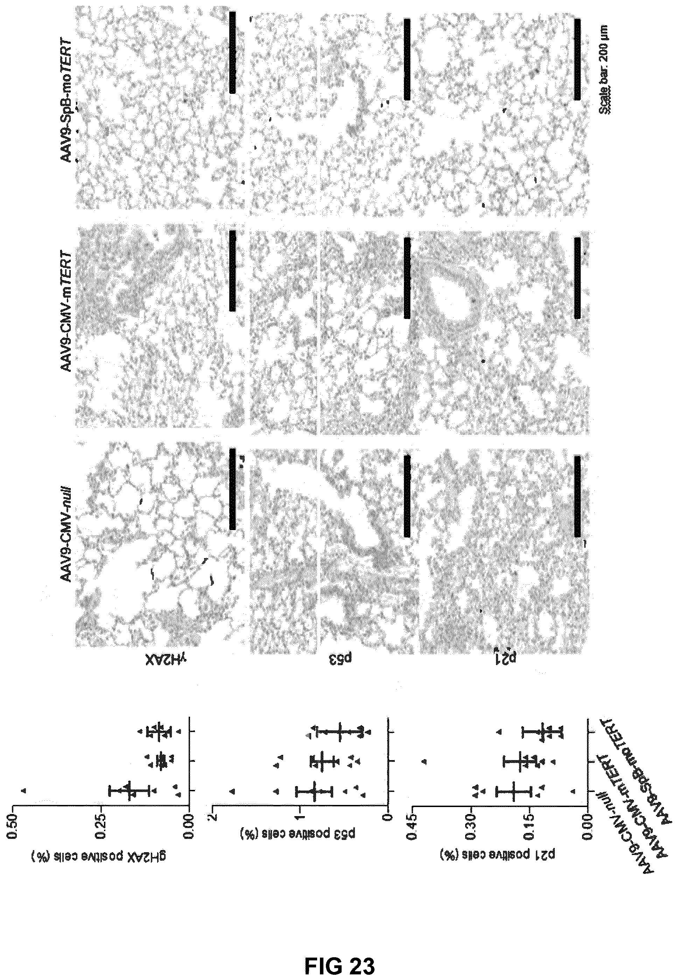

**Figure 23. Quantification of γH2AX, p53 and p21 positive cells in lung samples.**
Mice diagnosed with pulmonary fibrosis were intravenously (IV) administered either with $2\times10^{12}$ vg of AAV-CMV-null, with $2\times10^{12}$ vg of AAV9-CMV-mTERT or with $2\times10^{12}$ vg of AAV9-SpB-moTERT. Mice were sacrificed at 8 weeks post-treatment and yH2AX (upper panel), p53 (middle panel) and p21 (lower panel) positive cells were analyzed in lung samples. Representative IHQ images of yH2AX, p53 and p21 staining are shown to the right.

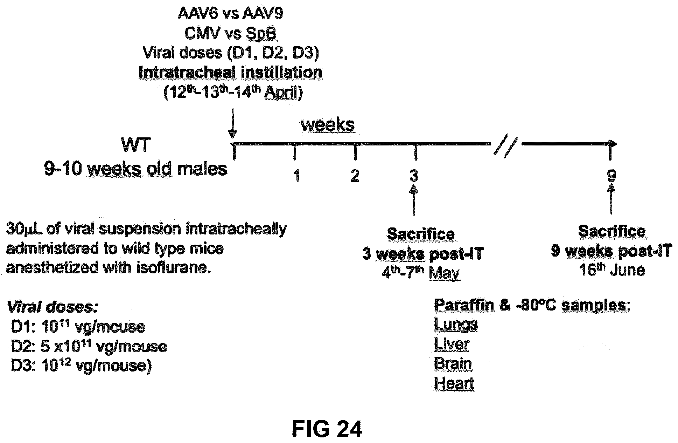

**Figure 24. experimental lay-out.**
Healthy wild-type mice are intratraqueally administered with the vectors AAV9-CMV-null (Batch 729, UAB), AAV9-CMV-GFP (Batch 941, UAB), AAV9-SpB-GFP (Batch 893, UAB), AAV6-CMV-null (Batch 942, UAB), AAV6-CMV-GFP (Batch 943, UAB) and AAV6-SpB-GFP (Batch 944, UAB) at the 10E11 vg/mouse (D1), 5x10E11 vg/mouse (D2) and 10E12 vg/mouse (D3) doses. Mice are sacrificed at 3 and 9 weeks post-infection. GFP expression is evaluated in the five lung lobes; right superior, right middle, right inferior, post-caval and left lobes. GFP expression is also evaluated in liver, heart and brain to address off-target tissues.

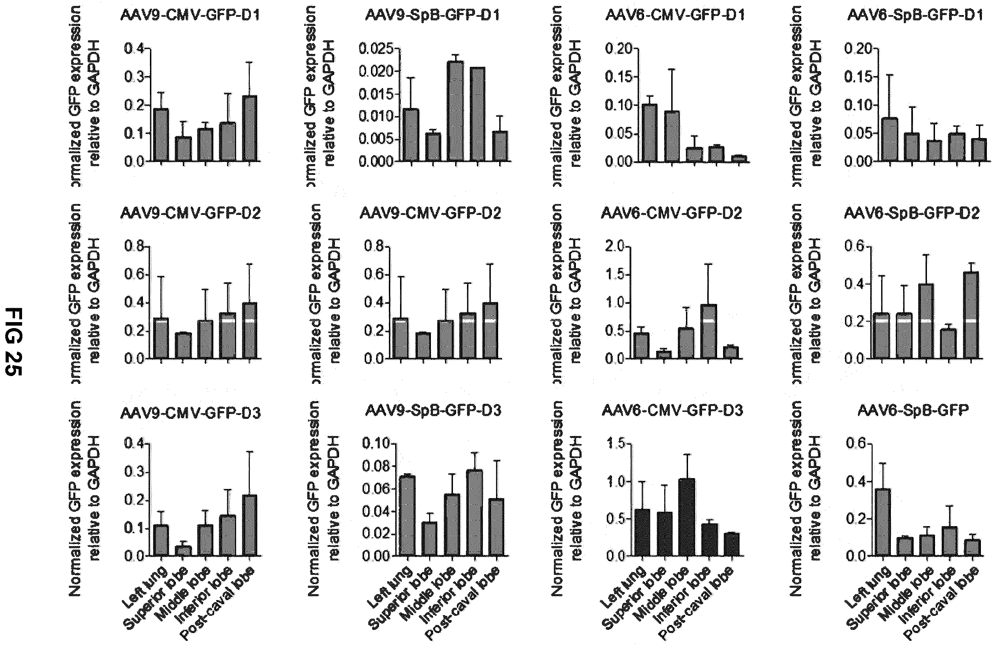

**Figure 25. q-RT-PCR analysis in lung lobes at three weeks post infection with viral vectors**
Healthy wild-type mice are intratraqueally administered with the vectors AAV9-CMV-null (Batch 729, UAB), AAV9-CMV-GFP (Batch 941, UAB), AAV9-SpB-GFP (Batch 893, UAB), AAV6-CMV-null (Batch 942, UAB), AAV6-CMV-GFP (Batch 943, UAB) and AAV6-SpB-GFP (Batch 944, UAB) at the 10E11 vg/mouse (D1), 5x10E11 vg/mouse (D2) and 10E12 vg/mouse (D3) doses. Mice are sacrificed at 3 weeks post-infection. GFP expression is evaluated in the five lung lobes; right superior, right middle, right inferior, post-caval and left lobes by qPCR.

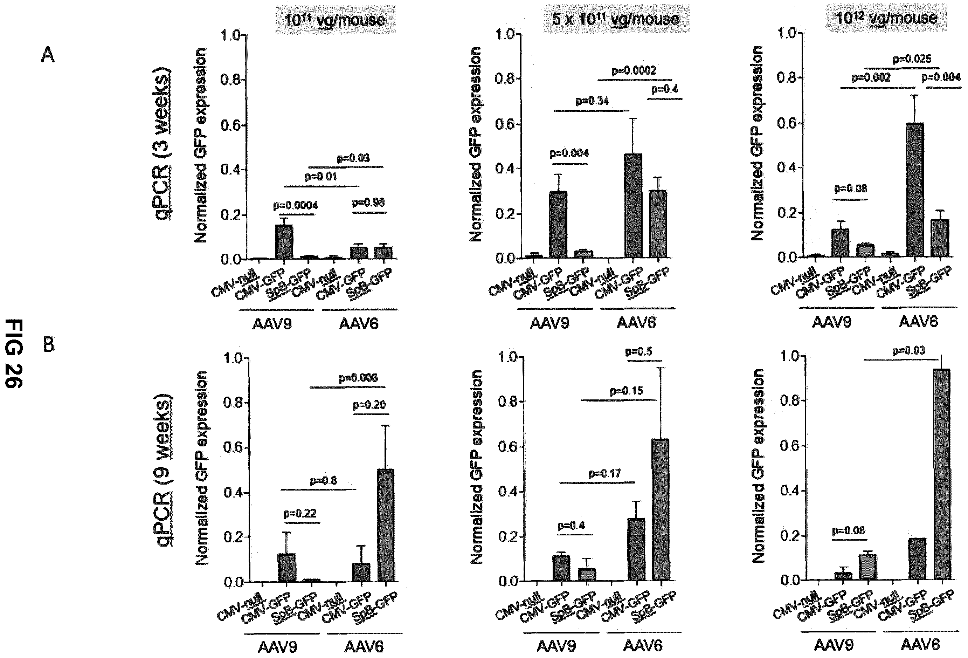

**Figure 26. q-RT-PCR analysis in lung samples at three and nine weeks post infection with viral vectors**
Healthy wild-type mice are intratraqueally administered with the vectors AAV9-CMV-null (Batch 729, UAB), AAV9-CMV-GFP (Batch 941, UAB), AAV9-SpB-GFP (Batch 893, UAB), AAV6-CMV-null (Batch 942, UAB), AAV6-CMV-GFP (Batch 943, UAB) and AAV6-SpB-GFP (Batch 944, UAB) at the 10E11 vg/mouse (D1), 5x10E11 vg/mouse (D2) and 10E12 vg/mouse (D3) doses. Mice are sacrificed at 3 and 9 weeks post-infection. GFP expression is evaluated in the lungs by qPCR (part a) at 3 weeks and part b) at 9 weeks).

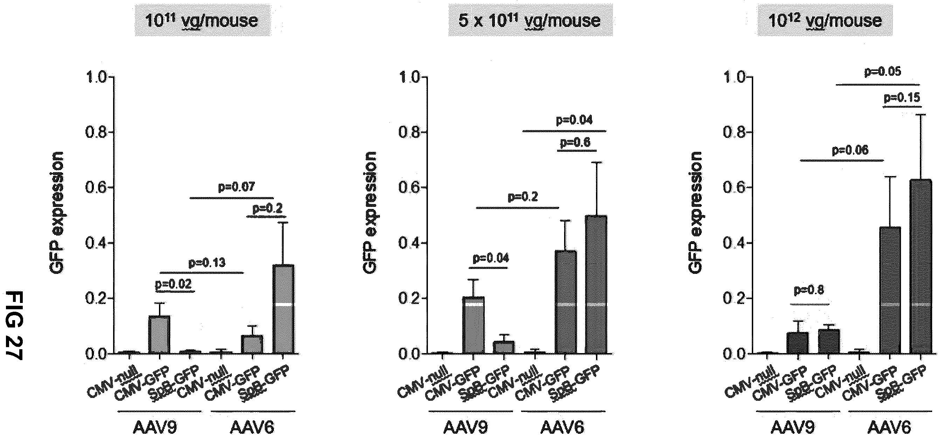

Figure 27. q-RT-PCR analysis in lung samples at three and nine weeks post infection with viral vectors.
Healthy wild-type mice are intratraqueally administered with the vectors AAV9-CMV-null (Batch 729, UAB), AAV9-CMV-GFP (Batch 941, UAB), AAV9-SpB-GFP (Batch 893, UAB), AAV6-CMV-null (Batch 942, UAB), AAV6-CMV-GFP (Batch 943, UAB) and AAV6-SpB-GFP (Batch 944, UAB) at the 10E11 vg/mouse (D1), 5x10E11 vg/mouse (D2) and 10E12 vg/mouse (D3) doses. Mice are sacrificed at 3 and 9 weeks post-infection and the results at both time points are pulled. GFP expression is evaluated in the lungs by qPCR.

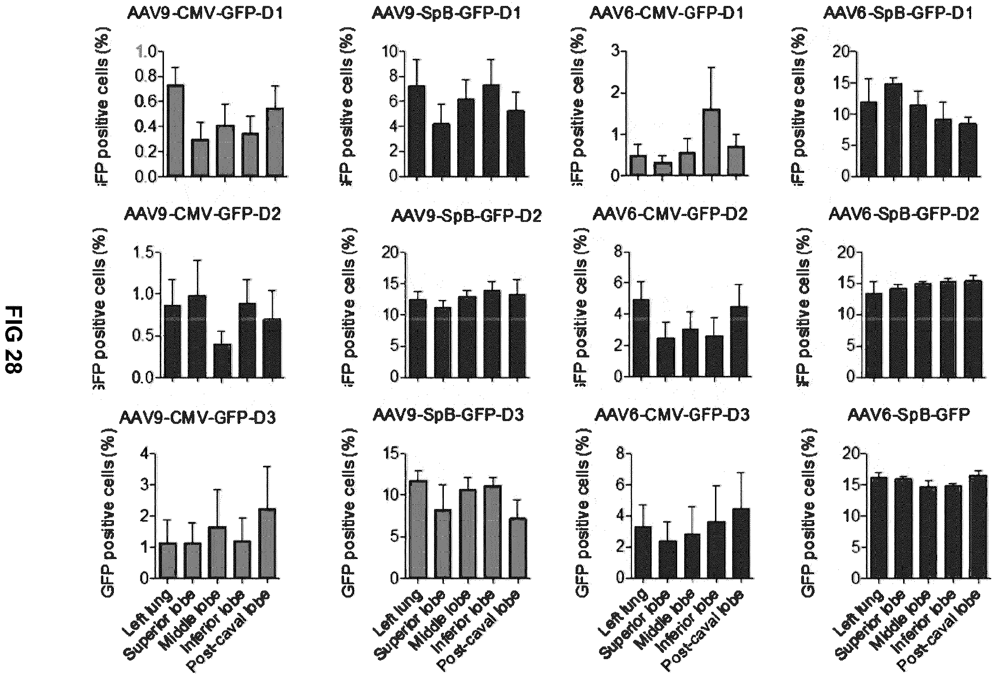

Figure 28. IHC analysis in lung lobes at three weeks post infection with viral vectors.
Healthy wild-type mice are intratraqueally administered with the vectors AAV9-CMV-null (Batch 729, UAB), AAV9-CMV-GFP (Batch 941, UAB), AAV9-SpB-GFP (Batch 893, UAB), AAV6-CMV-null (Batch 942, UAB), AAV6-CMV-GFP (Batch 943, UAB) and AAV6-SpB-GFP (Batch 944, UAB) at the 10E11 vg/mouse (D1), 5x10E11 vg/mouse (D2) and 10E12 vg/mouse (D3) doses. Mice are sacrificed at 3 weeks post-infection. The percentage of GFP positive cells is evaluated in the five lung lobes; right superior, right middle, right inferior, post-caval and left lobes by IHQ.

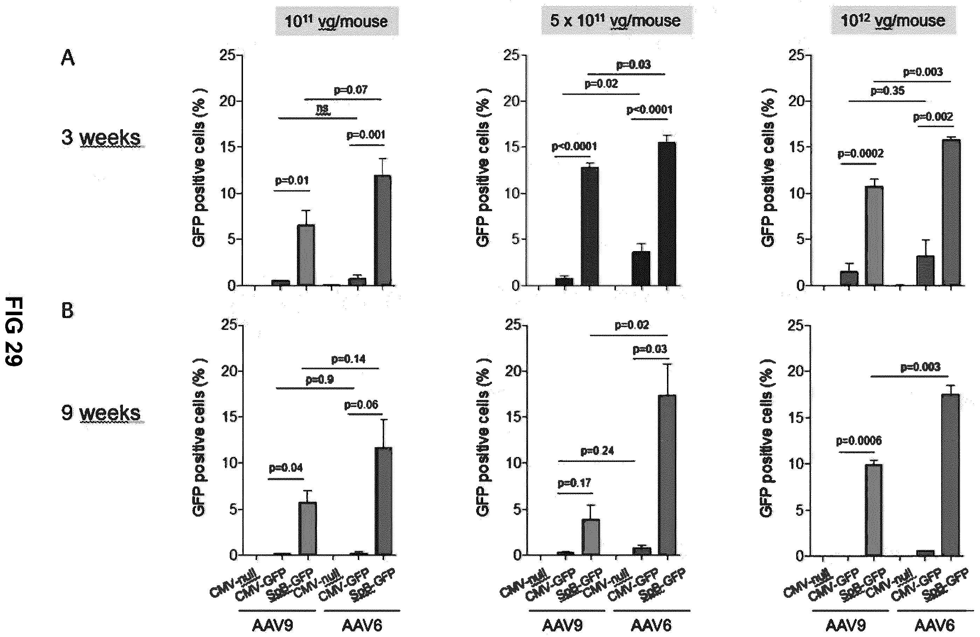

**Figure 29. IHC analysis in lung samples at three and nine weeks post infection with viral vectors.**
Healthy wild-type mice are intratraqueally administered with the vectors AAV9-CMV-null (Batch 729, UAB), AAV9-CMV-GFP (Batch 941, UAB), AAV9-SpB-GFP (Batch 893, UAB), AAV6-CMV-null (Batch 942, UAB), AAV6-CMV-GFP (Batch 943, UAB) and AAV6-SpB-GFP (Batch 944, UAB) at the 10E11 vg/mouse (D1), 5x10E11 vg/mouse (D2) and 10E12 vg/mouse (D3) doses. Mice are sacrificed at 3 (data represented in figure a)) and 9 (data represented in figure b)) weeks post-infection. The percentage of GFP positive cells is evaluated the lungs by IHQ.

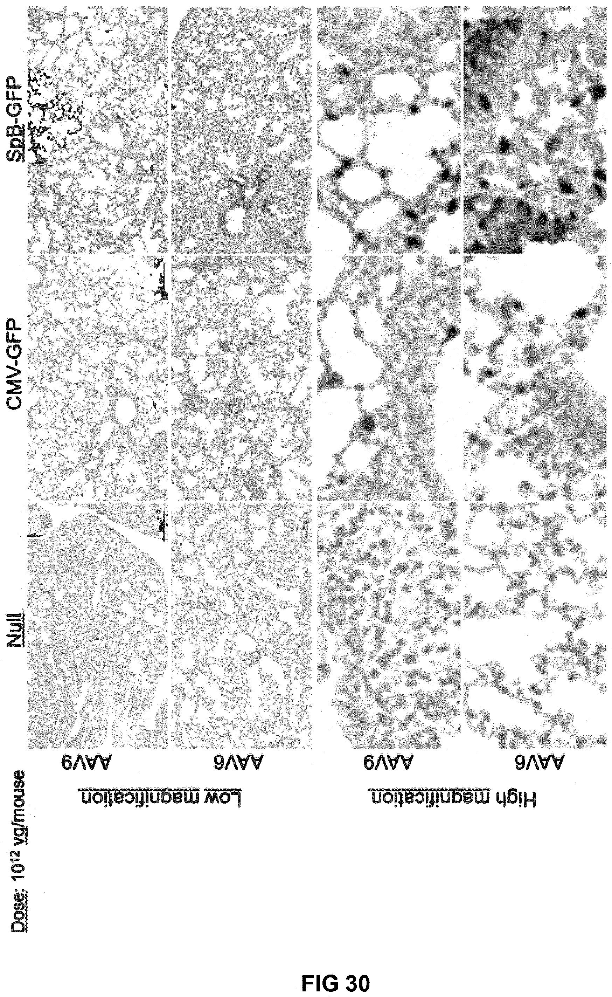

**Figure 30. Representative images of GFP IH**
Representative IHQ images of GFP staining in lungs transduced either with AAV9-CMV-null (Batch 729, UAB), AAV9-CMV-GFP (Batch 941, UAB), AAV9-SpB-GFP (Batch 893, UAB), AAV6-CMV-null (Batch 942, UAB), AAV6-CMV-GFP (Batch 943, UAB) and AAV6-SpB-GFP (Batch 944, UAB) at the 10E12 vg/mouse.

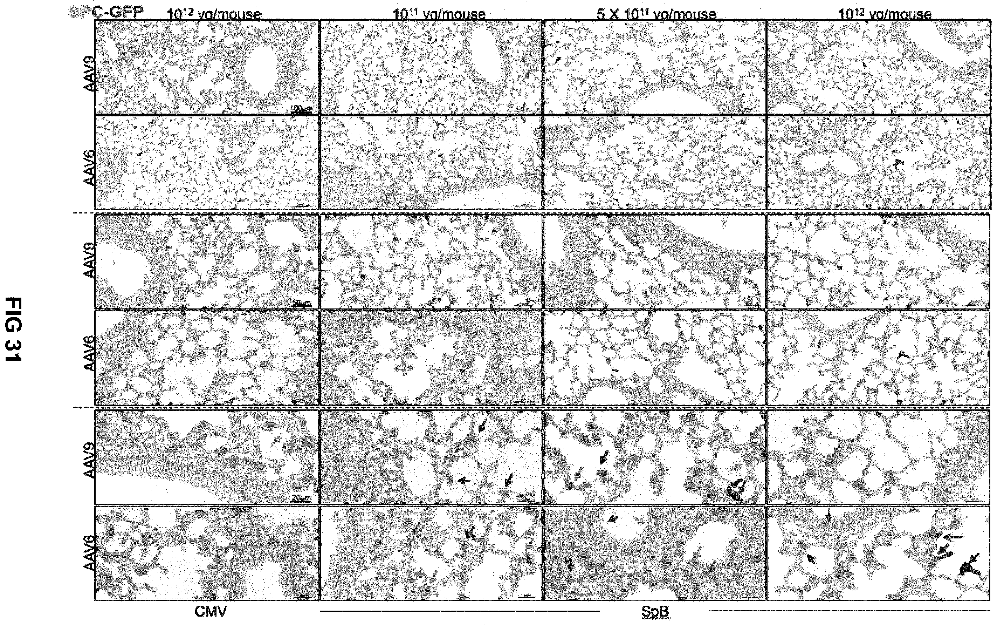

**Figure 31. Representative images of double IHC staining with anti-SpC and anti GFP**

Representative IHQ images of GFP and SpC staining in lungs transduced either with AAV9-CMV-null (Batch 729, UAB), AAV9-CMV-GFP (Batch 941, UAB), AAV9-SpB-GFP (Batch 893, UAB), AAV6-CMV-null (Batch 942, UAB), AAV6-CMV-GFP (Batch 943, UAB) and AAV6-SpB-GFP (Batch 944, UAB) at the 10E11 vg/mouse (D1), 5x10E11 vg/mouse (D2) and 10E12 vg/mouse (D3) doses. Mice are sacrificed at 9 weeks post-infection. SpC is a marker of alveolar type II cells.

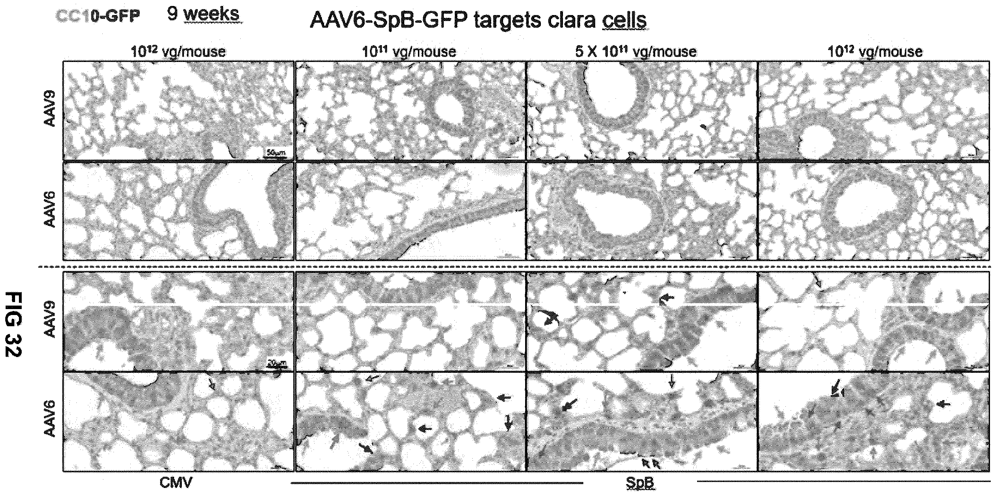

**Figure 32. Representative images of double IHC staining with anti-CC10 and anti GFP**

Representative IHQ images of GFP and CC10 staining in lungs transduced either with AAV9-CMV-null (Batch 729, UAB), AAV9-CMV-GFP (Batch 941, UAB), AAV9-SpB-GFP (Batch 893, UAB), AAV6-CMV-null (Batch 942, UAB), AAV6-CMV-GFP (Batch 943, UAB) and AAV6-SpB-GFP (Batch 944, UAB) at the 10E11 vg/mouse (D1), 5x10E11 vg/mouse (D2) and 10E12 vg/mouse (D3) doses. Mice are sacrificed at 9 weeks post-infection. CC10 is a marker of clara cells.

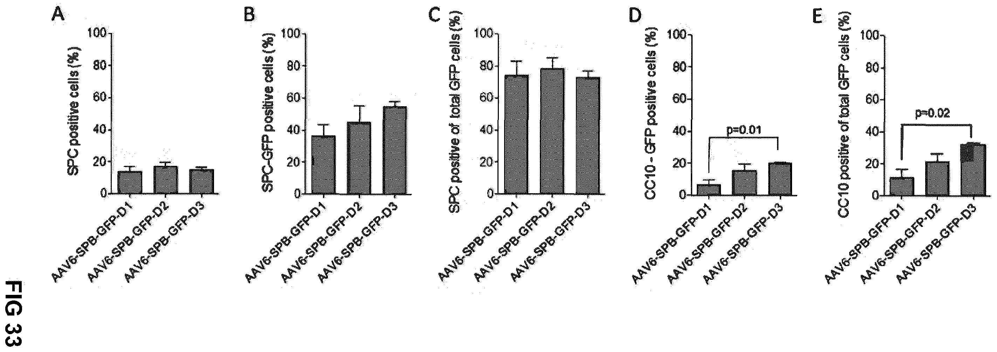

**Figure 33. IHC analysis in lung samples at nine weeks post infection with viral vectors**

Quantification of the percentage of SPC positive cells(A), double SPC-GFP positive cells (B), the percentage of SPC positive of total GFP positive cells (C), double CC10-GFP positive cells (D) and the percentage of CC10 positive cells of total GFP positive cells (E) in lung samples after 9 weeks post-infection with AAV6-SpB-GFP (Batch 944, UAB) at the 10E11 vg/mouse (D1), 5x10E11 vg/mouse (D2) and 10E12 vg/mouse (D3) doses.

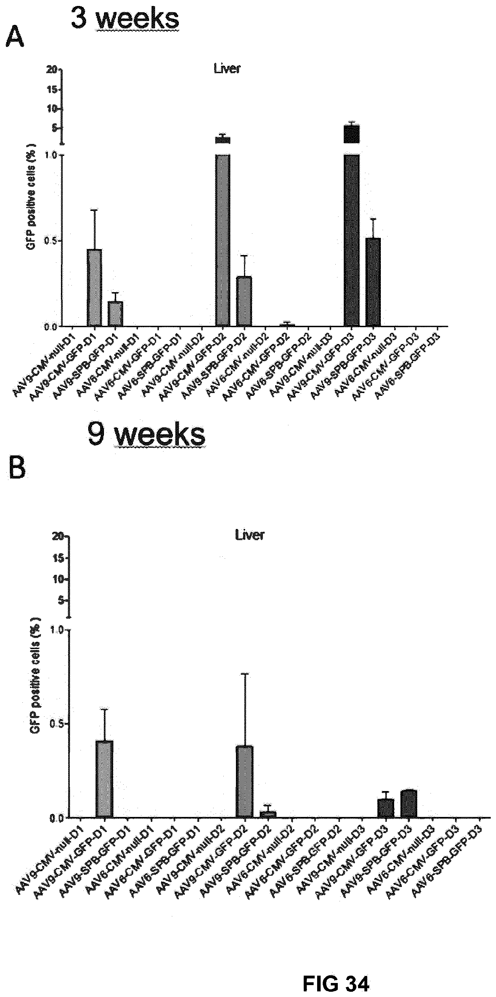

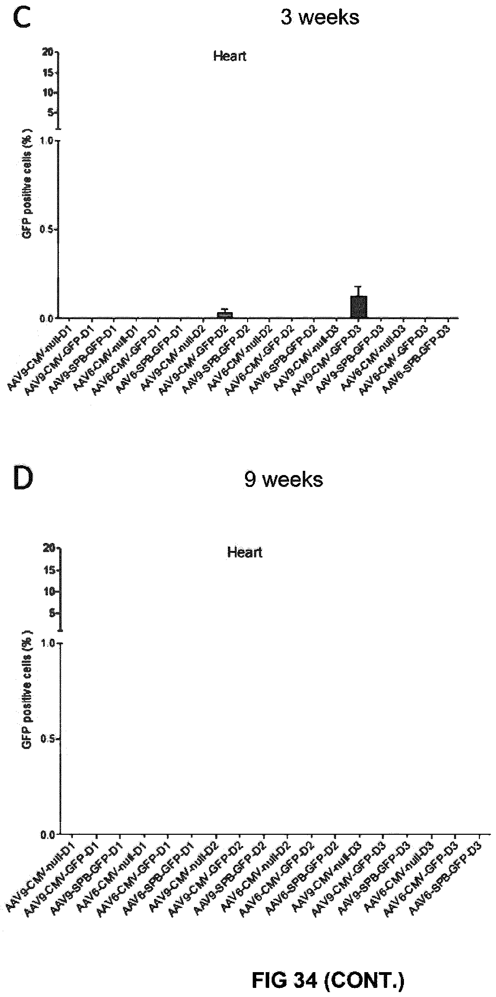

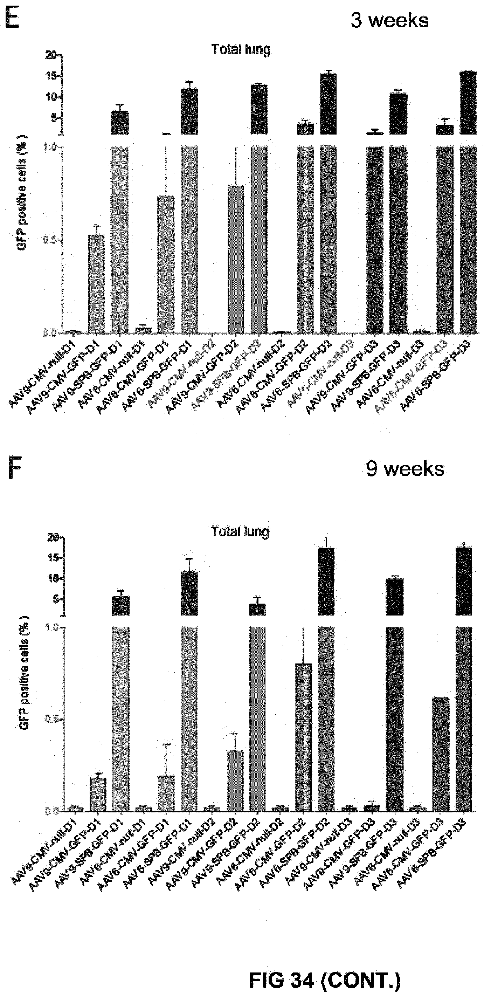

**Figure 34. IHC analysis in lung, liver a heart samples at 3 and 9 weeks post-infection with viral vectors.**

Healthy wild-type mice are intratraqueally administered with the vectors AAV9-CMV-null (Batch 729, UAB), AAV9-CMV-GFP (Batch 941, UAB), AAV9-SpB-GFP (Batch 893, UAB), AAV6-CMV-null (Batch 942, UAB), AAV6-CMV-GFP (Batch 943, UAB) and AAV6-SpB-GFP (Batch 944, UAB) at the 10E11 vg/mouse (D1), 5x10E11 vg/mouse (D2) and 10E12 vg/mouse (D3) doses. Mice are sacrificed at 3 and 9 weeks post-infection. The percentage of GFP positive cells is evaluated in liver (A,B), heart (C,D) and in the lungs (E,F) by IHQ.

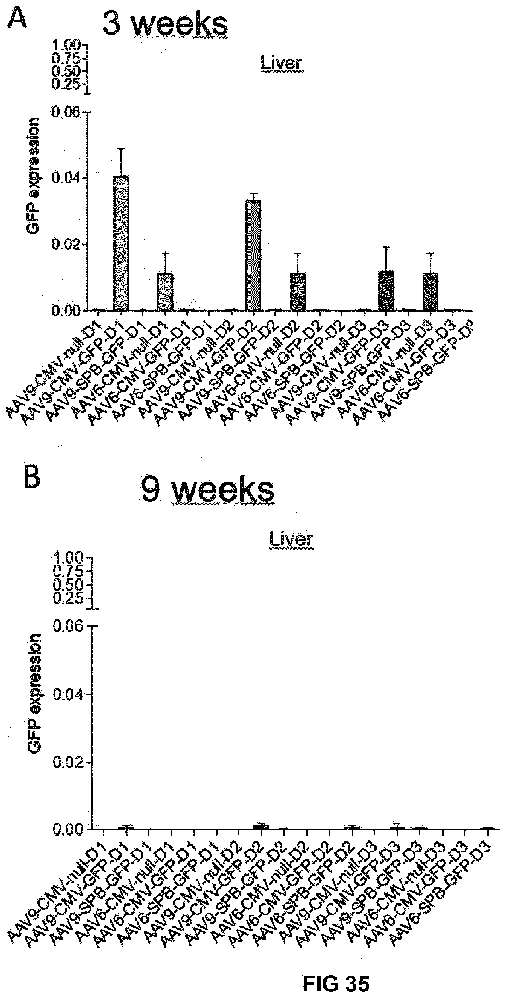

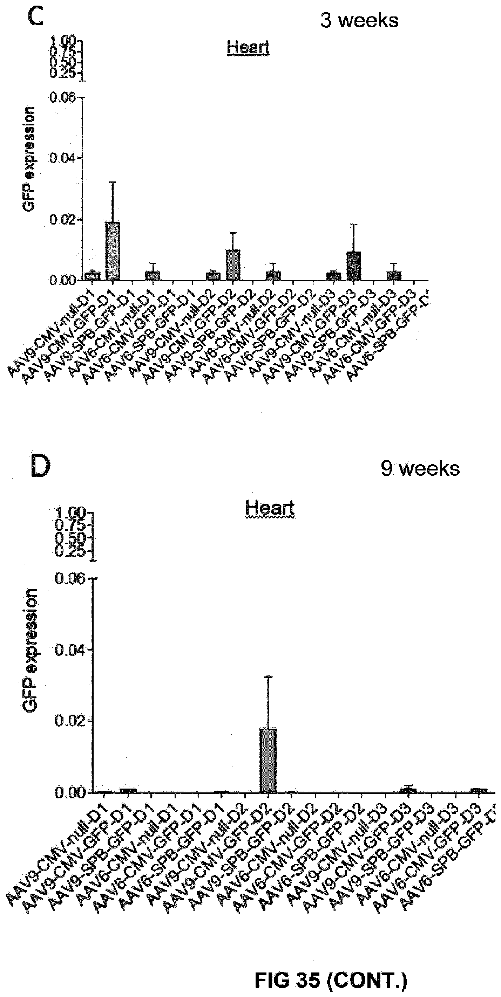

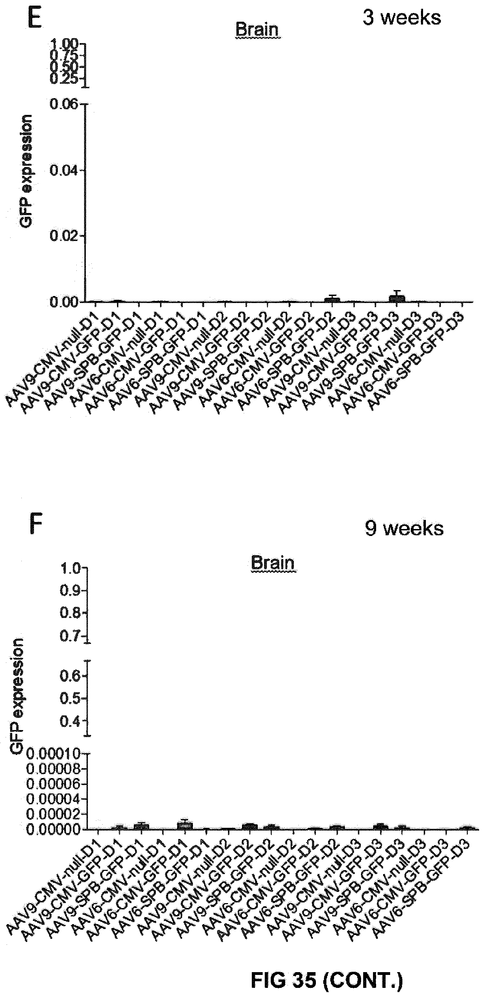

**Figure 35. GFP expression levels in liver, heart and brain samples by q-RT-PCR at 3 and 9 weeks post-infection with viral vectors.**

Healthy wild-type mice are intratraqueally administered with the vectors AAV9-CMV-null (Batch 729, UAB), AAV9-CMV-GFP (Batch 941, UAB), AAV9-SpB-GFP (Batch 893, UAB), AAV6-CMV-null (Batch 942, UAB), AAV6-CMV-GFP (Batch 943, UAB) and AAV6-SpB-GFP (Batch 944, UAB) at the 10E11 vg/mouse (D1), 5x10E11 vg/mouse (D2) and 10E12 vg/mouse (D3) doses. Mice are sacrificed at 3 and 9 weeks post-infection. GFP expression is evaluated in liver (A,B), heart (C,D) and in the lungs (E,F) by qPCR.

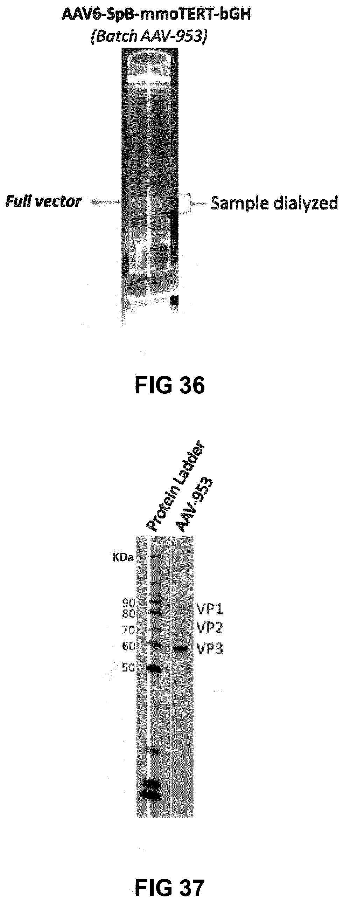

**Figure 36.** Isopycnic CsCl gradient of the AAV6-SpB-moTERT-bGH vector batch AAV-953.

**Figure 37.** Protein staining (Sypro Ruby staining) of the AAV-953 batch. VP, viral particles.

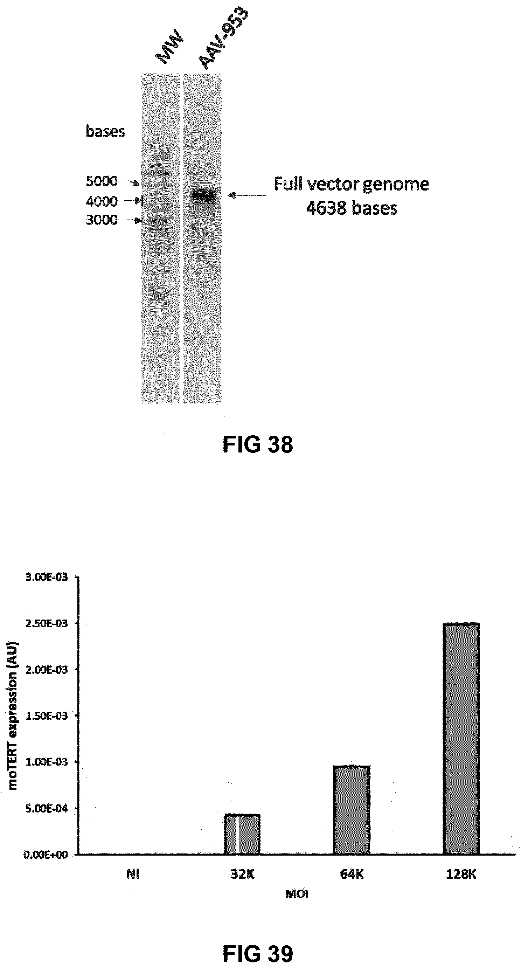

**Figure 38.** Alkaline agarose gel of the AAV-953 batch. MW, molecular weight.

**Figure 39.** moTERT mRNA expression. Mouse optimized TERT mRNA expression levels in 2v6.11 cells infected with vector AAV-953 at three different MOIs (32000 vg/cell (32K), 64000 vg/cell (64K) and 128000 vg/cell (128K)). NI, non-infected. AU, arbitrary units.

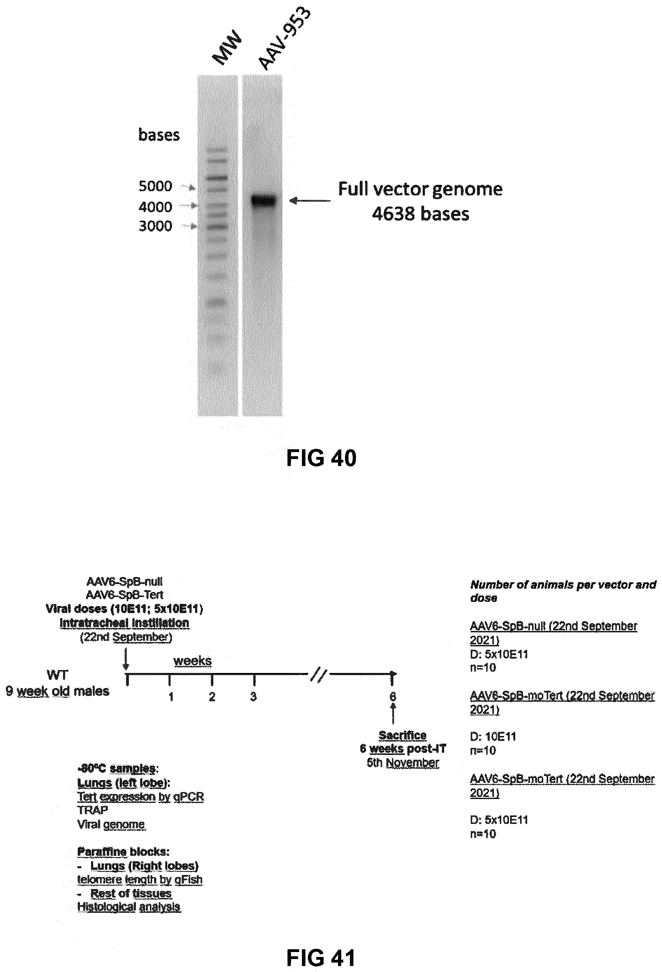

**Figure 40.** Alkaline agarose gel of AAV6-SpB-moTERT (AAV-953 batch). MW, molecular weight.

**Figure 41. Experimental lay-out**

Healthy wild-type mice (9 weeks old) are intratraqueally administered with the AAV6-SpB-null at $5x10^{11}$ vg/mouse (D2) and with AAV6-SpB-moTert at $1x10^{11}$ (D1) and at $5x10^{11}$ vg/mouse (D2). Ten mice are included per group. Mice are sacrificed at 6 weeks post-infection.

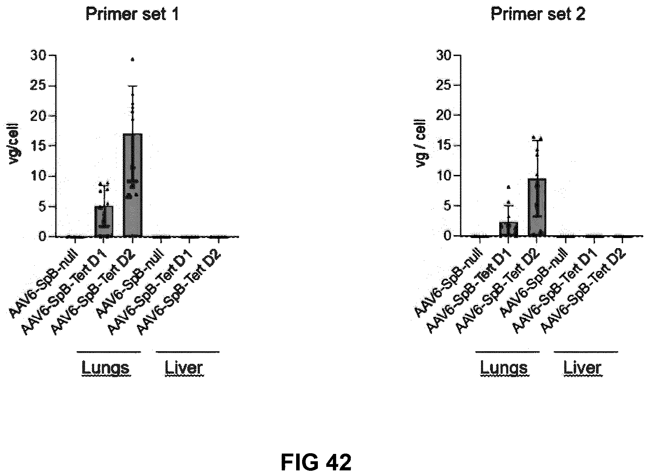

**Figure 42. Viral genome quantification**

Healthy wild-type mice (9 weeks old) are intratraqueally administered with the AAV6-SpB-null at $5x10^{11}$ vg/mouse (D2) and with AAV6-SpB-moTert at $1x10^{11}$ (D1) and at $5x10^{11}$ vg/mouse (D2). Ten mice are included per group. Mice are sacrificed at 6 weeks post-infection. The number of viral genomes per cell in lung and liver samples was analyzed by qPCR using two different primer sets, primer set 1 and 2.

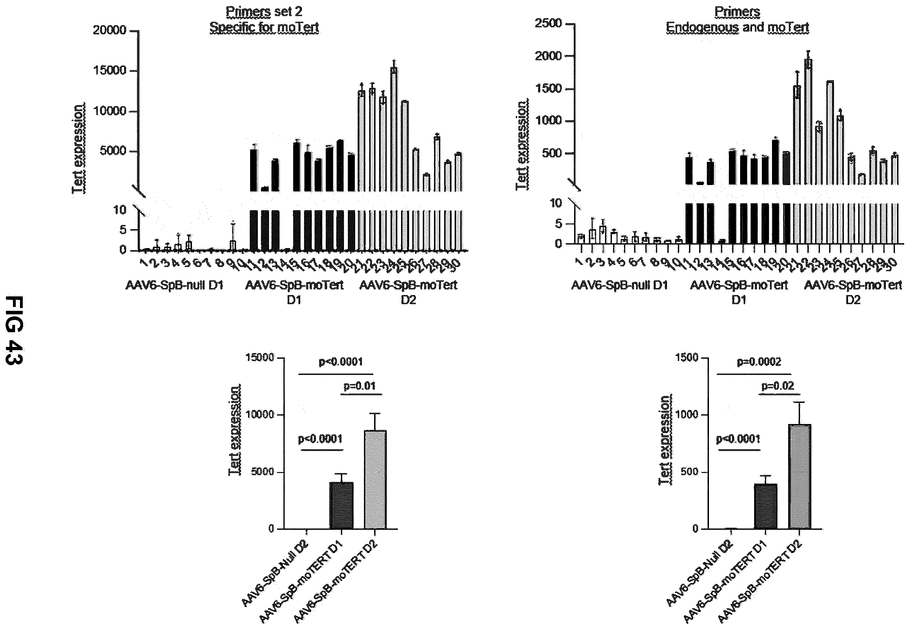

### Figure 43. Tert expression in lungs

Healthy wild-type mice (9 weeks old) are intratraqueally administered with the AAV6-SpB-null at $5x10^{11}$ vg/mouse (D2) and with AAV6-SpB-moTert at $1x10^{11}$ (D1) and at $5x10^{11}$ vg/mouse (D2). Ten mice are included per group. Mice are sacrificed at 6 weeks post-infection. The transcriptional expression levels of Tert in the lungs are analysed by q-RT-PCR using two different sets of primers, set 1 and set 2. Set 1 is composed of the forward (5'- TAG CCC TGG CAA GGA TAG A -3') and the reverse primer (5'- GCT CTC GGT GAT GTA GAA GAA G -3'). Set 2 is composed of the forward primer (5'- CAC CAG ACA CAA CGA GAG AAG -3') and the reverse primer (5'- CTC GAC CTT CAT CTT CCA CAT C -3').

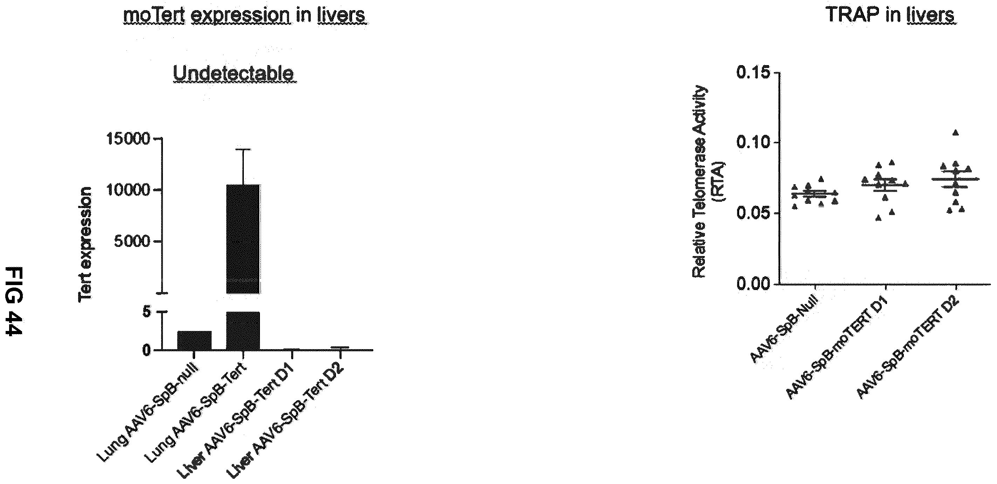

### Figure 44. Tert expression and activity in off-target organs

Healthy wild-type mice (9 weeks old) are intratraqueally administered with the AAV6-SpB-null at $5x10^{11}$ vg/mouse (D2) and with AAV6-SpB-moTert at $1x10^{11}$ (D1) and at $5x10^{11}$ vg/mouse (D2). Ten mice are included per group. Mice are sacrificed at 6 weeks post-infection. The transcriptional expression levels of Tert in the livers are analysed by q-RT-PCR using primer set 1. Set 1 is composed of the forward (5'- TAG CCC TGG CAA GGA TAG A -3') and the reverse primer (5'- GCT CTC GGT GAT GTA GAA GAA G -3') (left panel). Telomerase activity in liver samples is analyzed by q-PCR-based telomeric repeat amplification protocol (TRAP) (right panel).

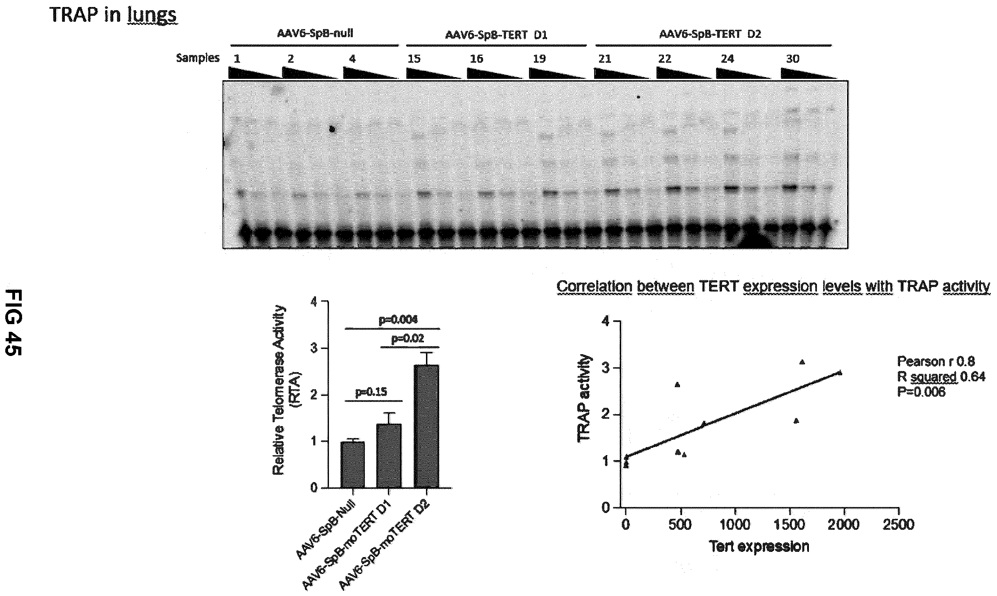

### Figure 45. Telomerase activity in lungs.

Healthy wild-type mice (9 weeks old) are intratraqueally administered with the AAV6-SpB-null at $5x10^{11}$ vg/mouse (D2) and with AAV6-SpB-moTert at $1x10^{11}$ (D1) and at $5x10^{11}$ vg/mouse (D2). Mice are sacrificed at 6 weeks post-infection. Telomerase activity in lung samples is analyzed by telomeric repeat amplification protocol (TRAP). A correlation between telomerase expression and telomerase activity in lung samples is shown,

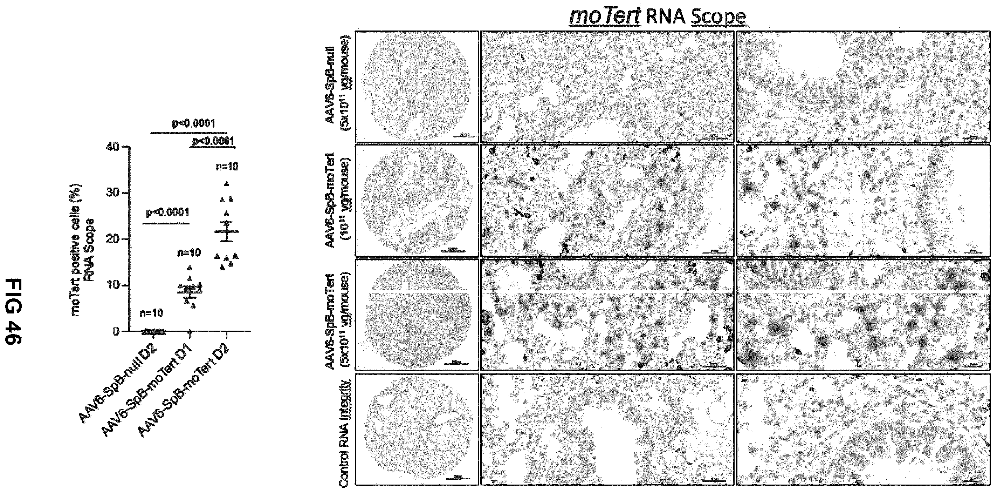

### Figure 46. moTert RNA Scope

Healthy wild-type mice (9 weeks old) are intratraqueally administered with the AAV6-SpB-null at $5x10^{11}$ vg/mouse (D2) and with AAV6-SpB-moTert at $1x10^{11}$ (D1) and at $5x10^{11}$ vg/mouse (D2). Mice are sacrificed at 6 weeks post-infection. The percentage of lung cells expressing moTert is analysed by moTert RNA scope positive cells. A specific probe for moTert RNA was designed and purchased from ACDBIO (www.acdbio.com) to analyze lung transduction efficacy.

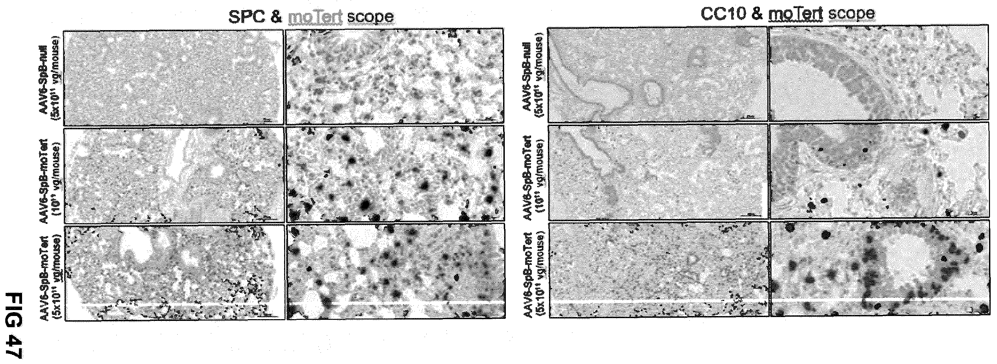

### Figure 47. AAV6-SpB-moTert is exclusively expressed in ATII and clara cells

Healthy wild-type mice (9 weeks old) are intratraqueally administered with the AAV6-SpB-null at $5x10^{11}$ vg/mouse (D2) and with AAV6-SpB-moTert at $1x10^{11}$ (D1) and at $5x10^{11}$ vg/mouse (D2). Mice are sacrificed at 6 weeks post-infection. Representative images of double immune staining with Prosurfactant Protein C (SP-C) and moTert RNA scope and of Secretoglobin Family 1A Member 1 (SCGB1A1 or CC10) and moTert RNA scope in lung samples.

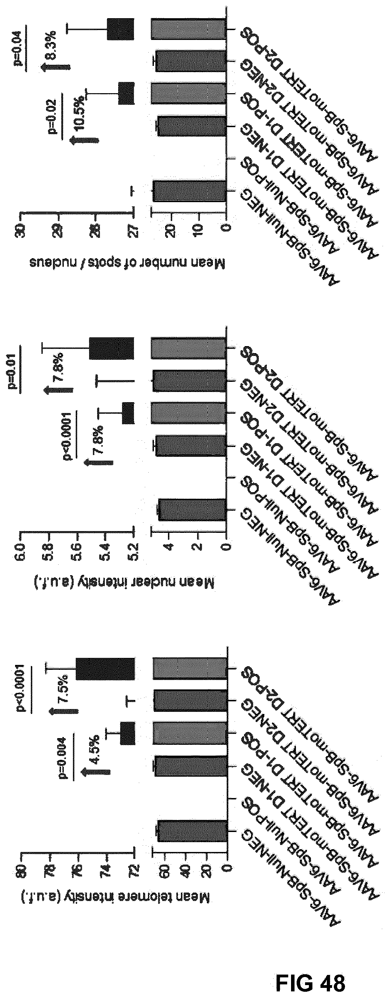

### Figure 48. AAV6-SpB-moTert lengthens the telomeres and leads to a higher number of telomeric spots per nucleus in transduced cells in six weeks.

Healthy wild-type mice (9 weeks old) are intratraqueally administered with the AAV6-SpB-null at $5x10^{11}$ vg/mouse (D2) and with AAV6-SpB-moTert at $1x10^{11}$ (D1) and at $5x10^{11}$ vg/mouse (D2). Mice are sacrificed at 6 weeks post-infection. Double fluorescence in situ hybridization with the moTert RNA scope and a telomeric probe (q-Fish) was performed to analyze telomere length in cells expressing the moTert in lung samples. Mean telomere intensity, mean nuclear intensity and mean number of telomeric spots per nucleus in cells not expressing moTert and in cells expressing moTert are shown. Results are expressed as mean + SEM. n=10 mice.

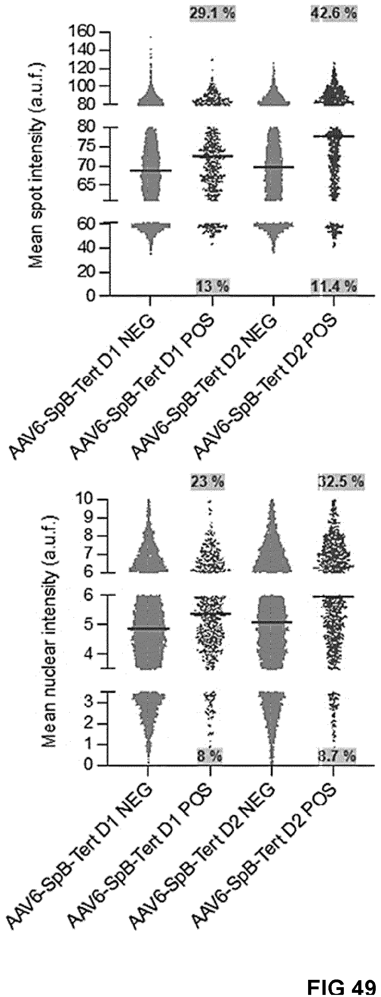

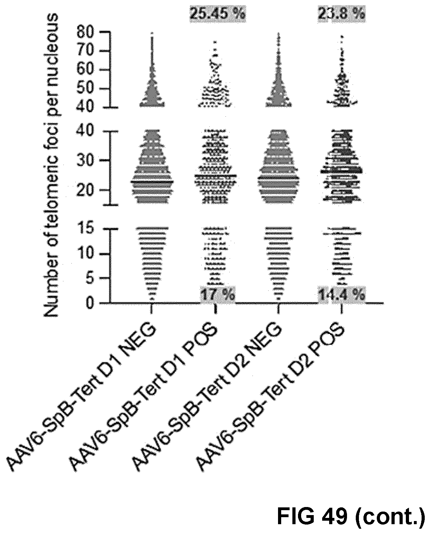

### Figure 49. AAV6-SpB-Tert Transduced mice. Telomeric distributions: 20% and 80 % percentile.

Healthy wild-type mice (9 weeks old) are intratraqueally administered with the AAV6-SpB-null at $5x10^{11}$ vg/mouse (D2) and with AAV6-SpB-moTert at $1x10^{11}$ (D1) and at $5x10^{11}$ vg/mouse (D2). Mice are sacrificed at 6 weeks post-infection. Double fluorescence in situ hybridization with the moTert RNA scope and a telomeric probe (q-Fish) was performed to analyze telomere length in cells expressing the moTert in lung samples. Mean telomere intensity, mean nuclear intensity and mean number of telomeric spots per nucleus in cells not expressing moTert and in cells expressing moTert in the two experimental groups (AAV6-SpB-moTert -D1 and AAV6-SpB-moTert -D2 were pulled to calculate the 20% and 80% percentile as regards to untransduced cells (Tert RNA scope negative cells.

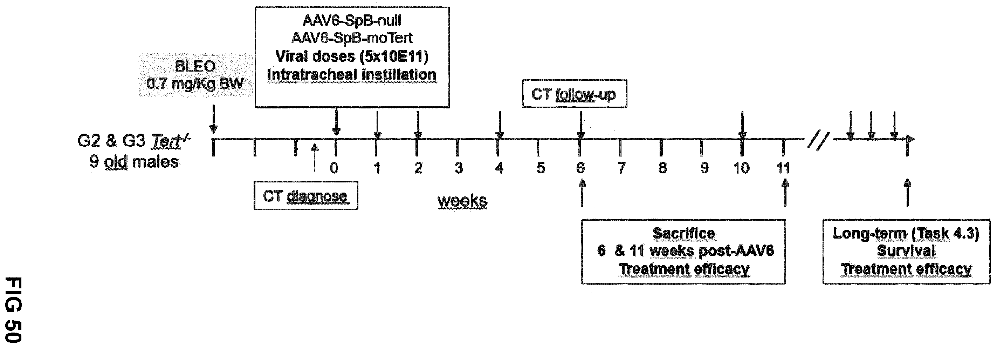

### Figure 50. Evaluation of therapeutic efficacy of by AAV6-SpB-moTERT in the treatment of pulmonary fibrosis induced by short telomeres. Experimental lay-out.

Bleomycin (0.7 mg/kg body weight) was instilled intratracheally in 8-week-old G2 & G3-Tert-/- male mice. Mice diagnosed with pulmonary fibrosis were intratracheally (IT) administered either with $5 \times 10^{11}$ vg of AAV-CMV-null or with $5 \times 10^{11}$ vg AAV6-SpB-moTERT vg per mouse. Disease progression was followed by CT-scans. CT scans were performed at 1, 2, 4, 6 weeks post-infection to measure the decrease in the fibrotic lung volume relative to the start of the treatment. Mice are sacrificed either at 6 or 11 weeks post-infections. Ten mice per group are left for long term follow-up.

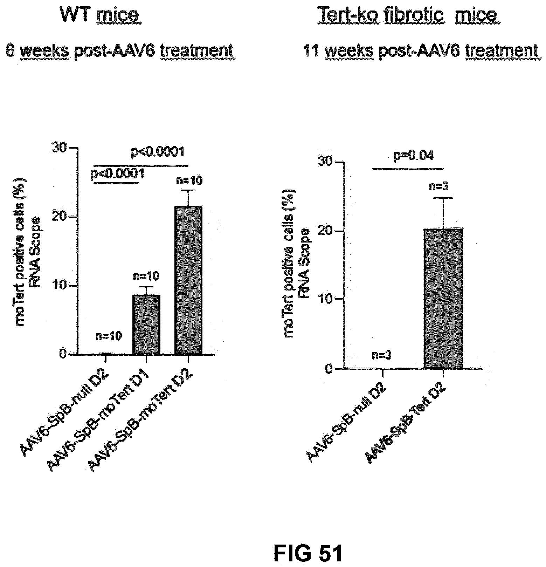

**Figure 51. Lung transduction efficacy of AAV6-SpB-moTert in fibrotic lungs by RNA scope**

Mice diagnosed with pulmonary fibrosis were intratracheally (IT) administered either with $5 \times 10^{11}$ vg of AAV-CMV-null or with $5 \times 10^{11}$ vg AAV6-SpB-moTERT vg per mouse. The percentage of lung cells expressing moTert is analysed by moTert RNA scope positive cells in lung samples after 11 weeks post-infection (right panel). For comparison purposes the percentage of lung cells in wild type healthy animals transduced with AAV6-SpB-null at $5 \times 10^{11}$ vg/mouse (D2) and with AAV6-SpB-moTert at $1 \times 10^{11}$ (D1) and at $5 \times 10^{11}$ vg/mouse (D2) after 6 weeks post-infection is shown (left panel).

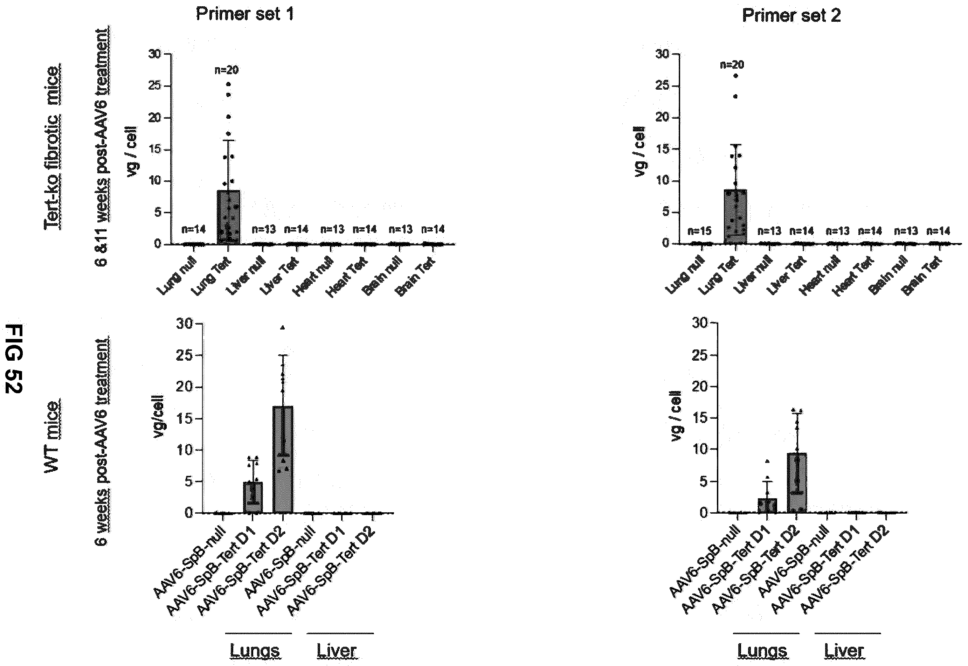

**Figure 52**. **Viral genome quantification**

Mice diagnosed with pulmonary fibrosis were intratracheally (IT) administered either with $5 \times 10^{11}$ vg of AAV-CMV-null or with $5 \times 10^{11}$ vg AAV6-SpB-moTERT vg per mouse. The number of viral genomes per cell in lung, liver, heart and brain samples was analyzed by qPCR using two different primer sets, primer set 1 and 2. Samples correspond to mice sacrificed at 6 and 11 weeks post infection (upper panels). For comparison purposes the number of viral genomes per cell in lung and liver samples of wild type healthy animals transduced with AAV6-SpB-null at $5 \times 10^{11}$ vg/mouse (D2) and with AAV6-SpB-moTert at $1 \times 10^{11}$ (D1) and at $5 \times 10^{11}$ vg/mouse (D2) after 6 weeks post-infection is shown (lower panels)

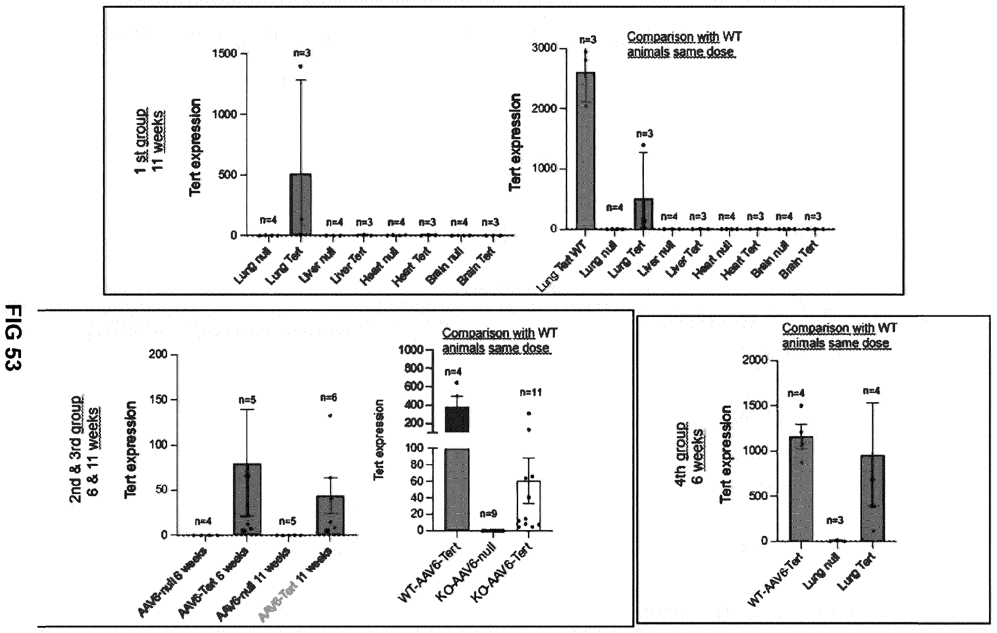

**Figure 53. Tert expression-qPCR**

Mice diagnosed with pulmonary fibrosis were intratracheally (IT) administered either with $5 \times 10^{11}$ vg of AAV-CMV-null or with $5 \times 10^{11}$ vg AAV6-SpB-moTERT vg per mouse. Telomerase expression is evaluated in lungs, liver, heart and brain samples by qPCR at 11 weeks post infection (upper panel). Telomerase expression is evaluated in lungs at 6 and 11 weeks post-infection (lower left panel) and at 6 weeks post-infection (lower right panel). The three plots correspond to three different and independent PCR runs analyzing individual independent mice. Lung samples of wild type healthy mice transduced with $5 \times 10^{11}$ vg AAV6-SpB-moTERT vg were run in parallel (first bar) as positive control.

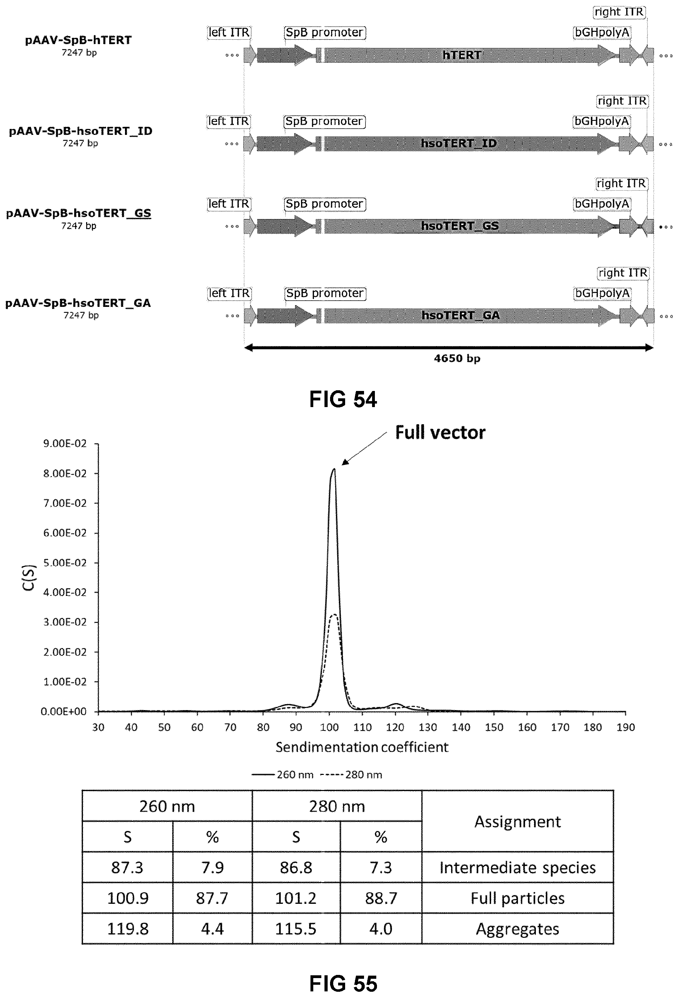

**Figure 54.** The four different TERT sequences cloned in the AAV vector. The 4650bp AAV vector genome comprises the SpB promoter, the corresponding TERT CDS, and the bGH PolyA signal all flanked by the two AAV2 ITR.

**Figure 55.** Sedimentation profile obtained by analytical ultracentrifugation of AAV6-SpB-hoTERT-ID (AAV-962 vector batch). The x axis represents the sedimentation coefficient expressed in Svedberg units (S), and the y axis represents the normalized value of the concentration as a function of S (c(S)) measured at 260 nm (solid line) and 280 nm (dashed line).

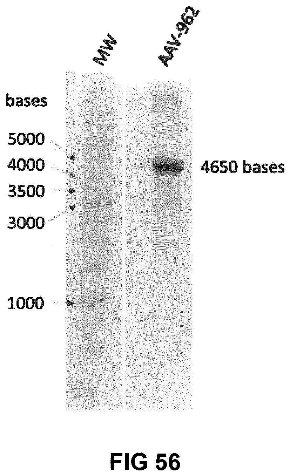

**Figure 56.** Alkaline agarose gel of AAV6-SpB-hoTERT-ID (AAV-962 batch). MW, molecular weight.

**Examples**

General procedures to the Example 1

*Subject characteristics*

For evaluation of tissue-specificity of shortened human SpB, CMVenh-MLC2v and TNT promoters, male C57Bl/6J mice were used. Mice were fed *ad libitum* with a standard diet (2018S Teklad Global Diets®, Harlan Labs., Inc., Madison, WI, US) and kept under a light-dark cycle of 12 h (lights on at 8:00 a.m.) and stable temperature (22°C ± 2). Prior to tissue sampling, mice were anesthetized by means of inhalational anesthetic isoflurane (IsoFlo®, Abbott Laboratories, Abbott Park, IL, US) and decapitated. Tissues of interest were excised and kept at -80°C or with formalin until analysis. All experimental procedures were approved by the Ethics Committee for Animal and Human Experimentation of the Universitat Autònoma de Barcelona.

*Recombinant AAV vectors*

Single-stranded AAV vectors of serotype 9 were produced by triple transfection of HEK293 cells according to standard methods (Ayuso, E. et al., 2010. Curr Gene Ther. 10(6):423-36). Cells were cultured in 10 roller bottles (850 cm$^2$, flat; Corning™, Sigma-Aldrich Co., Saint Louis, MO, US) in DMEM 10% FBS to 80% confluence and co-transfected using the calcium phosphate method with a plasmid carrying the expression cassette flanked by the AAV2 ITRs, a helper plasmid carrying the AAV2 *rep* gene and the AAV of serotype 9 cap gene, and a plasmid carrying the adenovirus helper functions. Transgenes used were: mouse codon-optimized (SEQ ID NO: 3) TERT coding-sequence driven by 1) the shortened human SpB promoter (SEQ ID NO: 12); 2) the CMV enhancer fused to shortened human MLC2v promoter (SEQ ID NO: 15); or 3) the shortened human TNT promoter (SEQ ID NO: 16). Wild-type mouse TERT coding sequence including 3' UTR (SEQ ID NO: 1) driven by the ubiquitous CMV promoter was used as control (Bernardes de Jesus, B. et al., 2012. EMBO Mol Med 4(8):691-704). A noncoding plasmid was used to produce null vectors. AAV were purified with an optimized method based on a polyethylene glycol precipitation step and two consecutive cesium chloride (CsCl) gradients. This second-generation CsCl-based protocol reduced empty AAV capsids and DNA and protein impurities dramatically (Ayuso, E. et al., 2010. Curr Gene Ther. 10(6):423-36). Purified AAV vectors were dialyzed against PBS, filtered and stored at - 80°C. Viral vectors were determined by fluorescence using the Quant-iT™ PicoGreen™ dsDNA Assay Kit (Invitrogen). A phage lambda DNA was used as standard curve to calculate the titer of viral vectors. The vectors were constructed according to molecular biology techniques well known in the art.

*Systemic administration of AAV vectors*

The appropriate amount of the AAV solution was diluted in PBS with 0.001% Pluronic® and was manually injected into the lateral tail vein without exerting pressure at the moment of delivery. Before the injection, the animals were put under a 250 W infrared heat lamp (Philips NV, Amsterdam, NL) for a few minutes to dilate the blood vessels and facilitate viewing and easier access to the tail vein. A plastic restrainer (Harvard Apparatus, Holliston, MA, US) was used to secure the animal for injection. No anesthesia was used since an appropriate restraining device was employed. A 30-gauge needle was utilized to inject the animals.

*RNA analysis*

Total RNA was obtained from different tissues using isolation reagents (Tripure, Roche, for lung, liver and heart samples and QIAzol, Qiagen, for eWAT samples), and the RNeasy Tissue Minikit (Qiagen NV, Venlo, NL), according to the manufacturer's protocol. In order to eliminate residual viral genomes, total RNA was treated with DNAseI (Qiagen NV, Venlo, NL). The concentration and purity of the obtained RNA was determined using a Nanodrop (ND-1000, Thermo-Cientific). For RT-PCR, 1 μg of RNA samples was reverse-transcribed using the Transcriptor First Strand cDNA Synthesis Kit (04379012001, Roche, California, USA), according to the manufacturer's protocol. Real-time quantitative PCR was performed in a SmartCyclerII® (Cepheid, Sunnyvale, USA) using LightCycler 480 SYBR Green I Master (Roche, ref. 04887352001, Roche Diagnostics, Germany). Data was normalized with Rplp0 values and analyzed as previously described (Pfaffl, M., Nucleic Acids Res. 2001; 29(9):e45).

*Characterization of AAV vectors by sedimentation velocity analytical ultracentrifugation*

AAV vectors were characterized by analytical ultracentrifugation (AUC). Four hundred and fifty microliters of sample were loaded into the sample sector of a two-sector velocity cell, and 450 μl of PBS + 0.001% PF68 was loaded into the corresponding reference sector (Beckman-Coulter analytical ultracentrifuge Optima XLA equipped with UV-VIS absorbance optics).

Sedimentation velocity centrifugation was performed at 20,000 rpm. Particle sedimentation was recorded at 260 nm and 280 nm in the same run. The relative percentage of each peak in the C(S) distribution was calculated based on the molar concentration of each species in relation to the sum of the molar concentration of all species in the distribution as previously described (Burnham, B. et al., 2015, Hum Gene Ther Methods 26(6):228-42).

*Determination of AAV vector genome integrity by alkaline agarose gel electrophoresis*

AAV vector genome integrity was assessed by alkaline agarose gel electrophoresis (denaturing conditions) where single stranded DNA can be analyzed. First, 8 μg of vector genome was purified with the DNeasy blood and tissue kit (Qiagen), according to the manufacturer's protocol. Extracted DNA (~1 μg) was mixed with alkaline loading buffer and boiled to 95 °C for 5 min and incubated on ice for 3 min. Next, DNA was subjected to agarose gel electrophoresis under denaturing conditions. Finally, band size and quality were examined to assess genome integrity.

*Visualisation of expression*

GFP positive cells in heart, brown adipose tissue (BAT), epididymis, intestine, kidney, liver, lungs, muscle, pancreas, spleen, testis and white adipose tissue (WAT) were detected by immunohistochemistry staining using a rabbit monoclonal antibody against GFP (D5.1, Cell Signaling, #2956) and visualised by light microscopy.

*Computerized tomography*

In-vivo lung imaging by computerized tomography (CT) was made on a high-resolution CT system (CT Locus, GE Healthcare) specifically designed for small laboratory animals. Mice were anesthetized with a 4% rate of isoflurane (IsoVet Braun) during the induction and 2% during the maintenance period (scanning time). Micro-CT image acquisition consisted of 400 projections collected in one full rotation of the gantry in approximately 14 min in a single bed focused on the legs, with a 450 $\mu$A/80kV X-ray tube. 2-D and 3-D images were obtained and analysed using the software program MicroView (GE Healthcare).

*Statistical analysis*

All results are expressed as mean $\pm$ SEM. Differences between groups were compared by Student's t-test. Statistical significance was considered if $P < 0.05$.

Example 1.1. AAV-mediated lung-specific transgene expression using a short version of the human SpB promoter

The long length of TERT sequences typically used in the art in conjunction with the low encapsidation capacity of AAV vectors (approximately 4.7 kb) highly restrict the number of promoter sequences that can be used in the expression cassette encoding telomerase without exceeding the encapsidation capacity of AAV vectors. To not exceed the AAV encapsidation capacity caused by utilization of lung-specific promoters in conjunction with TERT coding sequences and further components of the expression cassettes (i.e. polyA sequence, AAV ITRs) a shortened version (632-nucleotides, SEQ ID NO: 12) of the human surfactant protein B (SpB) promoter (size of the wild-type human SpB promoter: 1597-nucleotides, SEQ ID NO: 11) was designed. The wild-type human SpB promoter drives expression in Clara and ATII cells in the lung.

To study whether the shortened human SpB promoter was be able to mediate lung-specific transgene expression, AAV9 vectors encoding the marker protein GFP under the control of this shortened promoter (AAV9-SpB-GFP) or under the control of the ubiquitous CMV promoter (AAV9-CMV-GFP) were cloned and produced by triple transfection in HEK293 producer cells. A dose of $1.75 \times 10^{12}$ viral genomes (vg) of AAV9-SpB-GFP or AAV9-CMV-GFP vectors was administered intravenously (IV) to mice via tail vein. A group of mice administered IV with saline solution was used as control. Four weeks post-AAV administration, GFP expression levels were evaluated in lungs, liver and epididymal white adipose tissue (eWAT). Although GFP expression levels in lungs were lower than those mediated by the CMV promoter (Figure 1A), the shortened version of the SpB promoter prevented GFP expression in white adipose tissue and mediated 80% reduction of GFP expression levels in the liver (Figure 1B). Quantification of the percentage of GFP+ cells in lungs, liver, heart and brown adipose tissue (BAT), further confirmed the lung-specificity of the shortened SpB promoter (Figures 2 and 3). The use of shortened promoters could result in loss of specificity and promiscuous transgene expression in off-target tissues other than the lungs. Surprisingly, the shortened promoter used herein was able to mediate highly-specific lung expression.

Example 1.2. AAV-mediated heart-specific transgene expression using shortened heart-specific promoters

A shortened (296-nucleotides, SEQ ID NO: 13) version of the cardiomyocyte-specific MLC2v promoter was also designed. In order to increase the promoter strength of this shortened version of the wild type MLC2v promoter, the CMV enhancer (380-nucleotides, SEQ ID NO: 14) was used in conjunction with said promoter (CMVenh-MLC2v, SEQ ID NO: 15).

To study whether the CMVenh-MLC2v promoter was capable of mediating specific expression in the heart, 8-week-old male C57Bl6 mice were treated with two different doses ($5 \times 10^{11}$ (low (L) dose) or $2 \times 10^{12}$ (high (H) dose) viral genomes (vg)/mouse) of AAV9-CMVenh-MLC2v-GFP vectors. As controls, two additional groups of mice were treated with the same doses of AAV9 vectors encoding GFP under the control of the ubiquitous CMV promoter (AAV9-CMV-GFP). Three weeks post-AAV, the animals were sacrificed and the levels of GFP expression and production were evaluated in different tissues. Mice treated with AAV9-CMVenh-MLC2v-GFP vectors, particularly at dose $2 \times 10^{12}$ vg/mouse, showed robust GFP expression in the heart (Figure 4A). In addition, the quantification of the percentage of GFP+ cells revealed higher number of transduced cells in heart in animals treated with the low and high dose of AAV9-CMVenh-MLC2v-GFP vectors than in mice administered with the high dose of AAV9-CMV-GFP vectors (Figure 5A and 5B). Moreover, the CMV-enhMLC2v promoter precluded GFP expression and production in testis, epididymis, white adipose tissue, skeletal muscle, lung, intestine, brain, spleen and kidney (Figures 4B and 5B). However, it was unable to restrict GFP production in pancreas, brown adipose tissue, or liver, tissues in which the percentage of GFP$^+$ cells was similar in mice treated with AAV9-CMVenh-MLC2v-GFP or AAV9-CMV-GFP vectors (Figure 4B and 5B). Although transgene expression mediated by the CMVenh-MLC2v promoter was not completely heart-specific, the use of this promoter prevented GFP expression in a greater number of tissues in comparison with the CMV promoter.

To attain higher cardiac specificity than the one displayed by the CMVenh-MLC2v promoter, a shortened (544-nucleotides, SEQ ID NO: 16) version of the human troponin (TNT) promoter was used. To evaluate the strength and tissue-specificity of the shortened version of the TNT promoter, $1.75 \times 10^{12}$ vg of AAV9-TNT-GFP vectors were administered to 7-week-old male C57Bl6 mice. As control, a group of mice was treated with $1.75 \times 10^{12}$ vg of AAV9-CMV-GFP vectors. Four weeks after AAV administration, mice were sacrificed and GFP expression levels were assessed. The shortened version of the TNT promoter mediated GFP expression levels in the heart similar to those obtained with the CMV promoter (Figure 6). Furthermore, mice treated with AAV9-TNT-GFP vectors showed lower levels of GFP in the liver than animals that received AAV9-CMV-GFP vectors (25% decrease) and absence of transgene expression in white and brown adipose tissue (Figures 6 and 7).

Example 1.3. Optimization of the viral genome size prevents production of AAV intermediate species

Telomerase expression in lungs using AAV9 vectors encoding mouse telomerase under the control of the CMV promoter (AAV9-CMV-mTert) has been reported to mediate therapeutic benefit in pulmonary fibrosis and myocardial infarction (Povedano, J.M. et al. (2018) Elife.7: e31299; Bär, C. et al. (2014) Nat Comm Dec 18;5:5863). However, the size of the viral genome of AAV9-CMV-mTert vectors was 5291 nucleotides, which largely exceeded the AAV encapsidation limit of approximately 4.7 kb. Moreover, due to the large size of the viral genome, a polyA sequence could not be included in the expression cassette. Production of said AAV vectors resulted in vectors comprising several different intermediate species (Figure 8A). Noticeably, AAV vectors containing full length viral genomes were not clearly detected (Figure 8A). Moreover, analysis of genome integrity of AAV9-CMV-mTert vectors revealed a band of approximately 5.3 kb that corresponded to the full length AAV genome and a broad smear corresponding to truncated genomes (Figure 9A and 9B). Similar results were obtained when genome integrity and production of AAV intermediate species were evaluated for AAV9 vectors encoding a catalytically inactive form of murine telomerase under the control of the CMV promoter (AAV9-CMV-mTERT-DN) (Bernardes de Jesus, B. et al., 2012. EMBO Mol Med 4(8):691-704), whose viral genome size was also 5291 nucleotides (Figures 8B and 9A).

To avoid production of intermediate species in AAV vectors encoding telomerase under the control of the short version of the human SpB promoter, an exhaustive study of the coding sequences of mouse (mTERT) and human telomerases (hTERT) was carried out. The cDNA of mTERT (3786 nucleotides, SEQ ID NO: 1) was modified without altering the amino acid sequence of the protein by deleting the 3' UTR of the mRNA, resulting in a coding sequence of 3369 nucleotides in length (SEQ ID NO: 2), in order to minimize the length of the TERT encoding sequence. Moreover, said sequence was codon-optimized to improve protein production (moTERT, SEQ ID NO: 3). As a result of the decreased size of the TERT coding sequence and the use of the shortened human SpB promoter, the CMVenh-MLC2v promoter or the TNT promoter, the bovine growth hormone polyA sequence (SEQ ID NO: 18) could be included in the expression cassette without exceeding the encapsidation limit of AAV. Specifically, viral genomes size were: 4638 nucleotides for AAV9-SpB-moTERT (SEQ ID NO: 21), 4676 nucleotides for AAV9-CMVenh-MLC2v-moTERT (SEQ ID NO: 22) and 4536 nucleotides for AAV9-TNT-moTERT (SEQ ID NO: 23). Inclusion of a polyA sequence in the expression cassette can allow for improved TERT protein production due to increased mRNA stability, translational efficiency and for promotion of proper termination of mRNA avoiding the generation of unwanted 3' UTR sequences. No AAV intermediate species were detected for these vectors (Figures 8C-E). Genome integrity analysis revealed a band of approximately 4.7 kb that corresponded to full-length AAV genome and further confirmed no presence of truncated genomes (Figure 9A and 9B). Moreover, the higher intensity of the band corresponding to full-length vector genomes observed in AAV9-Spb-moTERT, AAV9-CMVenh-MLC2v-moTERT and AAV9-TNT-moTert indicated higher number of full-length genomes in these AAV preparations in comparison with AAV9-CMV-mTERT and AAV9-CMV-mTERT-DN (Figure 9A and 9B).

Example 1.4. AAV9-SpB-moTERT vectors mediate therapeutic benefit in pulmonary fibrosis

Next, we evaluated whether AAV9-SpB-moTERT vectors could mediate therapeutic benefit in pulmonary fibrosis. A mouse model of pulmonary fibrosis induced by short telomeres was used (Povedano el al (2015) Cell Rep 12, 286-299). This model is based on the intratracheal instillation of a low dose of bleomycin (0.5 mg/kg body weight) in 8-week-old G3-*Tert*$^{-/-}$ male mice, a dose that does not induce fibrosis in wild type mice with normal length telomeres. Two weeks post-Bleo insult, a computerized tomography (CT) scan was performed for each mouse to diagnose *bona-fide*

pulmonary fibrotic pattern. Mice diagnosed with pulmonary fibrosis were intravenously (IV) administered either with 2x10$^{12}$ vg of AAV9-SpB-moTERT or AAV9-null vectors. One, 3 and 5 weeks post-viral treatment a CT scan was performed for longitudinal follow-up of the disease progression. The decrease in the lung affected volume showing an aberrant CT pattern relatively to the start of the treatment was significantly higher in mice transduced with AAV9-SpB-moTERT as compared to those transduced with AAV9-null already after one week (Figure 10A-B).

Example 1.5. Comparative study of AAV9-CMV-mTERT and AAV9-SpB-moTERT efficacy in pulmonary fibrosis regression

The therapeutic efficacy in pulmonary fibrosis treatment mediated by AAV9-SpB-moTERT in comparison with AAV9-CMV-mTERT vectors was also evaluated. G3-Tert-/- male mice were administered intratracheally with bleomycin and two weeks post-bleo insult a CT was performed for each mouse to diagnose *bona-fide* pulmonary fibrotic pattern. Mice diagnosed with pulmonary fibrosis were IV administered either with 2x10$^{12}$ vg of AAV-CMV-null, with 2x10$^{12}$ vg of AAV9-CMV-mTERT or with 2x10$^{12}$ vg of AAV9-SpB-moTERT. Before bleomycin insult, an initial CT was performed for each mouse to calculate the volume of healthy lungs at baseline for further calculation of disease progression.
The disease progression was longitudinally followed up by CT at 1, 2, 4, 6 and 8 weeks post-AAV9 treatment. No changes in the affected volume were detected between week 6 and 8 post-AAV9 treatment, indicating chronification of the disease (Figure 11A). The rate of decrease of affected volume was therefore calculated during the first six weeks of treatment. The rate of decrease of the affected lung volume was similar in both treatments, AAV9-CMV-mTERT and AAV9-SpB-moTERT and faster than in the control group (Figures 11A and 11B). Mice were sacrificed at 8 weeks post-treatment. TERT expression levels and activity (TRAP) were measured in lung samples. Telomerase expression was analyzed by qPCR. Telomerase activity was measured as previously described (Herbert et al. Nat Protoc 1;583-1590, (2006), incorporated herein by reference in its entirety). The results demonstrate higher TERT expression and higher telomerase activity in both AAV9-CMV-mTERT and AAV9-SpB-moTERT-treated animals as compared to untreated ones (Figure 12A and 12B).

Example 1.6. Telomere length, Tert expression and telomerase activity

We performed immune-q-Fish with anti-SPC, a specific antibody of ATII cells, and a telomeric probe for quantification of telomere intensity in lung sections of AAV9-CMV-mTert (n=7), AAV9-SpB-moTert (n=7) and AAV9-CMV-null (n=6) treated mice. Both AAV9-CMV-mTert and AAV9-SpB-moTert groups showed an increased mean nuclear and mean spot intensity in ATII cells as well as in SPC negative cells as compared to AAV9-CMV-null treated group (Fig 13 A-D). In addition, the percentage of short telomeres (below 20th percentile of AAV9-CMV-null control samples) was lower in AAV9-CMV-mTert and AAV9-SpB-moTert as compared to AAV9-CMV-null control samples (Fig. 13 E-F). The percentage of long telomeres (above 80th percentile of AAV9-CMV-null control samples) was higher in AAV9-CMV-mTert and AAV9-SpB-moTert as compared to AAV9-CMV-null control samples (Fig. 13 G-H). Representative images of the assay are shown in Fig. 13I. These results indicate a bona fide telomere length increase in AAV9-CMV-mTERT and in AAV9-SpB-moTERT treated lungs.
Expression levels of Tert and Telomerase activity have been determined in lungs, liver, brain and heart by q-PCR and TRAP analysis, respectively (Fig. 14). The results demonstrate that AAV9-SpB-moTERT is not expressed neither in heart nor in brain.

*Example 2. Comparative study of AAV9-CMV-mTERT and AAV9-SpB-moTERT efficacy in pulmonary fibrosis regression*

AAV9-SpB-moTERT and AAV9-CMV-mTERT were evaluated for their therapeutic efficacy in PF (pulmonary fibrosis) regression. A mouse model of pulmonary fibrosis induced by short telomeres was used. This model was based on the intratracheal instillation of a low dose of bleomycin (0.5-0.7 mg/Kg body weight) in 8 weeks old G3-Tert-/- male mice, a dose that does not induce fibrosis in wild type mice with normal length telomeres. Before bleomycin (Bleo) insult, an initial CT was performed for each mouse to calculate the volume of healthy lungs for further calculation of disease progression. Two weeks post-Bleo insult a computerized tomography (CT) was performed for each mouse to diagnosed bona-fide pulmonary fibrotic pattern.

*Objectives*

Evaluation of the therapeutic efficacy in PF regression mediated by AAV9-SpB-moTert in comparison with AAV9-CMV-mTert vectors

*Study Design*

*Vectors:*

- AAV9-CMV-null (SEQ ID NO 24 represents the AAV-CMV-null recombinant adeno-associated virus genome)
- AAV9-CMV-Tert (SEQ ID NO 25 represents the AAV-CMV-Tert recombinant adeno-associated virus genome)
- AAV9-SpB-moTert (SEQ ID NO 21 represents the AAV-SpB-moTert recombinant adeno-associated virus genome)

*Dosage:*

*- 2x10E12 vg/mouse*

G3-Tert-/- male mice (n=48) were administered intratracheally with bleomycin (0.7 mg/Kg BW). Before bleomycin (Bleo) insult, an initial CT was performed for each mouse to calculate the volume of healthy lungs for further calculation of disease progression. Two weeks post-Bleo insult a computerized tomography (CT) was performed for each mouse to diagnosed bona-fide pulmonary fibrotic pattern. Mice diagnosed with pulmonary fibrosis were IV administered either with $2x10^{12}$ vg of AAV9-CMV-null (n=7), with $2x10^{12}$ vg of AAV9-CMV-mTert (n=8) or with $2x10^{12}$ vg of AAV9-SpB-moTert (n=9). The disease progression was longitudinally followed up by CT at 1, 2, 4, 6 and 8 weeks post-AAV9 treatment.
In Figure 15 the experimental lay-out is therein depicted.

*Material and Methods*

*Test and control articles*

A Bleomycin (Sigma-Aldrich 15361) solution (0.7 mg/ml) was prepared for intratracheal administration to induce pulmonary fibrosis in mice (0.7 mg/Kg BW). Mice were anesthetized by intraperitoneally (IP) injection of a solution (5 mL/g BW) containing fentanyl, medetomidine and Midazolam (40%-20%-40% v/v). The anesthesia was reversed by IP injection of a solution (3.5 mL/g BW) containing naloxone and atipamezole (85%-15% v/v).
The viral vectors to be tested were AAV9-CMV-null, AAV9-CMV-Tert, and AAV9-SpB-moTert. AAV9-CMV-null was used as negative control for telomerase expression. Viral vectors were administered intravenously (IV) via tail injection.

*Test System*

G3-Tert-/- male mice of 8 weeks of age (were inoculated by intratracheal instillation with a bleomycin (Sigma-Aldrich 15361) solution (0.7 mg/ml) to induce pulmonary fibrosis in mice (0.7 mg/Kg BW). For instillation an intravascular catheter 24 GA, o.75 IN, 0.7 x19 mm was used (BD Insyte, Ref: 381212, Lot: 9143584). Mice were anesthetized by intraperitoneally (IP) injection of a solution (5 mL/g BW) containing fentanyl, medetomidine and Midazolam (40%-20%-40% v/v). The anesthesia was reversed by IP injection of a solution (3.5 mL/g BW) containing naloxone and atipamezole (85%-15% v/v).
Three weeks post-bleo insult a CT was performed for each mouse to diagnose bona-fide pulmonary fibrotic pattern. Mice diagnosed with pulmonary fibrosis were IV administered either with $2x10^{12}$ vg of AAV-CMV-null, with $2x10^{12}$ vg of AAV9-CMV-mTert or with $2x10^{12}$ vg of AAV9-SpB-moTert. Before bleomycin insult, an initial CT was performed for each mouse to calculate the volume of healthy lungs at baseline for further calculation of disease progression. The disease progression was longitudinally followed up by CT at 1, 2, 4, 6 and 8 weeks post-AAV9 treatment. At 8 weeks post-viral treatment mice will be sacrificed for biochemical and histopathological studies

*Experimental protocol*

The objective of this task was to address the therapeutic efficacy in pulmonary fibrosis regression mediated by AAV9-SpB-moTert in comparison with AAV9-CMV-mTert vectors. After 8 weeks the animals were sacrificed and samples (lung, liver, heart and brain) were taken. Tissue samples to be analyzed by qPCR or TRAP are collected on dry ice and tissue samples for immunohistochemistry analysis are directly collected in formalin to be further embedded in paraffin blocks. Tissue samples (lung, liver, heart and brain) were collected for comparative analysis of telomerase expression (q-RT-PCR) and activity (TRAP) levels. For toxicology assessment paraffin blocks of mouse tissues are also collected. Immunohistopathological studies of the lung were performed by immunohistochemistry and immunofluorescence staining. In addition, telomere length studies was carried out by quantitative fluorescence in situ hybridization techniques (q-Fish) in clara cells and alveolar type II cells.

*Telomerase expression*

Transcriptional expression levels of Tert were analyzed by q-RT-PCR using the forward primer (5'- CTTCTT CCTGCACTTCCTGC -3') and the reverse primer (5'- GGGATGCCCTGGCACTG -3'). The RNA samples were extracted with trizol and zirconium beads. The cDNA was synthesized using iScript Adv cDNA kit for RT-qPCR (Bio-Rad, Ref: 1725037; Batch: 64375699).

*Telomerase activity*

Telomerase activity was analyzed by telomeric repeat amplification protocol (TRAP) as described in Herbert et al., 2006 (Herbert et al., Nature protocols, 2006)

*Immunohistochemistry*

Tissue samples were fixed in 10% buffered formalin, dehydrated, embedded in paraffin wax and sectioned at 2.5 $\mu$m. Tissue sections were deparaffinized in xylene and re-hydrated through a series of graded ethanol until water Immunohistochemistry (IHC) were performed on de-paraffined tissue sections processed with 10 mM sodium citrate (pH 6.5) cooked under pressure for 2 min. Masson's trichrome and picro Sirius red stainings were performed to quantified collagen depots. IHC staining of SMA, F4/80, gH2AX, p53, p21 in lung sections was performed with mouse monoclonal (1a4) DAKO IR611, rat monoclonal (CI:A3-1) ABD serotec MCA497, mouse monoclonal (JBW301) Millipore 05-636, rat monoclonal (POE316 A/E9) CNIO monoclonal antibodies core unit AM (POE316) and with rat monoclonal (291H/B5) CNIO monoclonal antibodies core unit, respectively. The slides were counterstained with hematoxylin and analyzed by light microscopy.

*Immuno q-Fish*

Immunostaining of Prosurfactant Protein C (SP-C) and of Secretoglobin Family 1A Member 1 (SCGB1A1 or CC10) in lung sections was performed with a rabbit polyclonal anti-proSP-C (Merck, AB3786) and with a mouse monoclonal anti-CC10 conjugated to Alexa Fluor 488 (Santa Cruz, sc-365992). After immunostaining a 20 minutes fixation with 4% paraformaldehyde is performed followed by a quantitative telomeric fluorescence in situ hybridization techniques (q-Fish) to analyze telomere length in alveolar type II and Clara cells.

*Data Analysis and Management*

Statistical significance is addressed by an unpaired Student Test, two-tailed

*RESULTS*

A total of 48 mice were administered with 0.7 mg Bleomycin/ Kg BW. A CT was performed after 3 weeks to diagnosed pulmonary fibrosis (Fig. 15). Twenty-four mice presented an abnormal CT pattern consistent with a fibrotic phenotype. Mice diagnosed with pulmonary fibrosis (n=24) were IV administered either with $2x10^{12}$ vg of AAV-CMV-null (n=7), with $2x10^{12}$ vg of AAV9-CMV-mTert (n=8) or with $2x10^{12}$ vg of AAV9-SpB-moTert (n=9).

*Molecular imaging results*

The disease progression was longitudinally followed up by CT at 1, 2, 4, 6 and 8 weeks post-AAV9 treatment (Fig. 15). No changes in the affected volume were detected between week 6 and 8 post-AAV9 treatment, indicating the disease becoming chronic. The rate of decrease of affected volume was therefore calculated during the first six weeks of treatment. The rate of decrease of the affected lung volume was similar in both treatments, AAV9-CMV-mTert and AAV9-SpB-moTert decreased faster than in the control group (Fig. 11 A,B). Mice were sacrificed at 8 weeks post-treatment and formalin and fresh tissue samples (liver, heart, brain and lung) were taken for immunohistochemistry and expression/activity analyses.

*Histopathological analysis results*

Histopathological analysis was performed by a mouse pathologist blindly. A pulmonary fibrosis histological score was given to every lung sample. A semiquantitative scoring of pulmonary fibrosis (Grade 0-Grade 4) based on the analysis of 10 visual fields (200x) stained with Sirius red (Lawson et al, 2005, 2011; Degryse AL et al 2012) (Fig. 16). The definition of each grade is as follows:

- Grade 0: normal lung architecture
- Grade 1: Increased thickness of some (≤50%) of interalveolar septa
- Grade 2: Thickening of >50% of interalveolar septa without formation of fibrotic lesions
- Grade 3: Thickening of the interalveolar septa with formation of isolated fibrotic lesions
- Grade 4: Multiple fibrotic lesions with total or subtotal distortion of parenchymal architecture

The results are represented in Figure 16. The AAV9-CMV-null treated control group showed two-fold higher PF score as compared to both AAV9-CMV-Tert and AAV9-SpB-Tert treated groups (Fig. 17A). Of note, no significant differences were observed between AAV9-CMV-Tert and AAV9-SpB-Tert treated groups, indicating that both vectors were equally efficient in ameliorating the fibrotic phenotype. These results are in agreement with the CT values at week 8 post-treatment (Fig. 17B). Indeed, a Pearson correlation analysis between CT values and PF score indicates a highly significant correlation (p<0.0001) (Fig. 17C).

A full histopathological analysis of all the organs was also performed, revealing no secondary effects of the telomerase gene therapy in an 8-week time window of the treatment. The pathological findings are described in Table 2. The observed pneumonia is induced by the bleomycin treatment. The pathologies observed in some mice in the liver, kidney and intestines are sporadic and common in mice. The tubular atrophy in testis is characteristic of telomerase deficient mice.

Table 2: Histopathological analysis at the end point (8 weeks post AAV9 treatment)

|  | AAV9-CMV-null | AAV9-CMV-mTert | AAV9-SpB-moTert |
| --- | --- | --- | --- |
| Lungs (pneumonia with lymphocite and macrophage infiltrates) | 6/6 | 6/8 | 8/8 |
| Liver (mild multifocal hepatitis) | 4/6 | 4/8 | 3/8 |
| intestines (mild difuse enteritis) | 0/6 | 0/8 | 2/8 |
| Kidneys (hydronephosi) | 0/6 | 0/8 | 1/8 |
| Spleen | 0/6 | 0/8 | 0/8 |
| Brain | 0/6 | 0/8 | 0/8 |
| Heart | 0/6 | 0/8 | 0/8 |
| Testis (tubular atrophy) | 6/6 | 8/8 | 8/8 |

*Telomerase expression and telomerase activity results*

Telomerase expression levels (q-RT-PCR) and activity (TRAP) was measured in lung samples. The results indicate higher Tert expression and higher Telomerase activity in both AAV9-CMV-mTert andAAV9-SpB-moTert treated animals as compared to untreated ones (Fig. 12A-B).

Telomerase expression levels (q-RT-PCR) (Fig. 18A-C) and activity (TRAP) was measured in liver, heart and brain samples samples (Fig. 19A-C). Telomerase was detected in liver with both vectors AAV9-CMV-Tert and AAV9-SpB-Tert and in heart with only AAV9-CMV-Tert. In brain samples, telomerase was detected with both vectors, AAV9-CMV-Tert and AAV9-SpB-Tert, though to very low levels as compared to the other analyzed tissues. At eight weeks post-treatment, no significant differences in telomerase activity were detected among the three vectors in liver, heart and brain with the only exception in liver samples infected with AAV9-CMV-Tert (Fig. 19A-C). The results demonstrate that AAV9-SpB-moTERT is not expressed neither in heart nor in the brain.

*Telomere length analysis by Quantitative Fluorescence In Situ Hybridization (q-Fish)*

We performed immune-q-Fish with anti-SPC, an specific antibody of ATII cells, and a telomeric probe for quantification of telomere intensity in lung sections of AAV9-CMV-null (n=6), AAV9-CMV-mTert (n=7) and AAV9-SpB-moTert (n=7) treated mice. Representative images of the assay are shown in Fig.13. Both AAV9-CMV-mTert and AAV9-SpB-moTert groups showed an increased mean nuclear and mean spot intensity in ATII cells as compared to AAV9-CMV-null treated group (Fig 13). In addition, the percentage of short telomeres (below 20th percentile of AAV9-CMV-null control samples) was lower in AAV9-CMV-mTert and AAV9-SpB-moTert as compared to AAV9-CMV-null control samples (Fig. 13). The percentage of long telomeres (above 80th percentile of AAV9-CMV-null control samples) was higher in AAV9-CMV-mTert and AAV9-SpB-moTert as compared to AAV9-CMV-null control samples (Fig. 13). These results indicate a bonafide telomere length increase in AAV9-CMV-moTERT and in AAV9-SpB-moTERT treated lungs.

We performed immune-q-Fish with anti-CC10, an specific antibody of Clara cells, and a telomeric probe for quantification of telomere intensity in lung sections of AAV9-CMV-null (n=6), AAV9-CMV-mTert (n=7) and AAV9-SpB-moTert (n=7) treated mice. Representative images of the assay are shown in Fig.20. No differences were detected either in mean nuclear, mean spot intensity, short telomeres (below 20th percentile of AAV9-CMV-null control samples) or in the percentage of long telomeres (above 80th percentile of AAV9-CMV-null control samples) among the three groups (Fig. 21 A-D). These results indicate that neither AAV9-CMV-mTert or AAV9-SpB-moTert vectors are expressed in Clara cells.

Immunohistochemistry analysis results

Histological examination of lungs at the end-point (8 weeks post-AAV9 treatment) showed that AAV9-SpB-moTert treated ones present less collagen depots, reduced presence of activated myofibroblasts (SMA) and less macrophages infiltration (F4/80) as compared to AAV9-CMV-null controls (Figure 22). In addition, AAV9-SpB-moTert treated lungs showed lower number of damaged cells (gH2AX positive cells) and a consequent reduction in the number of p53 and p21 positive cells (Figure 23).

*Conclusions*

- Less fibrotic lesions at the end point in Tert treated mice
- Decreased presence of activated fibroblast and macrophage infiltration (SMA and F4/80)
- Decreased DNA damage and dampened DDR
- Increased numbers of ATII cells
- Immuno qFISH Spc/Tel and CC10/Tel. Longer tels in ATII cells in Tert treated mice. No effect in tel length in Clara cells.

*Example 3. Evaluation of intratracheal delivery route and selection of the best AAV serotype and promoter to genetically engineer lungs*

Local delivery of AAV vectors into the respiratory tract has been reported via intranasal or intratracheal administration (Limberis, MP., et al. Proc Natl AcadSci U S A 2006 Aug 29;103(35):12993-8; Lisa A Santry. BMC Biotechnol. 2017 May 15;17(1):43; Laura P. van Lieshout, Methods Mol Biol. 2019;1950:361-372; Katz, MG. et al. J Cardiovasc Dev Dis. 2019 Feb 15;6(1):8;Kang, M. et al., Nat Commun. 2020 Aug6;11(1):3929). The development of neutralizing antibodies is a key barrier for all gene therapies involving viral vectors. In this sense, the lung is one of the organs where repeat dosing is feasible. In this regard, re-administration of AAV vectors has been reported upon local delivery into the respiratory tract in mouse, rabbits, non-human primates and patients suffering from cystic fibrosis (CF) (Limberis, MP., et al. Proc Natl AcadSci U S A 2006 Aug 29;103(35):12993-8; Guggino, BW., et al. Expert OpinBiolTher. 2017 Oct;17(10):1265-1273). Re-administration was feasible even in the presence of high levels of serum-circulating neutralizing antibodies as early as 1 month after initial exposure, with minimal effect on overall transgene gene expression (Limberis, MP., et al. Proc Natl AcadSci U S A 2006 Aug 29;103(35):12993-8; Guggino, BW., et al. Expert OpinBiolTher. 2017 Oct;17(10):1265-1273).

*Objectives of this example*

- Lung transduction efficiency of AAV9 and AAV6 serotypes by intratracheal local administration route.
- Lung tropism of AAV6 versus AAV9 serotypes.
- Comparative study between CMV and SpB promoter.
- Lung transduction efficiency with different viral titers.
- Biodistribution and tissue specificity of SpB promoter.

*Study Design*

*Vectors:*

- AAV9-CMV-null (SEQ ID NO 24 represents the AAV-CMV-null recombinant adeno-associated virus genome)
- AAV9-CMV-GFP (SEQ ID NO 26 represents the AAV-CMV-GFP recombinant adeno-associated virus genome)
- AAV9-SpB-GFP (SEQ ID NO 27 represents the AAV-SpB-GFP recombinant adeno-associated virus genome)
- AAV6-CMV-null (SEQ ID NO 24 represents the AAV-CMV-null recombinant adeno-associated virus genome)
- AAV6-CMV-GFP (SEQ ID NO 26 represents the AAV-CMV-GFP recombinant adeno-associated virus genome)
- AAV6-SpB-GFP (SEQ ID NO 27 represents the AAV-SpB-GFP recombinant adeno-associated virus genome)

*Dosages:*

- 10E11 vg/mouse
- 5x10E11 vg/mouse
- 10E12 vg/mouse

Healthy wild-type mice were intratracheally administered with the vectors at the doses indicated above. Mice were sacrificed at 3 or 9 weeks post-infection. GFP expression was evaluated in the five lung lobes; right superior, right middle, right inferior, post-caval and left lobes. GFP expression was also evaluated in liver, heart and brain to address off-target tissues. GFP expression was analyzed by quantitative PCR and by immunohistochemistry. In Figure 24 the experimental lay-out is depicted.

*Material and Methods*

*Test and control articles*

The viral vectors to be tested were AAV9-CMV-null, AAV9-CMV-GFP, AAV9-SpB-GFP, AAV6-CMV-null, AAV6-CMV-GFP and AAV6-SpB-GFP. The AAV9-CMV-null and the AAV6-CMV-null were used as negative control for GFP expression.

*Test System*

Wild type mice of genetic background C57BL/6J.OlaHsd of 9 weeks of age were inoculated by intratraqueal instillation with 30mL of PBS 0.001% Pluronic F-68 (Gibco, Ref: 240-032, Lot: 1836664) containing the viral vectors. For instillation an intravascular catheter 24 GA, o.75 IN, 0.7 x19 mm was used (BD Insyte, Ref: 381212, Lot: 9143584). Mice were anesthetized with 1-3% isofluorane in oxygen by inhalation.

*Experimental protocol*

The objectives of this example were to address:

1.- Lung transduction efficiency of AAV9 and AAV6 serotypes by intratracheal local administration route
2.- Lung tropism of AAV6 versus AAV9 serotypes
3.- Comparative study between CMV and SpB promoter
3.- Lung transduction efficiency with different viral titers
4.- Biodistribution and tissue specificity of SpB promoter

Six different vectors (AAV9-SpB-GFP, AAV6-SpB-GFP, AAV9-CMV-GFP, AAV6-CMV-GFP, AAV9-CMV-nul, AAV6-CMV-null) were intratracheally delivered at three different viral doses (10E11, 5x10E11 or 10E12) in healthy wild type mice. A total of 120 mice were allocated to this assay. After three weeks and nine weeks the animals were sacrificed and samples (lung, liver, heart and brain) were taken.

The five lung lobes were individually sampled to assay whether viral spreading was homogenous among the lobes. Lung samples to be analysed by qPCR were collected on dry ice and lung samples for immunohistochemistry analysis were directly collected in formalin to be further embedded in paraffin blocks.

The transcriptional expression levels of GFP were analysed by q-RT-PCR using the forward primer (5'-AGC TGG ACG GCG ACG TAA-3') and the reverse primer (5'- GTG CAG ATG AAC TTC AGG GTC A-3'). The RNA samples were extracted with trizol and zirconium beads. The cDNA was synthesized using iScript Adv cDNA kit for RT-qPCR (Bio-Rad, Ref: 1725037; Batch: 64375699).

*Immunohistochemistry detection of GFP, SPC and CC10*

Tissue samples were fixed in 10% buffered formalin, dehydrated, embedded in paraffin wax and sectioned at 2.5 μm. Tissue sections were deparaffinized in xylene and re-hydrated through a series of graded ethanol until water Immunohistochemistry (IHC) were performed on de-paraffined tissue sections processed with 10 mM sodium citrate (pH 6.5) cooked under pressure for 2 min. IHC staining of GFP in lung, liver, brain and heart sections was performed with rabbit monoclonal (D5.1) anti-GFP (1:100; Cell Signaling Technology). IHC staining of Prosurfactant Protein C (SP-C) in lung sections was performed with a rabbit monoclonal (EPR19839) anti-SPC (1:100; ABCAM, Ref: ab211326). IHC staining of Secretoglobin Family 1A Member 1 (SCGB1A1 or CC10) in lung sections was performed with a goat polyclonal (EPR19839) anti-CC10 (1:100; ABCAM, Ref: ab211326). The slides were counterstained with hematoxylin and analyzed by light microscopy.

*Data Analysis and Management*

Statistical significance is addressed by a unpaired Student Test, two-tailed

*Deviations*

Any deviation to the protocol either planned or unplanned are documented in this study report.

*Planned deviations*

Lung samples corresponding to mice sacrificed at nine weeks post-infection were taken as follows. The left lung lobe was collected on dry ice and stored at -80°C for q-RT-PCR analysis. The right lobes were fixed in formalin and included in a single cassette for paraffin embedment for IHC analysis. This deviation was planned based on the results from the analysis of GFP expression levels at three weeks post-infection given that no significant differences in viral spreading among the different lung lobes were detected.

*RESULTS*

*Quantification of GFP expression in lung samples by q-RT-PCR at three- and nine-weeks post-infection with viral vectors*

The results from q-RT-PCR analysis in the different lung lobes after three weeks post infection are represented in figure 25. No differential viral spreading was observed with any of the vectors used among the different lung lobes. We therefore pooled all the data of the five different lobes from each lung (Figure 26 A). In Figure 26 B, the data corresponding to the q-RT-PCR analysis in the left lung lobe after nine weeks post infection is represented. The expression levels were maintained from 3 to 9 weeks post-infection. This observation prompted us to pool the data from the mice sacrificed at 3 and 9 weeks to increase the sample size (Fig. 27). The results show that at all the viral doses under study, AAV6 serotype leads to higher expression levels than AAV9 serotype and that the SpB and the CMV promoters render similar expression levels. In addition, no differences in GFP expression levels were observed between a viral titer of $5x10^{11}$ vg/mouse and $10^{12}$ vg/mouse, indicating a saturation of infectivity at a viral titer below or equal to $5x10^{11}$ vg/mouse.

*Quantification of GFP positive cells in lung samples by IHC at three- and nine-weeks post-infection with viral vectors*

The results from IHC analysis in the different lung lobes after three weeks post infection are represented in figure 28. No differential viral spreading was observed with any of the vectors used among the different lung lobes, indicating a homogenous distribution of viral particles throughout the lobes (Figure 28). We therefore pooled all the data of the five different lobes from each lung (Figure 29 A). In Figure 29 B, the data corresponding to the IHC analysis in the left lung lobe after nine weeks post infection is represented. The percentage of GFP positive cells was maintained from 3 to 9 weeks post-infection with all the viral vectors except for AAV9-CMV-GFP that was undetectable at nine weeks post-infection at the three titers under assay (Fig. 29). The results indicate that at all the viral doses under study, AAV6 serotype shows higher lung tropism than the AAV9 serotype. Also, viral vectors driving GFP expression under the SpB promoter leads to higher number of infected cells than those with CMV promoter, independently of the serotype. In addition, no differences in the percentage of cells GFP positive are observed between a viral titer of $5x10^{11}$ vg/mouse and $10^{12}$ vg/mouse, indicating a saturation of infectivity at a viral titer below or equal to $5x10^{11}$ vg/mouse. The percentage of GFP positive cells were maintained from 3 to 9 weeks post-infection with AAV9-SpB-GFP and AAV6-SpB-GFP vectors at the three viral dosses under study.

Lungs transduced with AAV9-CMV-GFP and AAV6-CMV-GFP showed very few cells positive for GFP, below 0.3% and 1% of total lung cells, respectively, at 9 weeks post-infection.

Representative images corresponding to $10^{12}$ vg/mouse titer with all the vectors under study is shown in Fig. 30.

*Identification and quantification of alveolar type II cells and clara cells targeted by the viral vectors in lung samples.*

To determine the percentage of Alveolar Type II cells (ATII) and the percentage of Clara cells that are targeted by the different viral vectors under study in the present example, we performed a double staining by IHC with anti-GFP and either anti-SpC or anti-CC10, specific markers for ATII and clara cells, respectively. The lungs analyzed correspond to the mice sacrificed at 9 weeks post-infection. Representative images of these staining are shown in Figure 31 and in Figure 32. The AAV9-CMV-GFP infected lungs show below 0.3% of GFP positive cells (Fig. 29B). The AAV6-CMV-GFP infected lungs show below 1% of GFP positive cells (Fig. 29B) of which none of them were CC10 positive indicating that this vector does not target Clara cells. The GFP positive cells were mainly SPC positive although some GFP positive cells were also found to be SPC/CC10 negative, indicating that this vector also targets other cell types than Clara and ATII cells.

The AAV9-SpB-GFP infected lungs show 5.7%, 4% and 10% of GFP positive cells (Fig. 29B) of which 100% of GFP positive cells were SPC positive indicating that this vector targets exclusively ATII cells.

Quantification data in the lungs transduced with AAV6-SpB-GFP at the three viral titers is shown in Figure 33. The percentage of SpC positive, i.e. ATII cells, cells correspond to 14-17% of total lung cells at the three viral titers analyzed (Fig. 33A). Of total ATII cells, 36%, 45% and 55% were GFP positive at $10^{11}$ vg/mouse, $5x10^{11}$ vg/mouse and $10^{12}$ vg/mouse, respectively (Fig. 33B). Of total GFP positive cells, 73%, 78% and 73% 78% correspond to ATII cells positive at $10^{11}$ vg/mouse, $5x10^{11}$ vg/mouse and $10^{12}$ vg/mouse, respectively (Fig. 33C). The percentage of CC10 positive cells, i.e. Clara cells, targeted by AAV6-SpB-GFP correspond to 7%, 15.5% and 20.2% at $10^{11}$ vg/mouse, $5x10^{11}$ vg/mouse and $10^{12}$ vg/mouse, respectively (Fig. 33D). Of total GFP positive cells, 11.5%, 21.4% and 32.2% correspond to Clara cells (Fig. 33E). Thus, AAV6-SpB-GFP targets ATII cells (app. 80%) and Clara cells (app 20%).

*Viral tropism to off-target organs: Liver, heart and Brain*

*Quantification of GFP positive cells by IHC in liver and heart at three- and nine-weeks post-infection with viral vectors*

The results from IHC analysis in liver and heart samples after three- and nine-weeks post-infection are represented in figure 34. The results from lung samples are also shown for comparison purposes. The AAV6 serotype did not target either liver or heart since no GFP positive cells were detected at both time points. The AAV9-CMV-GFP and the AAV9-SpB-GFP render GFP positive cells in the liver at 3- and 9-weeks post-infection, being the percentage of GFP positive cells higher in AAV9-CMV-GFP than in AAV9-SpB-GFP at a given viral titer. The number of GFP positive cells was highly reduced at 9 weeks as compared to 3 weeks post-infection (Fig. 34 A,B). Only very few GFP positive cells, below 0.1% of total heart cells, were detected in heart samples from mice transduced with AAV9-CMV-GFP at 3 weeks post-infection at higher viral titers, $10^{11}$ and $5x10^{11}$ vg/mouse (Fig. 34 C,D).

Clearly, these results demonstrate the specificity of SpB promoter for lung cells, in particular ATII y clara cells as well as less off-target organs when using AAV6 as compared to AAV9 serotype (Fig. 34 E,F).

*Quantification of GFP expression levels by q-RT-PCR in liver, heart and brain at three- and nine-weeks post-infection with viral vectors*

The results of GFP expression levels by q-RT-PCR in liver, heart and brain samples at 3- and 9- weeks post-infection are shown in Figure 35. GFP expression was neglectable in these three organs at both time-points.

*Conclusions*

The AAV6 serotype and the SpB promoter were selected for further assays (see examples 5 and 6), although the AAV6 was better that the AAV9 serotype, the AAV9 serotype should not be discarded for further analyses. The viral titers chosen were $10^{11}$ and $5x10^{11}$ vg/mouse. The AAV6 serotype and the SpB promoter were highly specific for the lungs, presenting no tropism in off-target organs when intratracheally administered.

*Example 4. Production and characterization of research grade AAV6-SpB-moTERT vectors*

The objective of this example was to produce and characterize research grade AAV6-SpB-moTERT vectors. To this end, the plasmid pAAV-SpB-moTERT-bGH was co-transfected into HEK293 cells with the pRepCap6 and pAdHelper plasmids that contain the helper genes necessary for AAV serotype 6 production. AAV6-SpB-moTERT vectors were purified by PEG precipitation and a double CsCl gradient, formulated in PBS 2x + 0.001% PF68 and sterile filtrated. After purification, several quality attributes were determined to characterize the AAV vectors produced.

*Methods*

*AAV vector production*

Adherent HEK-293 cells in Roller Bottles were triple transfected with PEI-MAX as agent of transfection (Polyscience) and the following plasmids: 1) an optimized packaging plasmid pREPCAP that encodes for REP2 and CAP genes, 2) an optimized adenoviral helper plasmid pAdHelper that encodes for E2, E4 and VA-RNA genes, supplying all the adenoviral functions required for AAV vector production with the minimal plasmid size, 3) a plasmid containing the expression cassette flanked by the AAV2 ITR sequences.

Seventy-two hours post-transfection HEK-293 cells were harvested. Cells were separated from the medium (containing AAV vectors) by centrifugation and lysed by sonication. Lysed cells were centrifuged to eliminate the cell debris and the supernatant (containing AAV vectors) was kept and added to the medium containing AAV vectors. Then, all AAV particles were concentrated by PEG precipitation (1). After resuspension of the AAV-PEG pellet supernatant was treated with Benzonase (Merk) at a final concentration of 100U/ml. Next, two rounds of CsCl gradient centrifugation were performed to separate full from empty AAV capsids. In the first CsCl gradient (discontinuous gradient, 16 hours, swinging bucket rotor SW32 Beckman, 27000 rpm), full AAV vectors were separated from the major part of protein impurities and empty AAV capsids. In the second gradient (isopycnic gradient; 48 hours, swinging bucket rotor SW40Ti Beckman, 32000 rpm), full AAV vectors were separated from protein impurities, intermediate species and remaining empty AAV capsids. Next, full AAV vectors were dialyzed with Slide-A-Lyser Cassette (Life Technologies), with the desired formulation buffer. Finally, AAV vectors were sterile filtrated with Millex GV 0.2$\mu$m Syringe filters (Millipore) and stored at < -60°C.

*Quantification of vector genome titer by digital droplet PCR*

The quantification of the vector genome titer by digital droplet PCR was carried out using the Digital Droplet PCR (ddPCR) system by Bio Rad. Prior to the qPCR reaction, samples were digested with 10 units of DNAseI (Cat n° 04716728001; Roche) to eliminate possible DNA contaminants present outside AAV capsids. Then, AAV particles were lysed by the addition of SDS to a final concentration of 0.1% and incubated 1 hour at 70°C. Next, EDTA was added to a final concentration of 5mM to inhibit DNAseI activity. Samples were then diluted to 5E+6 vg/ml approximately to ensure that samples fell into the quantification range of the technique. Samples were then mixed with a PCR mix solution containing a fluorescent probe and primers targeting the moTERT sequence (Forward: CACCAGACACAACGAGAGAAG; Reverse: CTCGACCTTCATCTTCCACATC; FAM-Probe: AGGGACAGCTTGCCGTACTTCC) and the ITR2 sequence (Forward: GGAACCCCTAGTGATGGAGTT; Reverse: CGGCCTCAGTGAGCGA; HEX-Probe: HEX-CACTCCCTCTCTGCGCG CTCG-BBQ)

Droplets were subsequently formed by the addition of a Droplet Generation Oil specific for probes (cat n° 1863005; Bio Rad) and using the QX100 Droplet generator by Bio Rad (cat n° 186-3002). Droplets were then transferred to a PCR plate, placed in a thermocycler and subjected to PCR amplification (40 cycles at 60°C). Finally, droplets were counted for positive or negative amplification using the QX100 droplet reader (Cat n°: 186-3001; Bio Rad).

Data analysis was carried out using the QuantaSoft software version 1.7.4 (Bio Rad). Final titer was calculated by correcting the value of copies/ul provided by the software with the different dilutions performed during the procedure.

*Quantification of vector genome titer by PicoGreen assay*

AAV vectors were quantified using the Quant-iT PicoGreen dsDNA assay kit (Cat n° P11496; Invitrogen) that allows determination of the concentration of dsDNA by fluorometry using a Lambda DNA as standard (included in the kit). AAV vectors were incubated either with 0.1% of SDS for 1 hour at 70°C to lyse the capsids or at room temperature without 0.1% of SDS, as a negative control. Next, samples were progressively cooled to 25°C to form the dsDNA and diluted in Tris-EDTA buffer. Diluted samples were mixed with the PicoGreen dye and loaded into a black 96-well assay plate. Fluorescence was measured with a Synergy™ HTX Multi-Mode Microplate Reader.

To quantify the vector genome titer, fluorescence signal of non-lysed AAV vectors was subtracted from that of lysed AAV vectors to obtain delta fluorescence. The standard curve generated with the Lambda Standard serial dilution was used to interpolate the delta fluorescence values of the rAAV samples, thus obtaining the vector genome concentration in ng/ml. The following formula was used to calculate vector titer in vg/ml from ng/ml:

$$\text{vg/ml} = (\text{ng/ml}) \times 1.85\text{E}+12 \times \text{dilution} / (\text{length of AAV vectors})$$

*Analysis of AAV vector protein staining*

AAV vectors were treated with Laemmli buffer at 95°C for 5 min to denature the capsids. Polyacrylamide gel electrophoresis was used to separate the proteins that form the AAV vector capsid and possible contaminating proteins of the batch. Proteins were stained by Sypro Ruby Staining (Invitrogen). The presence of the 3 viral proteins (VP1, VP2 and VP3) forming the AAV vector capsid, their correct proportion (VP1 and VP2 bands with similar intensity and VP3 band with the highest intensity) and the presence of impurities was checked by fluorometry (Syngene Gene Genius bio imaging-system).

*Quantification of vector genome and viral particle titers by Optical Density*

Sample absorbance at 260 nm and 280 nm was measured in a Synergy™ HTX Multi-Mode Microplate Reader using the Take3 micro-volume plate. Vector genomes and viral particle titers were calculated according to formulas described by Sommer et al (2) modifying the extinction coefficient of dsDNA to ssDNA.

$$vp/vg = MW_{DNA} \times (\varepsilon_{ssDNA260} - \varepsilon_{ssDNA280} \times Abs_{260}/Abs_{280})/((Abs_{260}/Abs_{280}) \times \varepsilon_{capsid280} - \varepsilon_{capsid260}))$$

$$vg/ml = Abs_{260} \times (6{,}02E{+}20/(\varepsilon_{ssDNA260} \times MW + \varepsilon_{capsid260} \times vp/vg))$$

where,

$$\varepsilon_{ssDNA260} \text{ (Extinction Coefficient } _{ssDNA260}) = 27 \text{ g}^{-1} \times L \times cm^{-1}$$

$$\varepsilon_{ssDNA280} \text{ (Extinction Coefficient } _{ssDNA280}) = 15 \text{ g}^{-1} \times L \times cm^{-1}$$

$$MW_{DNA} \text{ (Molecular weight of the packaged DNA)} = \text{number of nucleotides} \times MW_{nucleotide}$$

$$MW_{ssDNA} = n°A \times 312.2Da + n°C \times 288.2Da + n°G \times 328.2Da + n°T \times 303.2Da.$$

$$\varepsilon_{capsid260} \text{ (Molar Extinction Coefficient of the AAV capsid at 260 nm)}$$

$$\varepsilon_{capsid280} \text{ (Molar Extinction Coefficient of the AAV capsid at 280 nm)}$$

*Determination of AAV vector genome integrity by alkaline gel electrophoresis*

AAV vector genome integrity was assessed by alkaline agarose gel electrophoresis (denaturing conditions) where single stranded DNA can be analyzed. First, 5E+11 vg of each batch was mixed with alkaline loading buffer and boiled to 95°C for 5 min to lyse the AAV particles. Next, DNA was subjected to agarose gel electrophoresis under denaturing conditions. Finally, band size and quality were examined to assess genome integrity. Although the same amount of vector genomes were processed to be loaded in the alkaline agarose gel, differences in band intensity may be observed, due to the characteristic of this technic. These putative differences in band intensity may not account for differences in vector concentration.

AAV vector genomes are single stranded DNA but are formed by sense and antisense strands (50% of each strand approximately). Double stranded DNA (such as the DNA ladder) or single stranded DNA with positive and negative strand may present a double band (or a diffuse band) in the denaturing agarose gel that corresponds to the sense and antisense strands, that run separately (Éva Hegedüs et al., 2009).

*Quantification of viral particle titer by ELISA*

AAV viral particles (full and empty particles) were quantified with the AAV Titration ELISA kit (AAV6 Titration ELISA kit, Progen, Art.No. PRAAV6). The capture-antibody detects a conformational epitope that is not present on unassembled capsid proteins. The assay is based on a sandwich ELISA technique. A monoclonal antibody specific for a conformational epitope on assembled AAV capsids was coated onto strips of a microtiter plate and was used to capture AAV particles from the sample. Captured AAV particles were detected in two steps. First, a biotin-conjugated monoclonal antibody to AAV was bound to the immune complex. Second, a streptavidin peroxidase conjugate reacted with the biotin molecules. Addition of substrate solution resulted in a color reaction, which was proportional to the amount of specifically bound viral particles. The absorbance was measured at 450 nm.

*In vitro potency assay: mRNA expression*

2v6.11 cells were used in this study because they present enhanced transduction by AAV vectors. In this cell line generated from HEK 293 cells, the Ad5 E4 is expressed under the control of the ecdysone-inducible promoter, which allows induction of E4 expression by the ecdysone analogue Ponasterone A.

The day before infection, 2v6.11 cells were seeded in 24-well plates at a density of 2E+05 cells/well. Cells were grown at 37°C and 5% $CO_2$ in growth media (DMEM + 10% FBS) supplemented with antibiotics (Penicillin= 10,000 U/ml, Streptomycin= 10,000 μg/ml) and 1 μg/ml Ponasterone A.

Prior to infection, cells were assessed for correct cell confluence (70-80%) at a bright field microscope. To determine the cell count, cells of 4 wells were trypsinized and cell number was quantified with a Scepter 2.0 Handheld Automated Cell Counter (Merck-Millipore). Cells were infected with AAV-953 vectors at MOIs= 32000 vg/cell, 64000 vg/cell and 128000 vg/cell. Cells were infected in triplicate. Non-infected 2v6.11 cells (NI) were used as a negative control.

Forty-eight hours post-infection and after assessing cell viability, cells were gently washed with 500 μl 1x PBS and collected in 350 μl RLT+β-mercaptoethanol (10 μl/ml) (RNeasy Mini Kit, Qiagen). RNA was extracted with the RNeasy Mini Kit (Qiagen) and RNase-free DNase I (Qiagen) following the protocol provided by the manufacturer except the extension of the on-column DNAse I digestion from the standard 15 minutes to 30 minutes, to ensure appropriate degradation of the infected AAV vector genome. One μg of each RNA sample was retrotranscribed using the Transcriptor FirstStrand cDNA Synthesis kit (Roche). qPCR was performed in triplicate using primers and 2 μl of sample (diluted 1/10). To quantify expression, a primer-probe mix targeting moTERT was used (Forward primer: GGA AGT GTT CTG CGA CTA CAG ; Reverse primer: CAG CTT GTT CCT CAT GGT CTT; Probe: /56-FAM/CTACGCCCA/ZEN/GACCAGCATCAAGAC/3IABkFQ/). A primer-probe mix for housekeeping gene hRplp0 was used to normalize (forward primer: CAG ACA GAC ACT GGC AAC AT; Reverse primer: GCA GCA TCT ACA ACC CTG AA; Probe: /5HEX/AA CTC TGC A/ZEN/TT CTC GCT TCC TGG A/3IABkFQ).

*Quality control: Results*

*AAV vectors production*

To produce the batch AAV-953 50 Roller Bottles were transfected.

After PEG precipitation and the first cesium chloride, the second CsCl gradient of batch AAV-953 presented an intense band that corresponded to full vectors (Figure 36). Empty particles were eliminated in the first gradient.

AAV vectors were dialyzed in PBS 2x + 0.001% Pluronic F68. After sterile filtration 2.695 ml of AAV6-SpB-moTERT-bGH were stored at -80°C.

Quantification of vector genome titer by digital droplet PCR

Two quantifications were performed in two independent days. AAV vectors were quantified with a duplex ddPCR assay targeting the transgene (moTERT) and the ITR2 present in all the AAV vectors.

Table 3. Summary of the quantification of the vector genome titer by digital droplet PCR with primers targeting the moTERT sequence.

| Vector name | Batch | vg/ml | SEM |
|---|---|---|---|
| AAV6-SpB-moTERT | AAV-953 | 1.73E+13 | ±0 |

Table 4. Summary of the quantification of the vector genome titer by digital droplet PCR with primers targeting the ITR2 sequence.

| Vector name | Batch | vg/ml | SEM |
|---|---|---|---|
| AAV6-SpB-moTERT | AAV-953 | 1.96E+13 | ±3.0E+11 |

Similar titers were obtained with the two primers sets used to quantify the AAV vectors.

Titer obtained with the primer set targeting the transgene (moTERT) was selected to be reported as the titer of the batch and to be used for the in vitro and the in vivo experiments.

*Quantification of vector genome titer by PicoGreen assay*

PicoGreen assay was used as an orthogonal method of ddPCR assay to determine the titer of the AAV vectors.

**Table 5.** Summary of the quantification of the vector genome titer by PicoGreen assay.

| Vector name | Batch | vg/ml | SEM |
|---|---|---|---|
| AAV6-SpB-moTERT | AAV-953 | 1.62E+13 | ±9.24E+11 |

A similar titer between PicoGreen and ddPCR assay was obtained.

*Analysis of AAV vector protein staining*

Vector batch purity was determined by Sypro Ruby protein staining. AAV-953 batch protein staining presented the three viral particles (VP) and an excellent purity (Figure 37).

*Quantification of viral particles titer by ELISA*

AAV6 ELISA kit from Progen (Art. No. PRAAV6) was used to quantify the AAV particles of the AAV-953 batch.

**Table 6.** Viral particle titers obtained by the AAV6 ELISA kit.

| Vector name | Batch | vp/ml | SEM vg/ml |
|---|---|---|---|
| AAV6-SpB-moTERT | AAV-953 | 1.32E+13 | ±5.04E+11 |

A similar titer was obtained of vector genomes and viral particles, indicating a batch mainly composed of full particles.

*Quantification of vector genome and viral particle titers by Optical Density*

Optical Density assay was used as both an orthogonal method of ddPCR assay to determine the titer of the AAV vectors and an orthogonal method of ELISA to determine the concentration of viral particles.

**Table 7.** Summary of the vector genomes and viral particle titers obtained by Optical density of the AAV-953 batch.

| Batch | vg/ml | SEM vg/ml | vp/ml | SEM vp/ml |
|---|---|---|---|---|
| AAV-953 | 1.67E+13 | ±3.70E+11 | 2.59E+13 | ±5.77E+11 |

A similar titer was obtained of vector genomes and viral particles, indicating a batch mainly composed of full particles.

*Determination of AAV vector genome integrity*

Vector genome integrity was determined by an alkaline agarose gel (Figure 38). AAV-953 batch presented a main band around 4500 bases that corresponded to full length vector genomes.

*In vitro potency assay: mRNA expression*

Infection of 2v6.11 cells with vector AAV-953 resulted in moTERT mRNA expression. Moreover, the expression levels of moTERT were proportional to the vector dose (MOI) (Figure 39).

Vector genome integrity of the AAV6-Spb-moTERT vectors used in this invention was determined by an alkaline agarose gel (Figure 40). Genome integrity analysis revealed a band of approximately 4.7 kb that corresponded to full-length AAV genome and further confirmed no presence of truncated genomes (Figure 40). Similarly to the observations made for AAV9-Spb-moTERT, AAV9-CMVenh-MLC2v-moTERT and AAV9-TNT-moTERT (Figure 9A and 9B), the higher intensity of the band corresponding to full-length vector genomes observed in AAV6-Spb-moTERT indicated higher number of full-length genomes in this AAV preparation in comparison with AAV vectors comprising oversized TERT-encoding expression cassettes (AAV9-CMV-mTERT and AAV9-CMV-mTERT-DN) (Figure 9A and 9B).

*Example 5. Evaluation of telomerase expression levels in lungs upon intratracheal administration of AAV6-*

*SpB-moTert in healthy mice*

In this example we measured the telomerase expression and activity in AAV6-SpB-moTert transduced mice by intratracheal administration and we compared between $10^{11}$ and $5 \times 10^{11}$ vg/mouse.

*Study Design*

Vectors:

- AAV6-SpB-null (SEQ ID NO 28 represents the AAV-SpB-null recombinant adeno-associated virus genome)
- AAV6-SpB-moTert (SEQ ID NO 21 represents the AAV-SpB-moTert recombinant adeno-associated virus genome)

*Dosages:*

- **D1**. 10E11 vg/mouse
- **D2**. 5x10E11 vg/mouse

Healthy wild-type mice (9 weeks old) were intratracheally administered with the AAV6-SpB-null at $5 \times 10^{11}$ vg/mouse and with AAV6-SpB-moTert at $1 \times 10^{11}$ and at $5 \times 10^{11}$ vg/mouse. Ten mice were included per group. Mice were sacrificed at 6 weeks post-infection. Viral genome, telomerase expression levels and telomerase activity were evaluated in lungs and off-target tissues. Percentage of AAV6 infected cells as well as telomere length were also analyzed. In Figure 41 is depicted the experimental lay-out.

*Material and Methods*

*Test and control articles*

The viral vectors to be tested were AAV6-SpB-null and AAV6-SpB-moTert. AAV6-SpB-null was used as negative control for telomerase expression.

*Test System*

Wild type mice of genetic background C57BL/6J.OlaHsd of 9 weeks of age were inoculated by intratracheal instillation with 30mL of 2xPBS 0.001% Pluronic F-68 (Gibco, Ref: 240-032, Lot: 1836664) containing the viral vectors. For instillation an intravascular catheter 24 GA, o.75 IN, 0.7 x19 mm was used (BD Insyte, Ref: 381212, Lot: 9143584). Mice were anesthetized with 1-3% isofluorane in oxygen by inhalation.

*Experimental protocol*

The objectives of this assay were to address:

- Two different vectors (AAV6-SpB-null and AAV6-SpB-moTert were intratracheally delivered at two different viral doses (10E11, 5x10E11 vg/mouse) in healthy wild type mice. A total of 30 mice were allocated to this assay. After 6 weeks the animals were sacrificed and samples (lung, liver, heart and brain) were taken.

- Tissue samples to be analysed by qPCR were collected on dry ice and tissue samples for immunohistochemistry analysis were directly collected in formalin to be further embedded in paraffin blocks.

*Telomerase expression*

The transcriptional expression levels of Tert were analysed by q-RT-PCR using two different sets of primers, set 1 and set 2. Set 1 was composed of the forward (5'- TAG CCC TGG CAA GGA TAG A -3') and the reverse primer (5'- GCT CTC GGT GAT GTA GAA GAA G -3'). Set 2 was composed of the forward primer (5'- CAC CAG ACA CAA CGA GAG AAG -3') and the reverse primer (5'- CTC GAC CTT CAT CTT CCA CAT C -3'). The RNA samples were extracted with trizol and zirconium beads. The cDNA was synthesized using iScript Adv cDNA kit for RT-qPCR (Bio-Rad, Ref: 1725037; Batch: 64375699).

*Viral genome quantification*

The DNA from the indicated tissues were extracted using a alt precipitation-based commercial kit (MasterPureTM DNA purification Kit, Epicentre, Lit. # 113 • 6/2012 EPILIT113 Rev. A, Cat. No. MCD85201). The number of viral genomes per cells was analyzed by qPCR using primer sets 1 and 2 (see above). A calibration curve was prepared by serial dilution of a linearized plasmid harboring moTert gene.

*Telomerase activity*

Telomerase activity was analyzed by telomeric repeat amplification protocol (TRAP) as described in Herbert et al., 2006 (Herbert et al., Nature protocols, 2006) and as described in Blasco et al1997 (Blasco et al., Cell 1997).

*Telomerase RNA scope*

A specific probe for moTert RNA was designed and purchased from ACDBIO (www.acdbio.com) to analyze lung transduction efficacy as well as to identified specific cell types expressing the moTert. Double immune staining with Prosurfactant Protein C (SP-C) with a rabbit monoclonal (EPR19839) anti-SPC (1:100; ABCAM, Ref: ab211326) and moTert RNA scope was performed to quantitatively analyzed the percentage of Alveolar type II cells expressing moTert. Double immune staining with Secretoglobin Family 1A Member 1 (SCGB1A1 or CC10) in lung sections was performed with a goat polyclonal (EPR19839) anti-CC10 (1:100; ABCAM, Ref: ab211326) and moTert RNA scope was performed to quantitatively analyzed the percentage of Clara cells expressing moTert.

*Telomere length analysis*

Double fluorescence in situ hybridization with the moTert RNA scope and a telomeric probe (q-Fish) was performed to analyze telomere length in cells expressing the moTert.

*Data Analysis and Management*

Statistical significance is addressed by an unpaired Student Test, two-tailed.

*RESULTS*

Lungs (6 weeks post-infection)

- Increased telomerase activity in lung. Good correlation between expression and TRAP.
- Over 20% cells positively stained for moTERT in lung (RNA scope) (see figure 46).
- Co-staining with SPC (ATII cells) and CC10 (Clara cells) (see figure 47)
- Telomere quantification (length). Comparing cells in same animal (infected vs non-infected cells). Telomeric lengthening is 4.5% (D1) 7.5% (D2) in average (see figure 48).
- Mean nuclear intensity. Another readout for telomere length (7.8%) (see figure 48)
- Number of spots increases. Lengthening short telomeres (8-10%) (see figure 48)
- Telomeric distributions showed that in cells transduced with AAV6-SpB-Tert the percentage of short telomeres (20 % percentile of untransduced cells) decreases and that the percentage of long telomeres (80 % percentile of untransduced cells) increases. Moreover, the number of telomeric spots also increases in cells transduced with AAV6-SpB-Tert as compared to cells non-transduced in the same mouse. These results indicate that short telomeres are indeed elongated (see figure 49).

Liver

- No telomerase expression detected in liver *in vivo* (see figure 44)

*Example 6. Evaluation of telomerase expression levels in lungs upon intratracheal administration of AAV6-SPB-MOTERT*

In this example we aim to address the efficacy of AAV6-SpB-moTert treatment in the regression and/or curation of lung fibrosis in the short and in the long-term. The effects of the AAV6-SpB-moTert treatment in survival of mice diagnosed with PF (pulmonary fibrosis). For this example, we have used the same mouse model of pulmonary fibrosis as described in Povedano et al, 2015; Povedano et al., 2018. We have used 20-30 male mice/experimental groups. PF mice were IT (intratracheally) treated with AAV6-SpB-moTert at $5x10^{11}$ vg/mouse dose. As control, another cohort of PF mice received AAV6-SpB-null vectors at $5x10^{11}$ vg/mouse. Three weeks post-Bleo insult a computerized tomography (CT) was performed for each mouse to diagnosed aberrant lung pattern as a read out of pulmonary fibrosis.

Mice diagnosed with pulmonary fibrosis were treated with AAV6-SpB-moTert vectors using intratracheal administration. One, two, four, six and ten weeks post AAV6 treatment a CT was performed for longitudinal follow-up of the disease progression short term. Ten mice per group were left for survival and long-term analysis. Immunohistopathological studies of the lung were performed by immunohistochemistry. In addition, telomere length studies were carried out by quantitative fluorescence in situ hybridization techniques (q-Fish). Expression levels of Tert were determined in lungs and liver by q-PCR and Telomerase activity was evaluated by TRAP analysis at the HEP .

The objectives of this example are summarized herein below as follows:

- Telomerase expression and activity in AAV6-SpB-moTert transduced mice by intratracheal administration in telomerase deficient fibrotic mice.
- Comparison between healthy and fibrotic mice at $5x10^{11}$ vg/mouse.
- Telomere lengthening.
- Efficacy of the telomerase gene therapy for the treatment of PF (pulmonary fibrosis).

*Study Design*

*Vectors:*

- AAV6-SpB-null (SEQ ID NO 28)
- AAV6-SpB-moTert (SEQ ID NO 21)

*Dosages:*

- 5x10E11 vg/mouse

*Material and Methods*

*Test and control articles*

The viral vectors to be tested were AAV6-SpB-null and AAV6-SpB-moTert. AAV6-SpB-null was used as negative control for telomerase expression.

Tissue samples to be analysed by qPCR were collected on dry ice and tissue samples for immunohistochemistry analysis were directly collected in formalin to be further embedded in paraffin blocks.

*Telomerase expression.*

The transcriptional expression levels of Tert were analysed by q-RT-PCR using primer set 1 composed of the forward (5'- TAG CCC TGG CAA GGA TAG A -3') and the reverse primer (5'- GCT CTC GGT GAT GTA GAA GAA G -3').

The RNA samples were extracted with trizol and zirconium beads. The cDNA was synthesized using iScript Adv cDNA kit for RT-qPCR (Bio-Rad, Ref: 1725037; Batch: 64375699).

*Viral genome quantification*

The DNA from the indicated tissues were extracted using a precipitation-based commercial kit (MasterPureTM DNA purification Kit, Epicentre, Lit. # 113 • 6/2012 EPILIT113 Rev. A, Cat. No. MCD85201). The number of viral genomes per cells was analyzed by qPCR using primer sets 1 and 2. Set 1 is composed of the forward (5'- TAG CCC TGG CAA GGA TAG A -3') and the reverse primer (5'- GCT CTC GGT GAT GTA GAA GAA G -3'). Set 2 is composed of the forward primer (5'- CAC CAG ACA CAA CGA GAG AAG -3') and the reverse primer (5'- CTC GAC CTT CAT CTT CCA CAT C -3'). A calibration curve was prepared by serial dilution of a linearized plasmid harboring moTert gene.

*Telomerase activity*

Telomerase activity was analyzed by telomeric repeat amplification protocol (TRAP) as described in Herbert et al., 2006 (Herbert BS, Hochreiter AE, Wright WE, Shay JW. Nonradioactive detection of telomerase activity using the telomeric repeat amplification protocol. Nat Protoc. 2006;1(3):1583-90. Epub 2007/04/05. doi: 10.1038/nprot.2006.239. PubMed PMID: 17406450) and as described in Blasco et al1997 (Blasco MA, Lee HW, Hande MP, Samper E, Lansdorp PM, DePinho RA, et al. Telomere shortening and tumor formation by mouse cells lacking telomerase RNA. Cell. 1997;91(1):25-34. Epub 1997/10/23. doi: 10.1016/s0092-8674(01)80006-4. PubMed PMID: 9335332.).

*Telomerase RNA scope*

A specific probe for moTert RNA was designed and purchased from ACDBIO (www.acdbio.com) to analyze lung transduction efficacy as well as to identified specific cell types expressing the moTert. Double immune staining with Prosurfactant Protein C (SP-C) with a rabbit monoclonal (EPR19839) anti-SPC (1:100; ABCAM, Ref: ab211326) and moTert RNA scope was performed to quantitatively analyzed the percentage of Alveolar type II cells expressing moTert. Double immune staining with Secretoglobin Family 1A Member 1 (SCGB1A1 or CC10) in lung sections was performed with a goat polyclonal (EPR19839) anti-CC10 (1:100; ABCAM, Ref: ab211326) and moTert RNA scope was performed to quantitatively analyzed the percentage of Clara cells expressing moTert.

*Telomere length analysis*

Double fluorescence in situ hybridization with the moTert RNA scope and a telomeric probe (q-Fish) was performed to analyze telomere length in cells expressing the moTert.

*RESULTS*

- Similar transduction efficiency in Tert-ko fibrotic mice as compared to wild type mice. Over 20% cells positively stained for moTERT in lung (RNA scope) (Figure 51)
- Similar levels of viral genomes in Tert-ko fibrotic mice as compared to wild type mice. (Figure 52)
- No viral genomes detected in liver (Figure 52)
- Telomerase expression as easily detected in lungs at 6 and 11 weeks post AAV6-SpB-moTert treatment (Figure 53)
- No telomerase expression detected in liver, heart and brain (Figure 53).

*Example 7. Design and generation of codon-optimized human telomerase coding sequences*

To improve gene expression and increase the translational efficiency of human TERT transgene, mRNA sequence was optimized. Codons represent the genetic code that transfers information from mRNA to protein. This process is mediated by tRNAs codon recognition and thus specific aminoacid incorporation to the polypeptide chain. Since one single aminoacid could be encoded by different codons there exists several tRNA encoding the same aminoacid. This results in preferential usage of one codon over another for the same amino acid in a specie specific manner. This phenomenon is known as codon bias. Thus, optimization of codon usage to a specific specie is broadly used to increase protein production from a transgene. Furthermore, there also exist parameters with important impacts on protein expression, such as the GC content, RNA secondary structure, repeats, and others that are incorporated also into the codon optimization algorithms. Many websites and software incorporate codon optimization algorithms with different approaches. Due to the complex nature of the optimization process, there is no clear advantage from using one particular over another, so several in-parallel optimizations are required to identify the best one.

hsoTERT_ID was generated using variant 1 codon optimization tool, hsoTERT_GS was generated using variant 2 codon optimization tool, and hsoTERT_GA was generated using variant 3 codon optimization tool. Finally, all the sequences incorporate specific restriction sites on 3' and 5' to clone in the final AAV vector (Figure 54) and ordered by gene synthesis.

*Example 8. Characterization of AAV6-SpB-hoTERT-ID vectors*

AAV6-SpB-hsoTERT-ID vectors presented a main band of full vectors and small bands of lower and higher sedimentation coefficient that may correspond to intermediate species and aggregates respectively (Figure 55). This batch didn't present empty particles. Analysis of vector genome integrity revealed that AAV6-SpB-hsoTERT-ID vectors presented a main band around 4700 that corresponded to full-length vector genomes (Figure 56).

**Sequences**

| SEQ ID NO | Name | Description | Sequence type |
|---|---|---|---|
| 1 | mTERT CDS + native 3' UTR | Mouse TERT CDS with native 3' UTR | nucleotide |
| 2 | mTERT CDS | Mouse TERT CDS | nucleotide |
| 3 | moTERT | Mouse TERT CDS codon optimized | nucleotide |
| 4 | mTERT protein | Mouse TERT protein | amino acid |
| 5 | hTERT long isoform | Human TERT long isoform CDS | nucleotide |
| 6 | hoTERT long isoform | Human TERT long isoform CDS codon optimized | nucleotide |
| 7 | hTERT long isoform protein | Human TERT long isoform protein | amino acid |
| 8 | hTERT short isoform | Human TERT short isoform CDS | nucleotide |
| 9 | hoTERT short isoform | Human TERT short isoform CDS codon optimized | nucleotide |
| 10 | hTERT short isoform protein | Human TERT short isoform protein | amino acid |
| 11 | SpB promoter full length | Full length version of surfactant protein B promoter | nucleotide |
| 12 | SpB promoter shortened | Shortened version of surfactant protein B promoter | nucleotide |
| 13 | MLC2v promoter shortened | Shortened version of ventricular myosin regulatory light chain 2 promoter | nucleotide |
| 14 | CMV enhancer | cytomegalovirus enhancer | nucleotide |
| 15 | CMVenh-MLC2v promoter | Fusion of CMV enhancer and shortened version of MLC2v promoter | nucleotide |
| 16 | TNT promoter shortened | Shortened version of human troponin promoter | nucleotide |
| 17 | kozak sequence | kozak sequence | nucleotide |
| 18 | BGH polyA | Bovine growth hormone polyA sequence | nucleotide |
| 19 | 5' ITR AAV2 | 5' ITR of AAV2 | nucleotide |
| 20 | 3' ITR AAV2 | 3' ITR of AAV2 | nucleotide |
| 21 | AAV-SpB-moTERT recombinant adeno-associated virus genome | Lung expression vector viral genome | nucleotide |

(continued)

| SEQ ID NO | Name | Description | Sequence type |
|---|---|---|---|
| 22 | AAV9-CMVenh-MLC2v-moTERT genome | Heart expression vector viral genome | nucleotide |
| 23 | AAV9-TNT-moTert genome | Heart expression vector viral genome | nucleotide |
| 24 | AAV9-CMV-null genome and AAV6-CMV-null genome | Expression vector viral genomes | nucleotide |
| 25 | AAV9-CMV-Tert viral genome | Expression vector viral genome | nucleotide |
| 26 | AAV-CMV-GFP recombinant adeno-associated virus genome | Expression vector viral genome | nucleotide |
| 27 | AAV-SpB-GFP recombinant adeno-associated virus genome | Lung expression vector viral genome | nucleotide |
| 28 | AAV6-SpB-null genome | Lung expression vector viral genome | nucleotide |

SEQ ID NO: 6 (hoTERT long isoform)

```
atgcctagag cacctagatg tagagctgtg cggagcctgc tgcggagcca ctatagagaa
gttctgcccc tggccacctt cgtgcgtaga cttggacctc aaggatggcg gctggtgcag
agaggcgatc ctgctgcttt tagagccctg gtggcccagt gtctcgtgtg cgttccatgg
gatgctagac ctccaccagc tgctcccagc ttcagacagg tgtcctgcct gaaagaactg
gtggccagag tgctgcagcg gctgtgtgaa aggggcgcca aaaatgtgct ggccttcggc
tttgctctgc tggatggtgc tagaggcgga cctcctgagg cctttacaac aagcgtgcgg
agctacctgc ctaacaccgt gacagatgcc ctgagaggat ctggcgcttg gggactgctg
ctgagaagag tgggagatga cgtgctggtg catctgctgg ctagatgcgc cctgtttgtg
ctggtggctc ctagctgtgc ctaccaagtc tgtggccctc cactgtatca gctgggcgct
gctacacagg ctagaccacc tccacatgcc agcggaccta gaagaaggct gggctgcgaa
agagcctgga accactctgt tagagaagcc ggcgtgccac tgggattgcc tgcacctggt
gcaagaagaa gaggcggcag cgcctctaga tctctgcctc tgcctaagag gcctcggaga
ggtgctgctc ctgagcctga gagaacacct gttggccaag gctcttgggc ccatcctggc
agaacaagag gccctagcga tagaggcttc tgcgtggtgt ctcctgccag acctgccgag
gaagccacat ctcttgaagg cgccctgagc ggcacaagac actctcaccc atctgtgggc
agacagcacc atgccggacc tccaagcaca agcagaccac ctagaccttg ggacacccct
tgtcctccag tgtacgccga dacaaagcac ttcctgtaca gcagcggcga caaagagcag
ctgaggccta gcttcctgct gtcctctctg aggccatctc tgaccggtgc tcggagactg
gtggaaacca tcttcctggg cagcagacct tggatgcccg gcacacctag aaggctgcct
agactgccac agcggtactg gcaaatgagg cccctgttcc tggaactgct gggcaatcac
```

```
gctcagtgcc cttatggcgt gctgctgaaa acccactgtc ctctgagagc cgccgtgaca
ccagcagctg gcgtttgtgc cagagagaag cctcaaggct ctgtggctgc ccctgaggaa
gaggacacag atcctagacg actggtgcag ctcctgcggc agcattctag tccatggcag
gtctacggat tcgtgcgggc ctgtctgaga aggcttgttc ctcctggact gtggggctcc
agacacaacg agcggcggtt tctgcggaac accaagaagt tcatcagcct gggaaagcac
gccaagctga gcctgcaaga gctgacctgg aagatgagcg tgcgggattg tgcatggctg
agaaggtccc caggcgtggg atgtgttcct gccgctgaac acagactgcg ggaagagatc
ctggccaagt tcctgcactg gctgatgtcc gtgtacgtgg tcgaactgct tcggagcttc
ttctacgtga ccgagacaac cttccagaag aaccggctgt tcttctaccg gaagtccgtg
tggtccaagc tgcagagcat cggcattcgg cagcacctga agagagtgca gctgagagag
ctgagcgagg ctgaagtccg gcagcacaga gaagctagac cagctctgct gaccagcagg
ctgagattca tccccaagcc tgatggcctg cggcctatcg tgaacatgga ctatgttgtg
ggcgccagaa cctttcggag agagaagaga gccgagcggc tgacctctag agtgaaggcc
ctgttcagcg tgctgaacta cgagagagcc agaaggcctg gactgctcgg agcctctgtt
ctgggcctcg acgatatcca cagagcttgg cggacctttg tgctgagagt cagagcccag
gatcctccac ctgagctgta cttcgtgaag gtggacgtga ccggcgccta cgacacaatc
cctcaggaca gactgaccga agtgatcgcc agcatcatca gcccccagaa cacctactgt
gtgcggagat acgccgtggt gcagaaagcc gctcatggac atgtgcgcaa ggccttcaag
tcccacgtgt ccacactgac cgacctgcag ccttacatga dacagttcgt ggcccatctg
caagagacaa gccctctgag ggatgccgtg gtcatcgaac agagcagcag cctgaatgag
gccagctccg gcctgtttga cgtgttcctc agattcatgt gccaccacgc cgtgcggatc
agaggcaaga gctatgtgca gtgccagggc attccacagg gcagcatcct gagcacactg
ctgtgcagcc tgtgctacgg cgacatggaa aacaagctgt cgccggcat cagacgcgac
ggcctgcttc tgagactggt cgacgatttc ctgctcgtga cccctcacct gacacacgcc
aagacctttc tgagaacact cgtgcggggc gtgccagagt atggctgtgt ggtcaacctg
agaaagaccg tggtcaactt ccccgtcgag gatgaagccc ttggcggcac agctttcgtg
cagatgcctg ctcacggact gttcccttgg tgcggactgc tcctggacac cagaacactg
gaagtgcaga gcgactacag cagctacgcc cggacatcta tcagagccag cctgaccttc
aaccggggct ttaaggccgg cagaaacatg cggagaaagc tgtttggagt gctgcggctg
aagtgccact ctttgtttct ggacctgcaa gtgaacagcc tgcagaccgt gtgcaccaac
atctacaaga ttctgctgct gcaagcctac cggttccacg cctgtgttct gcagctgccc
ttccaccagc aagtgtggaa gaaccctaca ttcttcctgc gcgtgatcag cgacaccgcc
agcctgtgtt actccatcct gaaggccaag aacgccggca tgtctctggg agctaaaggc
gctgctggac ctctgccttc tgaagcagtg cagtggctct gccaccaggc ctttctgctg
aagctgacca gacacagagt gacctacgtg ccctgctgg gctcactgag aacagctcag
acacagctga gcagaaagct gcctggcaca accctgacag ccctggaagc tgcagcaaac
cctgctctgc ccagcgactt caagaccatc ctggattga
```

48

SEQ ID NO: 9 (hoTERT short isoform)

atgcctcggg ctcctagatg tagagccgtc agaagcctgc tgcggagcca ctatagagag

gtgctgcctc tggccacctt cgtgcgtaga cttggacctc aaggatggcg gctggtgcag

agaggcgatc ctgctgcttt tagagccctg gtggcccagt gtctcgtgtg cgttccatgg

gatgctagac ctccaccagc tgctcccagc ttcagacagg tgtcctgcct gaaagaactg

gtggccaggg tgctgcagag actgtgtgaa aggggcgcca agaacgtgct ggcctttgga

tttgctctgc tggatggcgc tagaggcgga cctcctgagg cctttacaac aagcgtgcgg

agctacctgc ctaacaccgt gacagatgcc ctgagaggat ctggcgcttg gggactgctg

ctgagaagag tgggagatga cgtgctggtg catctgctgg ccagatgcgc tctgtttgtg

ctggtggctc ctagctgcgc ctaccaagtt tgtggccctc cactgtatca gctgggcgct

gctacacagg ctagaccacc tccacatgcc agcggaccta gaagaaggct gggctgcgaa

agagcctgga accactctgt tagagaagcc ggcgtgccac tgggattgcc tgcaccaggt

gcaagaagaa gaggcggcag cgcctctaga tctctgcctc tgcctaagag gcctagaaga

ggggctgccc ctgagcctga gagaacacct gttggccaag gctcttgggc ccatcctggc

agaacaagag gccctagcga tagaggcttc tgcgtggtgt ctcctgccag acctgccgag

gaagccacat ctcttgaagg cgccctgagc ggcacaagac actctcaccc atctgtgggc

agacagcacc atgccggacc tccaagcaca agcagaccac tagaccttg ggacacccct

tgtcctccag tgtacgccga gacaaagcac ttcctgtaca gcagcggcga caaagagcag

ctgaggccta gcttcctgct gtcctctctg aggccatctc tgaccggtgc tcggagactg

gtggaaacca tcttcctggg cagcagacct tggatgcccg gcacacctag aaggctgcct

agactgccac agcggtactg gcaaatgagg cccctgttcc tggaactgct gggcaatcac

gctcagtgcc cttatggcgt gctgctgaaa acccactgtc ctctgagagc cgccgtgaca

ccagcagctg gcgtttgtgc cagagagaag cctcaaggct ctgtggccgc tcctgaggaa

gaggacacag atcctagacg actggtgcag ctcctgagac agcacagctc tccatggcag

gtctacggat ttgtgcgggc ctgtctgaga aggctcgttc ctcctggact gtggggctcc

agacacaacg agcggcggtt tctgcggaac accaagaagt tcatcagcct gggaaagcac

gccaagctga gcctgcaaga gctgacctgg aagatgagcg tgcgggattg tgcatggctg

agaaggtccc caggcgtggg atgtgttcct gccgctgaac acagactgcg ggaagagatc

ctggccaagt tcctgcactg gctgatgtcc gtgtacgtgg tcgaactgct tcggagcttc

ttctacgtga ccgagacaac cttccagaag aaccggctgt tcttctaccg gaagtccgtg

tggtccaagc tgcagagcat cggcatccgg cagcatctga agagagtgca gctgagagag

ctgagcgaag ccgaagtgcg gcagcacaga gaagctagac cagctctgct gaccagcagg

ctgagattca tccccaagcc tgatggcctg cggcctatcg tgaacatgga ctatgttgtg

ggcgccagaa cctttcggag agagaagaga gccgagcggc tgacctctag agtgaaggcc

ctgttcagcg tgctgaacta cgagagagcc agaaggccag gactgctggg agcctctgtt

ctgggcctcg acgatatcca cagagcttgg cggacctttg tgctgagagt gcgagcccaa

gatcctccac ctgagctgta cttcgtgaag gtggacgtga ccggcgccta cgacacaatc

cctcaggaca gactgaccga agtgatcgcc agcatcatca agccccagaa cacctactgt
gtgcggagat acgccgtggt gcagaaagcc gctcatggac atgtgcgcaa ggccttcaag
tcccacgtgt ccacactgac cgacctgcag ccttacatga gacagttcgt ggcccatctg
caagagacaa gccctctgag ggatgccgtg gtcatcgaac agagcagcag cctgaatgag
gccagctccg gcctgtttga cgtgttcctc agattcatgt gccaccacgc cgtgcggatc
agaggcaaga gctatgtgca gtgccagggc attcctcagg cagcatcct gagcacactg
ctgtgcagcc tgtgctacgg cgacatggaa aacaagctgt tcgccggcat cagacgcgac
ggcctgcttc tgagactggt cgacgatttc ctgctcgtga ccccctcacct gacacacgcc
aagacctttc tgagctacgc ccggacctct atcagagcca gcctgacctt caaccggggc
tttaaggccg gcagaaacat gcggagaaag ctgtttggag tgctgcggct gaagtgccac
tctttgtttc tggacctgca agtgaacagc ctgcagaccg tgtgcaccaa catctacaag
attctgctgc tgcaagccta ccggttccac gcctgtgttc tgcagctgcc ctttcaccag
caagtgtgga agaaccctac attcttcctg cgcgtgatca gcgacaccgc cagcctgtgt
tactccatcc tgaaggccaa aaacgccggc atgagcctgg gagctaaagg cgctgctgga
cctctgcctt ctgaagcagt gcagtggctg tgtcaccagg cctttctgct gaagctgacc
cggcacagag tgacatatgt gcctctgctg ggctccctga gaaccgctca aacacagctg
agcagaaagc tgcctggcac aaccctgaca gccctggaag ctgcagcaaa ccctgctctg
cccagcgact tcaagaccat cctggattga

SEQ ID NO: 12 (SpB promoter shortened)

ATAGGGCTGTCTGGGAGCCACTCCAGGGCCACAGAAATCTTGTCTCTGACTCAGGGTATTTTGTTTTCTGTT
TTGTGTAAATGCTCTTCTGACTAATGCAAACCATGTGTCCATAGAACCAGAAGATTTTTCCAGGGGAAAAGGT
AAGGAGGTGGTGAGAGTGTCCTGGGTCTGCCCTTCCAGGGCTTGCCCTGGGTTAAGAGCCAGGCAGGAAG
CTCTCAAGAGCATTGCTCAAGAGTAGAGGGGGCCTGGGAGGCCCAGGGAGGGGATGGGAGGGGAACACC
CAGGCTGCCCCCAACCAGATGCCCTCCACCCTCCTCAACCTCCCTCCCACGGCCTGGAGAGGTGGGACCA
GGTATGGAGGCTTGAGAGCCCCTGGTTGGAGGAAGCCACAAGTCCAGGAACATGGGAGTCTGGGCAGGGG
GCAAAGGAGGCAGGAACAGGCCATCAGCCAGGACAGGTGGTAAGGCAGGCAGGAGTGTTCCTGCTGGGAA
AAGGTGGGATCAAGCACCTGGAGGGCTCTTCAGAGCAAAGACAAACACTGAGGTCGCTGCCACTCCTACAG
AGCCCCCACGCCCCGCCCAGCTATAAGGGGCCATGCACCAAGCAGGGTACCCAGGCTGCAGAGGTGC

SEQ ID NO: 15 (CMVenh-MLC2v promoter)

GACATTGATTATTGACTAGTTATTAATAGTAATCAATTACGGGGTCATTAGTTCATAGCCCATATATGGAGTTC
CGCGTTACATAACTTACGGTAAATGGCCCGCCTGGCTGACCGCCCAACGACCCCCGCCCATTGACGTCAAT
AATGACGTATGTTCCCATAGTAACGCCAATAGGGACTTTCCATTGACGTCAATGGGTGGACTATTTACGGTAA
ACTGCCCACTTGGCAGTACATCAAGTGTATCATATGCCAAGTACGCCCCCTATTGACGTCAATGACGGTAAA
TGGCCCGCCTGGCATTATGCCCAGTACATGACCTTATGGGACTTTCCTACTTGGCAGTACATCTACGTATTA
GTCATCGCTATTACCATGGTTAACGTTTAAACTTAGACAATGGCAGGACCCAGAGCACAGAGCATCGTTCCC

AGGCCAGGCCCCAGCCACTGTCTCTTTAACCTTGAAGGCATTTTTGGGTCTCACGTGTCCACCCAGGCGGG
TGTCGGACTTTGAACGGCTCTTACTTCAGAAGAACGGCATGGGGTGGGGGGGCTTAGGTGGCCTCTGCCTC
ACCTACAACTGCCAAAAGTGGTCATGGGGTTATTTTTAACCCCAGGGAAGAGGTATTTATTGTTCCACAGCA
GGGGCCGGCCAGCAGGCTCCTTGAATTC

SEQ ID NO: 16 (TNT promoter shortened)

CTCAGTCCATTAGGAGCCAGTAGCCTGGAAGATGTCTTTACCCCCAGCATCAGTTCAAGTGGAGCAGCACAT
AACTCTTGCCCTCTGCCTTCCAAGATTCTGGTGCTGAGACTTATGGAGTGTCTTGGAGGTTGCCTTCTGCCC
CCCAACCCTGCTCCCAGCTGGCCCTCCCAGGCCTGGGTTGCTGGCCTCTGCTTTATCAGGATTCTCAAGAG
GGACAGCTGGTTTATGTTGCATGACTGTTCCCTGCATATCTGCTCTGGTTTTAAATAGCTTATCTGAGCAGCT
GGAGGACCACATGGGCTTATATGGCGTGGGGTACATGTTCCTGTAGCCTTGTCCCTGGCACCTGCCAAAAT
AGCAGCCAACACCCCCCACCCCCACCGCCATCCCCCTGCCCCACCCGTCCCCTGTCGCACATTCCTCCCTC
CGCAGGGCTGGCTCACCAGGCCCCAGCCCACATGCCTGCTTAAAGCCCTCTCCATCCTCTGCCTCACCCAG
TCCCCGCTGAGACTGAGCAGACGCCTCCAGGATCTGTCGGCAG

SEQ ID NO: 21 (AAV-SpB-moTERT recombinant adeno-associated virus genome)

CCTGCAGGCAGCTGCGCGCTCGCTCGCTCACTGAGGCCGCCCGGGCAAAGCCCGGGCGTCGGGCGACCT
TTGGTCGCCCGGCCTCAGTGAGCGAGCGAGCGCGCAGAGAGGGAGTGGCCAACTCCATCACTAGGGGTTC
CTGCGGCCGCGATATCATAGGGCTGTCTGGGAGCCACTCCAGGGCCACAGAAATCTTGTCTCTGACTCAGG
GTATTTTGTTTTCTGTTTTGTGTAAATGCTCTTCTGACTAATGCAAACCATGTGTCCATAGAACCAGAAGATTT
TTCCAGGGGAAAAGGTAAGGAGGTGGTGAGAGTGTCCTGGGTCTGCCCTTCCAGGGCTTGCCCTGGGTTAA
GAGCCAGGCAGGAAGCTCTCAAGAGCATTGCTCAAGAGTAGAGGGGGCCTGGGAGGCCCAGGGAGGGGA
TGGGAGGGGAACACCCAGGCTGCCCCCAACCAGATGCCCTCCACCCTCCTCAACCTCCCTCCCACGGCCT
GGAGAGGTGGGACCAGGTATGGAGGCTTGAGAGCCCCTGGTTGGAGGAAGCCACAAGTCCAGGAACATGG
GAGTCTGGGCAGGGGGCAAAGGAGGCAGGAACAGGCCATCAGCCAGGACAGGTGGTAAGGCAGGCAGGA
GTGTTCCTGCTGGGAAAAGGTGGGATCAAGCACCTGGAGGGCTCTTCAGAGCAAAGACAAACACTGAGGTC
GCTGCCACTCCTACAGAGCCCCCACGCCCCGCCCAGCTATAAGGGGCCATGCACCAAGCAGGGTACCCAG
GCTGCAGAGGTGCGAATTCATTTAAATTCTAGATCGCTAGCACGCGTGCCACCATGACAAGAGCCCCTAGAT
GTCCTGCCGTCAGAAGCCTGCTGAGAAGCAGATACAGAGAAGTGTGGCCCCTGGCCACCTTCGTCAGAAGG
CTTGGACCTGAAGGCAGACGGCTGGTTCAACCTGGCGACCCCAAGATCTACAGAACCCTGGTGGCTCAGTG
CCTCGTGTGTATGCACTGGGGATCTCAGCCACCTCCTGCCGATCTGAGCTTTCACCAGGTGTCCAGCCTGA
AAGAACTGGTGGCCAGAGTGGTGCAGAGACTGTGCGAGAGAAACGAGAGGAACGTGCTGGCCTTCGGCTT
CGAGCTGCTGAATGAAGCTAGAGGCGGCCCTCCTATGGCCTTCACATCTAGCGTGCGGAGCTACCTGCCTA
ACACCGTGATCGAGACACTGAGAGTGTCCGGCGCTTGGATGCTGCTGCTGAGCAGAGTGGGAGATGACCT
GCTGGTGTACCTGCTGGCTCACTGTGCCCTCTATCTGCTGGTGCCTCCTAGCTGCGCTTACCAAGTGTGCG
GAAGCCCTCTGTACCAGATCTGTGCCACCACCGACATCTGGCCTAGCGTGTCCGCTTCCTACAGACCCACA
AGACCCGTGGGCAGAAACTTTACCAACCTGAGATTCCTGCAGCAGATCAAGAGCAGCTCCAGGCAAGAGGC

CCCTAAGCCTCTTGCTCTGCCTAGCAGAGGCACCAAGAGACACCTGAGCCTGACCAGCACAAGCGTGCCCT
CTGCCAAGAAAGCCAGATGCTACCCCGTGCCTAGAGTGGAAGAGGGCCCACATAGACAGGTGCTGCCTACA
CCTAGCGGCAAGAGCTGGGTTCCATCTCCTGCTAGAAGCCCTGAGGTGCCAACAGCCGAGAAGGACCTGA
GCAGCAAGGGCAAAGTGTCCGACCTGTCTCTGAGCGGCAGCGTGTGTTGCAAGCACAAGCCTAGCAGCAC
CAGCCTGCTGTCCCCACCTAGACAGAACGCTTTCCAGCTGAGGCCCTTTATCGAGACAAGACACTTCCTGTA
CAGCAGAGGCGACGGCCAAGAGAGACTGAACCCTAGCTTCCTGCTGTCTAACCTGCAGCCTAACCTGACCG
GCGCTAGAAGGCTGGTGGAAATCATCTTCCTGGGCAGCAGACCCAGAACCAGCGGACCTCTGTGTAGAACC
CACAGACTGAGCAGACGGTACTGGCAGATGAGGCCCCTGTTCCAGCAGCTCCTGGTCAACCATGCCGAGTG
TCAGTATGTGCGGCTGCTGAGGTCCCACTGCAGATTCAGAACCGCCAACCAGCAAGTGACAGACGCCCTGA
ACACAAGCCCTCCACACCTGATGGATCTGCTGAGACTGCACAGCAGCCCCTGGCAGGTCTACGGCTTTCTG
AGAGCCTGTCTGTGCAAGGTGGTGTCCGCATCTCTGTGGGGCACCAGACACAACGAGAGAAGATTCTTCAA
GAACCTGAAGAAGTTCATCAGCCTGGGGAAGTACGGCAAGCTGTCCCTGCAAGAACTGATGTGGAAGATGA
AGGTCGAGGACTGCCACTGGCTGAGGTCTAGCCCTGGCAAGGATAGAGTGCCTGCCGCTGAGCACAGGCT
GAGAGAGAGAATCCTGGCCACATTCCTGTTCTGGCTGATGGACACCTACGTGGTGCAGCTGCTGCGGAGCT
TCTTCTACATCACCGAGAGCACCTTCCAGAAGAACCGGCTGTTCTTCTACCGCAAGAGCGTGTGGTCCAAGC
TGCAGAGCATCGGCGTCAGACAGCACCTGGAAAGAGTGCGCCTGAGAGAGCTGAGCCAAGAGGAAGTGCG
GCACCACCAGGATACCTGGCTGGCCATGCCTATCTGCAGACTGAGATTCATCCCCAAGCCTAACGGCCTGA
GGCCTATCGTGAACATGAGCTACAGCATGGGCACTAGAGCCCTGGGCAGAAGAAAGCAGGCCCAGCACTTC
ACCCAGAGGCTGAAAACCCTGTTCTCCATGCTGAACTACGAGCGGACAAAGCACCCTCATCTGATGGGCTC
CTCTGTGCTGGGCATGAACGACATCTACAGGACCTGGCGGGCCTTCGTGCTGAGAGTCAGAGCACTGGACC
AGACACCTAGAATGTACTTCGTGAAGGCCGACGTGACAGGCGCCTACGATGCTATCCCTCAGGGCAAACTG
GTGGAAGTGGTGGCCAACATGATCAGGCACAGCGAGTCCACCTACTGCATCAGACAGTACGCCGTCGTGCG
GAGAGACTCTCAGGGACAAGTGCACAAGAGCTTCCGCAGACAAGTGACCACACTGAGCGATCTGCAGCCCT
ACATGGGCCAGTTTCTGAAGCACCTCCAGGACAGCGACGCCAGCGCTCTGAGAAACTCTGTGGTCATCGAG
CAGTCCATCTCCATGAACGAGTCCAGCTCCAGCCTGTTCGACTTCTTCCTGCACTTCCTGCGGCACAGCGTG
GTCAAGATCGGCGACAGATGTTACACCCAGTGCCAGGGCATCCCACAGGGAAGCAGTCTGAGCACACTGCT
GTGCTCCCTGTGTTTCGGCGACATGGAAAACAAGCTGTTCGCCGAAGTGCAGCGCGACGGACTGCTCCTGA
GATTTGTGGACGACTTCCTGCTCGTGACCCCTCACCTGGATCAGGCTAAGACCTTCCTGAGCACCCTGGTG
CATGGCGTGCCAGAGTACGGCTGCATGATCAACCTGCAGAAAACCGTGGTCAACTTCCCCGTGGAACCTGG
CACTCTTGGAGGCGCTGCTCCTTATCAGCTGCCCGCTCATTGTCTGTTCCCTTGGTGCGGACTGCTGCTGGA
CACACAGACCCTGGAAGTGTTCTGCGACTACAGCGGCTACGCCCAGACCAGCATCAAGACCAGCCTGACCT
TCCAGTCCGTGTTCAAGGCCGGCAAGACCATGAGGAACAAGCTGCTGAGTGTGCTGAGGCTGAAGTGCCAC
GGCCTGTTTCTGGACCTGCAAGTGAACAGCCTGCAGACCGTGTGCATCAATATCTACAAGATCTTTCTGCTG
CAAGCCTACCGGTTCCACGCCTGTGTGATTCAGCTGCCTTTCGACCAGAGAGTGCGGAAGAATCTGACATTC
TTTCTGGGCATCATCAGCAGCCAGGCCAGCTGCTGTTACGCCATCCTGAAAGTGAAGAACCCCGGCATGAC
CCTGAAGGCCTCTGGCAGCTTTCCACCTGAAGCTGCCCATTGGCTGTGCTACCAGGCTTTCCTGCTGAAACT
GGCTGCCCACAGCGTGATCTACAAGTGCCTGCTGGGCCCACTGAGAACAGCCCAGAAACTGCTGTGTCGGA

AGCTGCCTGAAGCCACAATGACAATCCTGAAGGCTGCCGCCGATCCAGCTCTGAGCACCGACTTCCAGACC
ATCCTGGACTGAGAATTCGGATCCGATTCGAGCATTTAAATACGTGGAGCTCGCTGATCAGCCTCGACTGTG
CCTTCTAGTTGCCAGCCATCTGTTGTTTGCCCCTCCCCCGTGCCTTCCTTGACCCTGGAAGGTGCCACTCCC
ACTGTCCTTTCCTAATAAAATGAGGAAATTGCATCGCATTGTCTGAGTAGGTGTCATTCTATTCTGGGGGGTG
GGGTGGGGCAGGACAGCAAGGGGGAGGATTGGGAAGACAATAGCAGGCATGCTGGGGATGCGGTGGGCT
CTATGGCCACGTGGATATCGCGGCCGCAGGAACCCCTAGTGATGGAGTTGGCCACTCCCTCTCTGCGCGCT
CGCTCGCTCACTGAGGCCGGGCGACCAAAGGTCGCCCGACGCCCGGGCTTTGCCCGGGCGGCCTCAGTG
AGCGAGCGAGCGCGCAGCTGCCTGCAGG

SEQ ID NO: 22 (AAV9-CMVenh-MLC2v-moTERT virus genome)

CCTGCAGGCAGCTGCGCGCTCGCTCGCTCACTGAGGCCGCCCGGGCAAAGCCCGGGCGTCGGGCGACCT
TTGGTCGCCCGGCCTCAGTGAGCGAGCGAGCGCGCAGAGAGGGAGTGGCCAACTCCATCACTAGGGGTTC
CTGCGGCCGCGATATCGACATTGATTATTGACTAGTTATTAATAGTAATCAATTACGGGGTCATTAGTTCATA
GCCCATATATGGAGTTCCGCGTTACATAACTTACGGTAAATGGCCCGCCTGGCTGACCGCCCAACGACCCC
CGCCCATTGACGTCAATAATGACGTATGTTCCCATAGTAACGCCAATAGGGACTTTCCATTGACGTCAATGG
GTGGACTATTTACGGTAAACTGCCCACTTGGCAGTACATCAAGTGTATCATATGCCAAGTACGCCCCCTATTG
ACGTCAATGACGGTAAATGGCCCGCCTGGCATTATGCCCAGTACATGACCTTATGGGACTTTCCTACTTGGC
AGTACATCTACGTATTAGTCATCGCTATTACCATGGTTAACGTTTAAACTTAGACAATGGCAGGACCCAGAGC
ACAGAGCATCGTTCCCAGGCCAGGCCCCAGCCACTGTCTCTTTAACCTTGAAGGCATTTTTGGGTCTCACGT
GTCCACCCAGGCGGGTGTCGGACTTTGAACGGCTCTTACTTCAGAAGAACGGCATGGGGTGGGGGGGCTT
AGGTGGCCTCTGCCTCACCTACAACTGCCAAAAGTGGTCATGGGGTTATTTTTAACCCCAGGGAAGAGGTAT
TTATTGTTCCACAGCAGGGGCCGGCCAGCAGGCTCCTTGAATTCATTTAAATTCTAGATCGCTAGCACGCGT
GCCACCATGACAAGAGCCCCTAGATGTCCTGCCGTCAGAAGCCTGCTGAGAAGCAGATACAGAGAAGTGTG
GCCCCTGGCCACCTTCGTCAGAAGGCTTGGACCTGAAGGCAGACGGCTGGTTCAACCTGGCGACCCCAAG
ATCTACAGAACCCTGGTGGCTCAGTGCCTCGTGTGTATGCACTGGGGATCTCAGCCACCTCCTGCCGATCT
GAGCTTTCACCAGGTGTCCAGCCTGAAAGAACTGGTGGCCAGAGTGGTGCAGAGACTGTGCGAGAGAAAC
GAGAGGAACGTGCTGGCCTTCGGCTTCGAGCTGCTGAATGAAGCTAGAGGCGGCCCTCCTATGGCCTTCAC
ATCTAGCGTGCGGAGCTACCTGCCTAACACCGTGATCGAGACACTGAGAGTGTCCGGCGCTTGGATGCTGC
TGCTGAGCAGAGTGGGAGATGACCTGCTGGTGTACCTGCTGGCTCACTGTGCCCTCTATCTGCTGGTGCCT
CCTAGCTGCGCTTACCAAGTGTGCGGAAGCCCTCTGTACCAGATCTGTGCCACCACCGACATCTGGCCTAG
CGTGTCCGCTTCCTACAGACCCACAAGACCCGTGGGCAGAAACTTTACCAACCTGAGATTCCTGCAGCAGAT
CAAGAGCAGCTCCAGGCAAGAGGCCCCTAAGCCTCTTGCTCTGCCTAGCAGAGGCACCAAGAGACACCTGA
GCCTGACCAGCACAAGCGTGCCCTCTGCCAAGAAAGCCAGATGCTACCCCGTGCCTAGAGTGGAAGAGGG
CCCACATAGACAGGTGCTGCCTACACCTAGCGGCAAGAGCTGGGTTCCATCTCCTGCTAGAAGCCCTGAGG
TGCCAACAGCCGAGAAGGACCTGAGCAGCAAGGGCAAAGTGTCCGACCTGTCTCTGAGCGGCAGCGTGTG
TTGCAAGCACAAGCCTAGCAGCACCAGCCTGCTGTCCCCACCTAGACAGAACGCTTTCCAGCTGAGGCCCT
TTATCGAGACAAGACACTTCCTGTACAGCAGAGGCGACGGCCAAGAGAGACTGAACCCTAGCTTCCTGCTG

TCTAACCTGCAGCCTAACCTGACCGGCGCTAGAAGGCTGGTGGAAATCATCTTCCTGGGCAGCAGACCCAG

AACCAGCGGACCTCTGTGTAGAACCCACAGACTGAGCAGACGGTACTGGCAGATGAGGCCCCTGTTCCAGC

AGCTCCTGGTCAACCATGCCGAGTGTCAGTATGTGCGGCTGCTGAGGTCCCACTGCAGATTCAGAACCGCC

AACCAGCAAGTGACAGACGCCCTGAACACAAGCCCTCCACACCTGATGGATCTGCTGAGACTGCACAGCAG

CCCCTGGCAGGTCTACGGCTTTCTGAGAGCCTGTCTGTGCAAGGTGGTGTCCGCATCTCTGTGGGGCACCA

GACACAACGAGAGAAGATTCTTCAAGAACCTGAAGAAGTTCATCAGCCTGGGGAAGTACGGCAAGCTGTCC

CTGCAAGAACTGATGTGGAAGATGAAGGTCGAGGACTGCCACTGGCTGAGGTCTAGCCCTGGCAAGGATAG

AGTGCCTGCCGCTGAGCACAGGCTGAGAGAGAATCCTGGCCACATTCCTGTTCTGGCTGATGGACACCT

ACGTGGTGCAGCTGCTGCGGAGCTTCTTCTACATCACCGAGAGCACCTTCCAGAAGAACCGGCTGTTCTTCT

ACCGCAAGAGCGTGTGGTCCAAGCTGCAGAGCATCGGCGTCAGACAGCACCTGGAAAGAGTGCGCCTGAG

AGAGCTGAGCCAAGAGGAAGTGCGGCACCACCAGGATACCTGGCTGGCCATGCCTATCTGCAGACTGAGAT

TCATCCCCAAGCCTAACGGCCTGAGGCCTATCGTGAACATGAGCTACAGCATGGGCACTAGAGCCCTGGGC

AGAAGAAAGCAGGCCCAGCACTTCACCCAGAGGCTGAAAACCCTGTTCTCCATGCTGAACTACGAGCGGAC

AAAGCACCCTCATCTGATGGGCTCCTCTGTGCTGGGCATGAACGACATCTACAGGACCTGGCGGGCCTTCG

TGCTGAGAGTCAGAGCACTGGACCAGACACCTAGAATGTACTTCGTGAAGGCCGACGTGACAGGCGCCTAC

GATGCTATCCCTCAGGGCAAACTGGTGGAAGTGGTGGCCAACATGATCAGGCACAGCGAGTCCACCTACTG

CATCAGACAGTACGCCGTCGTGCGGAGAGACTCTCAGGGACAAGTGCACAAGAGCTTCCGCAGACAAGTGA

CCACACTGAGCGATCTGCAGCCCTACATGGGCCAGTTTCTGAAGCACCTCCAGGACAGCGACGCCAGCGCT

CTGAGAAACTCTGTGGTCATCGAGCAGTCCATCTCCATGAACGAGTCCAGCTCCAGCCTGTTCGACTTCTTC

CTGCACTTCCTGCGGCACAGCGTGGTCAAGATCGGCGACAGATGTTACACCCAGTGCCAGGGCATCCCACA

GGGAAGCAGTCTGAGCACACTGCTGTGCTCCCTGTGTTTCGGCGACATGGAAAACAAGCTGTTCGCCGAAG

TGCAGCGCGACGGACTGCTCCTGAGATTTGTGGACGACTTCCTGCTCGTGACCCCTCACCTGGATCAGGCT

AAGACCTTCCTGAGCACCCTGGTGCATGGCGTGCCAGAGTACGGCTGCATGATCAACCTGCAGAAAACCGT

GGTCAACTTCCCCGTGGAACCTGGCACTCTTGGAGGCGCTGCTCCTTATCAGCTGCCCGCTCATTGTCTGTT

CCCTTGGTGCGGACTGCTGCTGGACACACAGACCCTGGAAGTGTTCTGCGACTACAGCGGCTACGCCCAGA

CCAGCATCAAGACCAGCCTGACCTTCCAGTCCGTGTTCAAGGCCGGCAAGACCATGAGGAACAAGCTGCTG

AGTGTGCTGAGGCTGAAGTGCCACGGCCTGTTTCTGGACCTGCAAGTGAACAGCCTGCAGACCGTGTGCAT

CAATATCTACAAGATCTTTCTGCTGCAAGCCTACCGGTTCCACGCCTGTGTGATTCAGCTGCCTTTCGACCA

GAGAGTGCGGAAGAATCTGACATTCTTTCTGGGCATCATCAGCAGCCAGGCCAGCTGCTGTTACGCCATCC

TGAAAGTGAAGAACCCCGGCATGACCCTGAAGGCCTCTGGCAGCTTTCCACCTGAAGCTGCCCATTGGCTG

TGCTACCAGGCTTTCCTGCTGAAACTGGCTGCCCACAGCGTGATCTACAAGTGCCTGCTGGGCCCACTGAG

AACAGCCCAGAAACTGCTGTGTCGGAAGCTGCCTGAAGCCACAATGACAATCCTGAAGGCTGCCGCCGATC

CAGCTCTGAGCACCGACTTCCAGACCATCCTGGACTGAGAATTCGGATCCGATTCGAGCATTTAAATACGTG

GAGCTCGCTGATCAGCCTCGACTGTGCCTTCTAGTTGCCAGCCATCTGTTGTTTGCCCCTCCCCCGTGCCTT

CCTTGACCCTGGAAGGTGCCACTCCCACTGTCCTTTCCTAATAAAATGAGGAAATTGCATCGCATTGTCTGA

GTAGGTGTCATTCTATTCTGGGGGGTGGGGTGGGGCAGGACAGCAAGGGGGAGGATTGGGAAGACAATAG

CAGGCATGCTGGGGATGCGGTGGGCTCTATGGCCACGTGGATATCGCGGCCGCAGGAACCCCTAGTGATG

GAGTTGGCCACTCCCTCTCTGCGCGCTCGCTCGCTCACTGAGGCCGGGCGACCAAAGGTCGCCCGACGCC
CGGGCTTTGCCCGGGCGGCCTCAGTGAGCGAGCGAGCGCGCAGCTGCCTGCAGG

SEQ ID NO: 23 (AAV9-TNT-moTert virus genome)

CCTGCAGGCAGCTGCGCGCTCGCTCGCTCACTGAGGCCGCCCGGGCAAAGCCCGGGCGTCGGGCGACCT
TTGGTCGCCCGGCCTCAGTGAGCGAGCGAGCGCGCAGAGAGGGAGTGGCCAACTCCATCACTAGGGGTTC
CTGCGGCCGCGATATCCTCAGTCCATTAGGAGCCAGTAGCCTGGAAGATGTCTTTACCCCCAGCATCAGTTC
AAGTGGAGCAGCACATAACTCTTGCCCTCTGCCTTCCAAGATTCTGGTGCTGAGACTTATGGAGTGTCTTGG
AGGTTGCCTTCTGCCCCCCAACCCTGCTCCCAGCTGGCCCTCCCAGGCCTGGGTTGCTGGCCTCTGCTTTA
TCAGGATTCTCAAGAGGGACAGCTGGTTTATGTTGCATGACTGTTCCCTGCATATCTGCTCTGGTTTTAAATA
GCTTATCTGAGCAGCTGGAGGACCACATGGGCTTATATGGCGTGGGGTACATGTTCCTGTAGCCTTGTCCCT
GGCACCTGCCAAAATAGCAGCCAACACCCCCCACCCCCACCGCCATCCCCCTGCCCCACCCGTCCCCTGTC
GCACATTCCTCCCTCCGCAGGGCTGGCTCACCAGGCCCCAGCCCACATGCCTGCTTAAAGCCCTCTCCATC
CTCTGCCTCACCCAGTCCCCGCTGAGACTGAGCAGACGCCTCCAGGATCTGTCGGCAGGAATTATCGCTAG
CACGCGTGCCACCATGACAAGAGCCCCTAGATGTCCTGCCGTCAGAAGCCTGCTGAGAAGCAGATACAGAG
AAGTGTGGCCCCTGGCCACCTTCGTCAGAAGGCTTGGACCTGAAGGCAGACGGCTGGTTCAACCTGGCGA
CCCCAAGATCTACAGAACCCTGGTGGCTCAGTGCCTCGTGTGTATGCACTGGGGATCTCAGCCACCTCCTG
CCGATCTGAGCTTTCACCAGGTGTCCAGCCTGAAAGAACTGGTGGCCAGAGTGGTGCAGAGACTGTGCGAG
AGAAACGAGAGGAACGTGCTGGCCTTCGGCTTCGAGCTGCTGAATGAAGCTAGAGGCGGCCCTCCTATGG
CCTTCACATCTAGCGTGCGGAGCTACCTGCCTAACACCGTGATCGAGACACTGAGAGTGTCCGGCGCTTGG
ATGCTGCTGCTGAGCAGAGTGGGAGATGACCTGCTGGTGTACCTGCTGGCTCACTGTGCCCTCTATCTGCT
GGTGCCTCCTAGCTGCGCTTACCAAGTGTGCGGAAGCCCTCTGTACCAGATCTGTGCCACCACGACATCT
GGCCTAGCGTGTCCGCTTCCTACAGACCCACAAGACCCGTGGGCAGAAACTTTACCAACCTGAGATTCCTG
CAGCAGATCAAGAGCAGCTCCAGGCAAGAGGCCCCTAAGCCTCTTGCTCTGCCTAGCAGAGGCACCAAGAG
ACACCTGAGCCTGACCAGCACAAGCGTGCCCTCTGCCAAGAAAGCCAGATGCTACCCCGTGCCTAGAGTGG
AAGAGGGCCCACATAGACAGGTGCTGCCTACACCTAGCGGCAAGAGCTGGGTTCCATCTCCTGCTAGAAGC
CCTGAGGTGCCAACAGCCGAGAAGGACCTGAGCAGCAAGGGCAAAGTGTCCGACCTGTCTCTGAGCGGCA
GCGTGTGTTGCAAGCACAAGCCTAGCAGCACCAGCCTGCTGTCCCCACCTAGACAGAACGCTTTCCAGCTG
AGGCCCTTTATCGAGACAAGACACTTCCTGTACAGCAGAGGCGACGGCCAAGAGAGACTGAACCCTAGCTT
CCTGCTGTCTAACCTGCAGCCTAACCTGACCGGCGCTAGAAGGCTGGTGGAAATCATCTTCCTGGGCAGCA
GACCCAGAACCAGCGGACCTCTGTGTAGAACCCACAGACTGAGCAGACGGTACTGGCAGATGAGGCCCCT
GTTCCAGCAGCTCCTGGTCAACCATGCCGAGTGTCAGTATGTGCGGCTGCTGAGGTCCCACTGCAGATTCA
GAACCGCCAACCAGCAAGTGACAGACGCCCTGAACACAAGCCCTCCACACCTGATGGATCTGCTGAGACTG
CACAGCAGCCCCTGGCAGGTCTACGGCTTTCTGAGAGCCTGTCTGTGCAAGGTGGTGTCCGCATCTCTGTG
GGGCACCAGACACAACGAGAGAAGATTCTTCAAGAACCTGAAGAAGTTCATCAGCCTGGGGAAGTACGGCA
AGCTGTCCCTGCAAGAACTGATGTGGAAGATGAAGGTCGAGGACTGCCACTGGCTGAGGTCTAGCCCTGGC
AAGGATAGAGTGCCTGCCGCTGAGCACAGGCTGAGAGAGAGAATCCTGGCCACATTCCTGTTCTGGCTGAT

GGACACCTACGTGGTGCAGCTGCTGCGGAGCTTCTTCTACATCACCGAGAGCACCTTCCAGAAGAACCGGC
TGTTCTTCTACCGCAAGAGCGTGTGGTCCAAGCTGCAGAGCATCGGCGTCAGACAGCACCTGGAAAGAGTG
CGCCTGAGAGAGCTGAGCCAAGAGGAAGTGCGGCACCACCAGGATACCTGGCTGGCCATGCCTATCTGCA
GACTGAGATTCATCCCCAAGCCTAACGGCCTGAGGCCTATCGTGAACATGAGCTACAGCATGGGCACTAGA
GCCCTGGGCAGAAGAAAGCAGGCCCAGCACTTCACCCAGAGGCTGAAAACCCTGTTCTCCATGCTGAACTA
CGAGCGGACAAAGCACCCTCATCTGATGGGCTCCTCTGTGCTGGGCATGAACGACATCTACAGGACCTGGC
GGGCCTTCGTGCTGAGAGTCAGAGCACTGGACCAGACACCTAGAATGTACTTCGTGAAGGCCGACGTGACA
GGCGCCTACGATGCTATCCCTCAGGGCAAACTGGTGGAAGTGGTGGCCAACATGATCAGGCACAGCGAGT
CCACCTACTGCATCAGACAGTACGCCGTCGTGCGGAGAGACTCTCAGGGACAAGTGCACAAGAGCTTCCGC
AGACAAGTGACCACACTGAGCGATCTGCAGCCCTACATGGGCCAGTTTCTGAAGCACCTCCAGGACAGCGA
CGCCAGCGCTCTGAGAAACTCTGTGGTCATCGAGCAGTCCATCTCCATGAACGAGTCCAGCTCCAGCCTGT
TCGACTTCTTCCTGCACTTCCTGCGGCACAGCGTGGTCAAGATCGGCGACAGATGTTACACCCAGTGCCAG
GGCATCCCACAGGGAAGCAGTCTGAGCACACTGCTGTGCTCCCTGTGTTTCGGCGACATGGAAAACAAGCT
GTTCGCCGAAGTGCAGCGCGACGGACTGCTCCTGAGATTTGTGGACGACTTCCTGCTCGTGACCCCTCACC
TGGATCAGGCTAAGACCTTCCTGAGCACCCTGGTGCATGGCGTGCCAGAGTACGGCTGCATGATCAACCTG
CAGAAAACCGTGGTCAACTTCCCCGTGGAACCTGGCACTCTTGGAGGCGCTGCTCCTTATCAGCTGCCCGC
TCATTGTCTGTTCCCTTGGTGCGGACTGCTGCTGGACACACAGACCCTGGAAGTGTTCTGCGACTACAGCG
GCTACGCCCAGACCAGCATCAAGACCAGCCTGACCTTCCAGTCCGTGTTCAAGGCCGGCAAGACCATGAGG
AACAAGCTGCTGAGTGTGCTGAGGCTGAAGTGCCACGGCCTGTTTCTGGACCTGCAAGTGAACAGCCTGCA
GACCGTGTGCATCAATATCTACAAGATCTTTCTGCTGCAAGCCTACCGGTTCCACGCCTGTGTGATTCAGCT
GCCTTTCGACCAGAGAGTGCGGAAGAATCTGACATTCTTTCTGGGCATCATCAGCAGCCAGGCCAGCTGCT
GTTACGCCATCCTGAAAGTGAAGAACCCCGGCATGACCCTGAAGGCCTCTGGCAGCTTTCCACCTGAAGCT
GCCCATTGGCTGTGCTACCAGGCTTTCCTGCTGAAACTGGCTGCCCACAGCGTGATCTACAAGTGCCTGCT
GGGCCCACTGAGAACAGCCCAGAAACTGCTGTGTCGGAAGCTGCCTGAAGCCACAATGACAATCCTGAAGG
CTGCCGCCGATCCAGCTCTGAGCACCGACTTCCAGACCATCCTGGACTGAGAATTCGGATCCGATTCGAGC
ATTTAAATACGTGGAGCTCGCTGATCAGCCTCGACTGTGCCTTCTAGTTGCCAGCCATCTGTTGTTTGCCCCT
CCCCCGTGCCTTCCTTGACCCTGGAAGGTGCCACTCCCACTGTCCTTTCCTAATAAAATGAGGAAATTGCAT
CGCATTGTCTGAGTAGGTGTCATTCTATTCTGGGGGGTGGGGTGGGGCAGGACAGCAAGGGGGAGGATTG
GGAAGACAATAGCAGGCATGCTGGGGATGCGGTGGGCTCTATGGCCACGTGGATATCGCGGCCGCAGGAA
CCCCTAGTGATGGAGTTGGCCACTCCCTCTCTGCGCGCTCGCTCGCTCACTGAGGCCGGGCGACCAAAGG
TCGCCCGACGCCCGGGCTTTGCCCGGGCGGCCTCAGTGAGCGAGCGAGCGCGCAGCTGCCTGCAGG

SEQ ID NO: 24 (AAV9-CMV-null genome and AAV6-CMV-null virus genome)

GCGCGCTCGCTCGCTCACTGAGGCCGCCCGGGCAAAGCCCGGGCGTCGGGCGACCTTTGGTCGCCCGGCCTC
AGTGAGCGAGCGAGCGCGCAGAGAGGGAGTGGCCAACTCCATCACTAGGGGTTCCTGCGGCCGCACGCGTG

GAGCTAGTTATTAATAGTAATCAATTACGGGGTCATTAGTTCATAGCCCATATATGGAGTTCCGCGTTACATAA
CTTACGGTAAATGGCCCGCCTGGCTGACCGCCCAACGACCCCCGCCCATTGACGTCAATAATGACGTATGTTCC
CATAGTAACGTCAATAGGGACTTTCCATTGACGTCAATGGGTGGAGTATTTACGGTAAACTGCCCACTTGGCA
GTACATCAAGTGTATCATATGCCAAGTACGCCCCCTATTGACGTCAATGACGGTAAATGGCCCGCCTGGCATTA
TGCCCAGTACATGACCTTATGGGACTTTCCTACTTGGCAGTACATCTACGTATTAGTCATCGCTATTACCATGGT
GATGCGGTTTTGGCAGTACATCAATGGGCGTGGATAGCGGTTTGACTCACGGGGATTTCCAAGTCTCCACCCC
ATTGACGTCAATGGGAGTTTGTTTTGCACCAAAATCAACGGGACTTTCCAAAATGTCGTAACAACTCCGCCCCA
TTGACGCAAATGGGCGGTAGGCGTGTACGGTGGGAGGTCTATATAAGCAGAGCTCGTTTAGTGAACCGTCAG
ATCGCCTGGAGACGCCATCCACGCTGTTTTGACCTCCATAGAAGACACCGGGACCGATCCAGCCTCCGCGGAT
TCGAATCCCGGCCGGGAACGGTGCATTGGAACGCGGATTCCCCGTGCCAAGAGTGACGTAAGTACCGCCTAT
AGAGTCTATAGGCCCACAAAAAATGCTTTCTTCTTTTAATATACTTTTTTGTTTATCTTATTTCTAATACTTTCCCT
AATCTCTTTCTTTCAGGGCAATAATGATACAATGTATCATGCCTCTTTGCACCATTCTAAAGAATAACAGTGATA
ATTTCTGGGTTAAGGCAATAGCAATATTTCTGCATATAAATATTTCTGCATATAAATTGTAACTGATGTAAGAG
GTTTCATATTGCTAATAGCAGCTACAATCCAGCTACCATTCTGCTTTTATTTTATGGTTGGGATAAGGCTGGATT
ATTCTGAGTCCAAGCTAGGCCCTTTTGCTAATCATGTTCATACCTCTTATCTTCCTCCCACAGCTCCTGGGCAAC
GTGCTGGTCTGTGTGCTGGCCCATCACTTTGGCAAAGAATTGGGATTCGAACATCGATTGAATTCCCCGGGGA
TCCTCTAGAGTCGACCTGCAGAAGCTTGCCTCGAGCAGCGCTGCTCGAGAGATCTACGGGTGGCATCCCTGTG
ACCCCTCCCCAGTGCCTCTCCTGGCCCTGGAAGTTGCCACTCCAGTGCCCACCAGCCTTGTCCTAATAAAATTAA
GTTGCATCATTTTGTCTGACTAGGTGTCCTTCTATAATATTATGGGGTGGAGGGGGGTGGTATGGAGCAAGGG
GCAAGTTGGGAAGACAACCTGTAGGGCCTGCGGGGTCTATTGGGAACCAAGCTGGAGTGCAGTGGCACAATC
TTGGCTCACTGCAATCTCCGCCTCCTGGGTTCAAGCGATTCTCCTGCCTCAGCCTCCCGAGTTGTTGGGATTCCA
GGCATGCATGACCAGGCTCAGCTAATTTTTGTTTTTTTGGTAGAGACGGGGTTTCACCATATTGGCCAGGCTGG
TCTCCAACTCCTAATCTCAGGTGATCTACCCACCTTGGCCTCCCAAATTGCTGGGATTACAGGCGTGAACCACT
GCTCCCTTCCCTGTCCTTCTGATTTTGTAGGTAACCACGTGCGGACCGAGCGGCCGCAGGAACCCCTAGTGATG
GAGTTGGCCACTCCCTCTCTGCGCGCTCGCTCGCTCACTGAGGCCGGGCGACCAAAGGTCGCCCGACGCCCGG
GCTTTGCCCGGGCGGCCTCAGTGAGCGAGCGAGCGCGC

SEQ ID NO: 25 (AAV9-CMV-mTERT virus genome)

CCTGCAGGCAGCTGCGCGCTCGCTCGCTCACTGAGGCCGCCCGGGCAAAGCCCGGGCGTCGGGCGACCTTTG
GTCGCCCGGCCTCAGTGAGCGAGCGAGCGCGCAGAGAGGGAGTGGCCAACTCCATCACTAGGGGTTCCTGCG
GCCGCACGCGTGGAGCTAGTTATTAATAGTAATCAATTACGGGGTCATTAGTTCATAGCCCATATATGGAGTTC
CGCGTTACATAACTTACGGTAAATGGCCCGCCTGGCTGACCGCCCAACGACCCCCGCCCATTGACGTCAATAAT
GACGTATGTTCCCATAGTAACGTCAATAGGGACTTTCCATTGACGTCAATGGGTGGAGTATTTACGGTAAACTG

CCCACTTGGCAGTACATCAAGTGTATCATATGCCAAGTACGCCCCCTATTGACGTCAATGACGGTAAATGGCCC
GCCTGGCATTATGCCCAGTACATGACCTTATGGGACTTTCCTACTTGGCAGTACATCTACGTATTAGTCATCGCT
ATTACCATGGTGATGCGGTTTTGGCAGTACATCAATGGGCGTGGATAGCGGTTTGACTCACGGGGATTTCCAA
GTCTCCACCCCATTGACGTCAATGGGAGTTTGTTTTGCACCAAAATCAACGGGACTTTCCAAAATGTCGTAACA
ACTCCGCCCCATTGACGCAAATGGGCGGTAGGCGTGTACGGTGGGAGGTCTATATAAGCAGAGCTCGTTTAGT
GAACCGTCAGATCGCCTGGAGACGCCATCCACGCTGTTTTGACCTCCATAGAAGACACCGGGACCGATCCAGC
CTCCGCGGATTCGAATCCCGGCCGGGAACGGTGCATTGGAACGCGGATTCCCCGTGCCAAGAGTGACGTAAG
TACCGCCTATAGAGTCTATAGGCCCACAAAAAATGCTTTCTTCTTTTAATATACTTTTTTGTTTATCTTATTTCTAA
TACTTTCCCTAATCTCTTTCTTTCAGGGCAATAATGATACAATGTATCATGCCTCTTTGCACCATTCTAAAGAATA
ACAGTGATAATTTCTGGGTTAAGGCAATAGCAATATTTCTGCATATAAATATTTCTGCATATAAATTGTAACTGA
TGTAAGAGGTTTCATATTGCTAATAGCAGCTACAATCCAGCTACCATTCTGCTTTTATTTTATGGTTGGGATAAG
GCTGGATTATTCTGAGTCCAAGCTAGGCCCTTTTGCTAATCATGTTCATACCTCTTATCTTCCTCCCACAGCTCCT
GGGCAACGTGCTGGTCTGTGTGCTGGCCCATCACTTTGGCAAAGAATTGGGATTCGAACATCGATTGAATTCA
TGACCCGCGCTCCTCGTTGCCCCGCGGTGCGCTCTCTGCTGCGCAGCCGATACCGGGAGGTGTGGCCGCTGGC
AACCTTTGTGCGGCGCCTGGGGCCCGAGGGCAGGCGGCTTGTGCAACCCGGGGACCCGAAGATCTACCGCAC
TTTGGTTGCCCAATGCCTAGTGTGCATGCACTGGGGCTCACAGCCTCCACCTGCCGACCTTTCCTTCCACCAGG
TGTCATCCCTGAAAGAGCTGGTGGCCAGGGTTGTGCAGAGACTCTGCGAGCGCAACGAGAGAAACGTGCTGG
CTTTTGGCTTTGAGCTGCTTAACGAGGCCAGAGGCGGGCCTCCCATGGCCTTCACTAGTAGCGTGCGTAGCTA
CTTGCCCAACACTGTTATTGAGACCCTGCGTGTCAGTGGTGCATGGATGCTACTGTTGAGCCGAGTGGGCGAC
GACCTGCTGGTCTACCTGCTGGCACACTGTGCTCTTTATCTTCTGGTGCCCCCCAGCTGTGCCTACCAGGTGTGT
GGGTCTCCCCTGTACCAAATTTGTGCCACCACGGATATCTGGCCCTCTGTGTCCGCTAGTTACAGGCCCACCCG
ACCCGTGGGCAGGAATTTCACTAACCTTAGGTTCTTACAACAGATCAAGAGCAGTAGTCGCCAGGAAGCACCG
AAACCCCTGGCCTTGCCATCTCGAGGTACAAAGAGGCATCTGAGTCTCACCAGTACAAGTGTGCCTTCAGCTAA
GAAGGCCAGATGCTATCCTGTCCCGAGAGTGGAGGAGGGGACCCCACAGGCAGGTGCTACCAACCCCATCAGG
CAAATCATGGGTGCCAAGTCCTGCTCGGTCCCCCGAGGTGCCTACTGCAGAGAAAGATTTGTCTTCTAAAGGA
AAGGTGTCTGACCTGAGTCTCTCTGGGTCGGTGTGCTGTAAACACAAGCCCAGCTCCACATCTCTGCTGTCACC
ACCCCGCCAAAATGCCTTTCAGCTCAGGCCATTTATTGAGACCAGACATTTCCTTTACTCCAGGGGAGATGGCC
AAGAGCGTCTAAACCCCTCATTCCTACTCAGCAACCTCCAGCCTAACTTGACTGGGGCCAGGAGACTGGTGGA
GATCATCTTTCTGGGCTCAAGGCCTAGGACATCAGGACCACTCTGCAGGACACACCGTCTATCGCGTCGATACT
GGCAGATGCGGCCCCTGTTCCAACAGCTGCTGGTGAACCATGCAGAGTGCCAATATGTCAGACTCCTCAGGTC
ACATTGCAGGTTTCGAACAGCAAACCAACAGGTGACAGATGCCTTGAACACCAGCCCACCGCACCTCATGGAT
TTGCTCCGCCTGCACAGCAGTCCCTGGCAGGTATATGGTTTTCTTCGGGCCTGTCTCTGCAAGGTGGTGTCTGC
TAGTCTCTGGGGTACCAGGCACAATGAGCGCCGCTTCTTTAAGAACTTAAAGAAGTTCATCTCGTTGGGGAAA
TACGGCAAGCTATCACTGCAGGAACTGATGTGGAAGATGAAAGTAGAGGATTGCCACTGGCTCCGCAGCAGC
CCGGGGAAGGACCGTGTCCCCGCTGCAGAGCACCGTCTGAGGAGAGGATCCTGGCTACGTTCCTGTTCTGGCT

GATGGACACATACGTGGTACAGCTGCTTAGGTCATTCTTTTACATCACAGAGAGCACATTCCAGAAGAACAGG

CTCTTCTTCTACCGTAAGAGTGTGTGGAGCAAGCTGCAGAGCATTGGAGTCAGGCAACACCTTGAGAGAGTGC

GGCTACGGGAGCTGTCACAAGAGGAGGTCAGGCATCACCAGGACACCTGGCTAGCCATGCCCATCTGCAGAC

TGCGCTTCATCCCCAAGCCCAACGGCCTGCGGCCCATTGTGAACATGAGTTATAGCATGGGTACCAGAGCTTT

GGGCAGAAGGAAGCAGGCCCAGCATTTCACCCAGCGTCTCAAGACTCTCTTCAGCATGCTCAACTATGAGCGG

ACAAAACATCCTCACCTTATGGGGTCTTCTGTACTGGGTATGAATGACATCTACAGGACCTGGCGGGCCTTTGT

GCTGCGTGTGCGTGCTCTGGACCAGACACCCAGGATGTACTTTGTTAAGGCAGATGTGACCGGGGCCTATGAT

GCCATCCCCCCAGGGTAAGCTGGTGGAGGTTGTTGCCAATATGATCAGGCACTCGGAGAGCACGTACTGTATC

CGCCAGTATGCAGTGGTCCGGAGAGATAGCCAAGGCCAAGTCCACAAGTCCTTTAGGAGACAGGTCACCACC

CTCTCTGACCTCCAGCCATACATGGGCCAGTTCCTTAAGCATCTGCAGGATTCAGATGCCAGTGCACTGAGGAA

CTCCGTTGTCATCGAGCAGAGCATCTCTATGAATGAGAGCAGCAGCAGCCTGTTTGACTTCTTCCTGCACTTCCT

GCGTCACAGTGTCGTAAAGATTGGTGACAGGTGCTATACGCAGTGCCAGGGCATCCCCCAGGGCTCCAGCCTA

TCCACCCTGCTCTGCAGTCTGTGTTTCGGAGACATGGAGAACAAGCTGTTTGCTGAGGTGCAGCGGGATGGGT

TGCTTTTACGTTTTGTTGATGACTTTCTGTTGGTGACGCCTCACTTGGACCAAGCAAAAACCTTCCTCAGCACCC

TGGTCCATGGCGTTCCTGAGTATGGGTGCATGATAAACTTGCAGAAGACAGTGGTGAACTTCCCTGTGGAGCC

TGGTACCCTGGGTGGTGCAGCTCCATACCAGCTGCCTGCTCACTGCCTGTTTCCCTGGTGTGGCTTGCTGCTGG

ACACTCAGACTTTGGAGGTGTTCTGTGACTACTCAGGTTATGCCCAGACCTCAATTAAGACGAGCCTCACCTTC

CAGAGTGTCTTCAAAGCTGGGAAGACCATGCGGAACAAGCTCCTGTCGGTCTTGCGGTTGAAGTGTCACGGTC

TATTTCTAGACTTGCAGGTGAACAGCCTCCAGACAGTCTGCATCAATATATACAAGATCTTCCTGCTTCAGGCCT

ACAGGTTCCATGCATGTGTGATTCAGCTTCCCTTTGACCAGCGTGTTAGGAAGAACCTCACATTCTTTCTGGGC

ATCATCTCCAGCCAAGCATCCTGCTGCTATGCTATCCTGAAGGTCAAGAATCCAGGAATGACACTAAAGGCCTC

TGGCTCCTTTCCTCCTGAAGCCGCACATTGGCTCTGCTACCAGGCCTTCCTGCTCAAGCTGGCTGCTCATTCTGT

CATCTACAAATGTCTCCTGGGACCTCTGAGGACAGCCCAAAAACTGCTGTGCCGGAAGCTCCCAGAGGCGACA

ATGACCATCCTTAAAGCTGCAGCTGACCCAGCCCTAAGCACAGACTTTCAGACCATTTTGGACTAACCCTGTCT

CCTTCCGCTAGATGAACATGGGCATTGTAGCCTCAGCACTCCTGGATCCACGTCACAAGAGGGACTGGTCAGT

TGTGAGGCTAGGTCATCCTCCAAACCTCTGTGTCATGGGTGGTATGGGAGATTGTCCCAGTGCCTTGTTTCCTG

TAACAGGCTTGATTTCTTTCCTGATGCCCTCAGGGAGGCAGATCCTATCCCTTTTAGTGGCAGGGATCCACTAG

CACCAGCACATGAGGAGTGCACCCAGTGCACATGGGCACTGGGACAGTGGACAGGTGTGAGATTCCTGGGCC

CTGGAGTCTTTTCACACCTAACCATGGGAGCCTGTCCCAGTACATCAGAGTGCCTCGGAGATGAAAAAGGACA

TCGAGCCAGTGACCTAAATTACAGCCTGAATATACTCTGAATTCCCCGGGGATCCTCTAGAGTCGACCTGCAGA

AGCTGTAACCACGTGCGGACCGAGCGGCCGCAGGAACCCCTAGTGATGGAGTTGGCCACTCCCTCTCTGCGCG

CTCGCTCGCTCACTGAGGCCGGGCGACCAAAGGTCGCCCGACGCCCGGGCTTTGCCCGGGCGGCCTCAGTGA

GCGAGCGAGCGCGCAGCTGCCTGCAGG

SEQ ID NO: 26 (AAV-CMV-GFP recombinant adeno-associated virus genome)

CCTGCAGGCAGCTGCGCGCTCGCTCGCTCACTGAGGCCGCCCGGGCAAAGCCCGGGCGTCGGGCGACCTTTG

GTCGCCCGGCCTCAGTGAGCGAGCGAGCGCGCAGAGAGGGAGTGGCCAACTCCATCACTAGGGGTTCCTGCG

GCCGCACGCGTGGAGCTAGTTATTAATAGTAATCAATTACGGGGTCATTAGTTCATAGCCCATATATGGAGTTC

CGCGTTACATAACTTACGGTAAATGGCCCGCCTGGCTGACCGCCCAACGACCCCCGCCCATTGACGTCAATAAT

GACGTATGTTCCCATAGTAACGTCAATAGGGACTTTCCATTGACGTCAATGGGTGGAGTATTTACGGTAAACTG

CCCACTTGGCAGTACATCAAGTGTATCATATGCCAAGTACGCCCCCTATTGACGTCAATGACGGTAAATGGCCC

GCCTGGCATTATGCCCAGTACATGACCTTATGGGACTTTCCTACTTGGCAGTACATCTACGTATTAGTCATCGCT

ATTACCATGGTGATGCGGTTTTGGCAGTACATCAATGGGCGTGGATAGCGGTTTGACTCACGGGGATTTCCAA

GTCTCCACCCCATTGACGTCAATGGGAGTTTGTTTTGCACCAAAATCAACGGGACTTTCCAAAATGTCGTAACA

ACTCCGCCCCATTGACGCAAATGGGCGGTAGGCGTGTACGGTGGGAGGTCTATATAAGCAGAGCTCGTTTAGT

GAACCGTCAGATCGCCTGGAGACGCCATCCACGCTGTTTTGACCTCCATAGAAGACACCGGGACCGATCCAGC

CTCCGCGGATTCGAATCCCGGCCGGGAACGGTGCATTGGAACGCGGATTCCCCGTGCCAAGAGTGACGTAAG

TACCGCCTATAGAGTCTATAGGCCCACAAAAAATGCTTTCTTCTTTTAATATACTTTTTTGTTTATCTTATTTCTAA

TACTTTCCCTAATCTCTTTCTTTCAGGGCAATAATGATACAATGTATCATGCCTCTTTGCACCATTCTAAAGAATA

ACAGTGATAATTTCTGGGTTAAGGCAATAGCAATATTTCTGCATATAAATATTTCTGCATATAAATTGTAACTGA

TGTAAGAGGTTTCATATTGCTAATAGCAGCTACAATCCAGCTACCATTCTGCTTTTATTTTATGGTTGGGATAAG

GCTGGATTATTCTGAGTCCAAGCTAGGCCCTTTTGCTAATCATGTTCATACCTCTTATCTTCCTCCCACAGCTCCT

GGGCAACGTGCTGGTCTGTGTGCTGGCCCATCACTTTGGCAAAGAATTGGGATTCGAACATTCGAGCGCAAGC

TTGAGCTAGCGCTACCGGTCGCCACCATGGTGAGCAAGGGCGAGGAGCTGTTCACCGGGGTGGTGCCCATCC

TGGTCGAGCTGGACGGCGACGTAAACGGCCACAAGTTCAGCGTGTCCGGCGAGGGCGAGGGCGATGCCACC

TACGGCAAGCTGACCCTGAAGTTCATCTGCACCACCGGCAAGCTGCCCGTGCCCTGGCCCACCCTCGTGACCAC

CCTGACCTACGGCGTGCAGTGCTTCAGCCGCTACCCCGACCACATGAAGCAGCACGACTTCTTCAAGTCCGCCA

TGCCCGAAGGCTACGTCCAGGAGCGCACCATCTTCTTCAAGGACGACGGCAACTACAAGACCCGCGCCGAGG

TGAAGTTCGAGGGCGACACCCTGGTGAACCGCATCGAGCTGAAGGGCATCGACTTCAAGGAGGACGGCAACA

TCCTGGGGCACAAGCTGGAGTACAACTACAACAGCCACAACGTCTATATCATGGCCGACAAGCAGAAGAACG

GCATCAAGGTGAACTTCAAGATCCGCCACAACATCGAGGACGGCAGCGTGCAGCTCGCCGACCACTACCAGCA

GAACACCCCCATCGGCGACGGCCCCGTGCTGCTGCCCGACAACCACTACCTGAGCACCCAGTCCGCCCTGAGC

AAAGACCCCAACGAGAAGCGCGATCACATGGTCCTGCTGGAGTTCGTGACCGCCGCCGGGATCACTCTCGGC

ATGGACGAGCTGTACAAGTCCGGACTCAGATCTCGAGCTCAAGCTTCGAATTCTGCAGTCGACGGTACCGCGG

GCCCGGGATCCACCGGATCTAGATAACTGATCCGCGCGATCTACGGGTGGCATCCCTGTGACCCCTCCCCAGT

GCCTCTCCTGGCCCTGGAAGTTGCCACTCCAGTGCCCACCAGCCTTGTCCTAATAAAATTAAGTTGCATCATTTT

GTCTGACTAGGTGTCCTTCTATAATATTATGGGGTGGAGGGGGGTGGTATGGAGCAAGGGGCAAGTTGGGAA

GACAACCTGTAGGGCCTGCGGGGTCTATTGGGAACCAAGCTGGAGTGCAGTGGCACAATCTTGGCTCACTGC

AATCTCCGCCTCCTGGGTTCAAGCGATTCTCCTGCCTCAGCCTCCCGAGTTGTTGGGATTCCAGGCATGCATGA

CCAGGCTCAGCTAATTTTTGTTTTTTTGGTAGAGACGGGGTTTCACCATATTGGCCAGGCTGGTCTCCAACTCCT

AATCTCAGGTGATCTACCCACCTTGGCCTCCCAAATTGCTGGGATTACAGGCGTGAACCACTGCTCCCTTCCCT

GTCCTTCTGATTTTGTAGGTAACCACGTGCGGACCGAGCGGCCGCAGGAACCCCTAGTGATGGAGTTGGCCAC

TCCCTCTCTGCGCGCTCGCTCGCTCACTGAGGCCGGGCGACCAAAGGTCGCCCGACGCCCGGGCTTTGCCCGG

GCGGCCTCAGTGAGCGAGCGAGCGCGCAGCTGCCTGCAGG

SEQ ID NO: 27 (AAV-SpB-GFP recombinant adeno-associated virus genome)

CCTGCAGGCAGCTGCGCGCTCGCTCGCTCACTGAGGCCGCCCGGGCAAAGCCCGGGCGTCGGGCGACCTTTG

GTCGCCCGGCCTCAGTGAGCGAGCGAGCGCGCAGAGAGGGAGTGGCCAACTCCATCACTAGGGGTTCCTGCG

GCCGCGATATCATAGGGCTGTCTGGGAGCCACTCCAGGGCCACAGAAATCTTGTCTCTGACTCAGGGTATTTT

GTTTTCTGTTTTGTGTAAATGCTCTTCTGACTAATGCAAACCATGTGTCCATAGAACCAGAAGATTTTTCCAGGG

GAAAAGGTAAGGAGGTGGTGAGAGTGTCCTGGGTCTGCCCTTCCAGGGCTTGCCCTGGGTTAAGAGCCAGGC

AGGAAGCTCTCAAGAGCATTGCTCAAGAGTAGAGGGGGCCTGGGAGGCCCAGGGAGGGGATGGGAGGGGA

ACACCCAGGCTGCCCCCAACCAGATGCCCTCCACCCTCCTCAACCTCCCTCCCACGGCCTGGAGAGGTGGGACC

AGGTATGGAGGCTTGAGAGCCCCTGGTTGGAGGAAGCCACAAGTCCAGGAACATGGGAGTCTGGGCAGGGG

GCAAAGGAGGCAGGAACAGGCCATCAGCCAGGACAGGTGGTAAGGCAGGCAGGAGTGTTCCTGCTGGGAAA

AGGTGGGATCAAGCACCTGGAGGGCTCTTCAGAGCAAAGACAAACACTGAGGTCGCTGCCACTCCTACAGAG

CCCCCACGCCCCGCCCAGCTATAAGGGGCCATGCACCAAGCAGGGTACCCAGGCTGCAGAGGTGCGAATTCA

TTTAAATTCTAGAGCCACCATGGTGAGCAAGGGCGAGGAGCTGTTCACCGGGGTGGTGCCCATCCTGGTCGA

GCTGGACGGCGACGTAAACGGCCACAAGTTCAGCGTGTCCGGCGAGGGCGAGGGCGATGCCACCTACGGCA

AGCTGACCCTGAAGTTCATCTGCACCACCGGCAAGCTGCCCGTGCCCTGGCCCACCCTCGTGACCACCCTGACC

TACGGCGTGCAGTGCTTCAGCCGCTACCCCGACCACATGAAGCAGCACGACTTCTTCAAGTCCGCCATGCCCG

AAGGCTACGTCCAGGAGCGCACCATCTTCTTCAAGGACGACGGCAACTACAAGACCCGCGCCGAGGTGAAGT

TCGAGGGCGACACCCTGGTGAACCGCATCGAGCTGAAGGGCATCGACTTCAAGGAGGACGGCAACATCCTGG

GGCACAAGCTGGAGTACAACTACAACAGCCACAACGTCTATATCATGGCCGACAAGCAGAAGAACGGCATCA

AGGTGAACTTCAAGATCCGCCACAACATCGAGGACGGCAGCGTGCAGCTCGCCGACCACTACCAGCAGAACA

CCCCCATCGGCGACGGCCCCGTGCTGCTGCCCGACAACCACTACCTGAGCACCCAGTCCGCCCTGAGCAAAGA

CCCCAACGAGAAGCGCGATCACATGGTCCTGCTGGAGTTCGTGACCGCCGCCGGGATCACTCTCGGCATGGAC

GAGCTGTACAAGTAAACTCGAGCATTTAAATACGTGGAGCTCGCTGATCAGCCTCGACTGTGCCTTCTAGTTGC

CAGCCATCTGTTGTTTGCCCCTCCCCCGTGCCTTCCTTGACCCTGGAAGGTGCCACTCCCACTGTCCTTTCCTAAT

AAAATGAGGAAATTGCATCGCATTGTCTGAGTAGGTGTCATTCTATTCTGGGGGGTGGGGTGGGGCAGGACA

GCAAGGGGGAGGATTGGGAAGACAATAGCAGGCATGCTGGGGATGCGGTGGGCTCTATGGCCACGTGGATA

TCGCGGCCGCAGGAACCCCTAGTGATGGAGTTGGCCACTCCCTCTCTGCGCGCTCGCTCGCTCACTGAGGCCG

GGCGACCAAAGGTCGCCCGACGCCCGGGCTTTGCCCGGGCGGCCTCAGTGAGCGAGCGAGCGCGCAGCTGCCTGCAGG

SEQ ID NO: 28 (AAV6-SpB-null genome and AAV9-SpB-null virus genome)

CCTGCAGGCAGCTGCGCGCTCGCTCGCTCACTGAGGCCGCCCGGGCAAAGCCCGGGCGTCGGGCGACCTTTGGTCGCCCGGCCTCAGTGAGCGAGCGAGCGCGCAGAGAGGGAGTGGCCAACTCCATCACTAGGGGTTCCTGCGGCCGCGATATCATAGGGCTGTCTGGGAGCCACTCCAGGGCCACAGAAATCTTGTCTCTGACTCAGGGTATTTTGTTTTCTGTTTTGTGTAAATGCTCTTCTGACTAATGCAAACCATGTGTCCATAGAACCAGAAGATTTTTCCAGGGGAAAAGGTAAGGAGGTGGTGAGAGTGTCCTGGGTCTGCCCTTCCAGGGCTTGCCCTGGGTTAAGAGCCAGGCAGGAAGCTCTCAAGAGCATTGCTCAAGAGTAGAGGGGGCCTGGGAGGCCCAGGGAGGGGATGGGAGGGGAACACCCAGGCTGCCCCCAACCAGATGCCCTCCACCCTCCTCAACCTCCCTCCCACGGCCTGGAGAGGTGGGACCAGGTATGGAGGCTTGAGAGCCCCTGGTTGGAGGAAGCCACAAGTCCAGGAACATGGGAGTCTGGGCAGGGGGCAAAGGAGGCAGGAACAGGCCATCAGCCAGGACAGGTGGTAAGGCAGGCAGGAGTGTTCCTGCTGGGAAAAGGTGGGATCAAGCACCTGGAGGGCTCTTCAGAGCAAAGACAAACACTGAGGTCGCTGCCACTCCTACAGAGCCCCCACGCCCCGCCCAGCTATAAGGGGCCATGCACCAAGCAGGGTACCCAGGCTGCAGAGGTGCGAATTCATTTAAATACGTGGAGCTCGCTGATCAGCCTCGACTGTGCCTTCTAGTTGCCAGCCATCTGTTGTTTGCCCCTCCCCCGTGCCTTCCTTGACCCTGGAAGGTGCCACTCCCACTGTCCTTTCCTAATAAAATGAGGAAATTGCATCGCATTGTCTGAGTAGGTGTCATTCTATTCTGGGGGGTGGGGTGGGGCAGGACAGCAAGGGGGAGGATTGGGAAGACAATAGCAGGCATGCTGGGGATGCGGTGGGCTCTATGGCCACGTGGATATCGCGGCCGCAGGAACCCCTAGTGATGGAGTTGGCCACTCCCTCTCTGCGCGCTCGCTCGCTCACTGAGGCCGGGCGACCAAAGGTCGCCCGACGCCCGGGCTTTGCCCGGGCGGCCTCAGTGAGCGAGCGAGCGCGCAGCTGCCTGCAGG

**Claims**

1. A recombinant *adeno-associated* viral genome comprising a nucleotide sequence encoding a telomerase reverse transcriptase (TERT) operably linked to a tissue-specific promoter, wherein the total length of the viral genome is less than 4700 nucleotides, and wherein the tissue-specific promoter consists of SEQ ID NOs: 12.

2. A recombinant *adeno-associated* viral genome according to claim 1, wherein the TERT encoding sequence is codon-optimized, preferably for expression in a human cell.

3. A recombinant *adeno-associated* viral genome according to any one of claims 1 or 2, wherein:

    a) the TERT encoding sequence comprises, essentially consists of, or consists of a nucleotide sequence encoding a polypeptide represented by an amino acid sequence having at least 60%, 70%, 80%, 90%, 95%, or 99% identity or similarity with any one of SEQ ID NOs: 4, 7, or 10, preferably with SEQ ID NO: 7 or 10, or;
    b) the TERT encoding sequence comprises, essentially consists of, or consists of a nucleotide sequence having at least 60%, 70%, 80%, 90%, 95%, or 99% identity with any one of SEQ ID NOs: 1, 2, 3, 5, 6, 8, and 9, preferably with any one of SEQ ID NOs: 6 and 9 or;
    c) the TERT encoding sequence comprises, essentially consists of, or consists of a nucleotide sequence the sequence of which differs from the sequence of a nucleotide sequence of a) or b) due to the degeneracy of the genetic code.

4. A recombinant *adeno-associated* viral genome according to any one of claims 1-3, wherein the recombinant viral genome further comprises a kozak consensus sequence operably linked to the nucleotide sequence encoding a TERT.

5. A recombinant *adeno-associated* viral genome according to any one of claims 1-4, wherein the recombinant viral genome further comprises a polyA sequence operably linked to the nucleotide sequence encoding a TERT.

6. An adeno-associated viral vector comprising a recombinant adeno-associated virus genome as defined in any of claims 1 to 5.

7. An adeno-associated viral vector according to claim 6, wherein the viral vector is of serotype 1, 2, 3, 4, 5, 6, 7, 8, 9, rh10, rh8, Cb4, rh74, DJ, 2/5, 2/1, 1/2 or Anc80.

8. An adeno-associated viral vector according to claim 7, wherein the viral vector is of serotype 6 or 9.

9. An adeno-associated viral vector according to claim 8, wherein the viral vector is of serotype 6.

10. An adeno-associated viral vector according to claim 9, wherein the viral vector is of serotype 6 and wherein the tissue-specific promoter is as defined in claim 1.

11. A pharmaceutical composition comprising a recombinant *adeno-associated* viral genome according to any one of claims 1-5 or an adeno-associated viral vector according to claims 6 to 10, optionally further comprising one or more pharmaceutically acceptable ingredients.

12. A recombinant *adeno-associated* viral genome according to any one of claims 1-5, an adeno-associated viral vector according to any one of claims 6-10, or a pharmaceutical composition according to claim 11, for use as a medicament.

13. A recombinant *adeno-associated* viral genome according to any one of claims 1-5, an adeno-associated viral vector according to claims 6-10, or a pharmaceutical composition according to claim 11, for use in the treatment and/or prevention of a condition associated with shortened telomere length.

14. A recombinant *adeno-associated* viral genome according to any one of claims 1-5, an adeno-associated viral vector according to claims 6-10, or a pharmaceutical composition according to claim 11, for use in the treatment and/or prevention of pulmonary fibrosis.

15. The recombinant *adeno-associated* viral genome according to any one of claims 1-5, an adeno-associated viral vector according to claims 6-10, or a pharmaceutical composition according to claim 11, for use in the treatment and/or prevention of pulmonary fibrosis via intratracheal administration.

**Patentansprüche**

1. Rekombinantes adeno-assoziiertes virales Genom umfassend eine Nukleotidsequenz, welche eine Telomerase Reverse Transkriptase (TERT) codiert, welche an einen gewebespezifischen Promotor operativ verknüpft ist, wobei die Gesamtlänge des viralen Genoms kleiner als 4700 Nukleotide ist, und wobei der gewebespezifische Promotor aus SEQ ID NO: 12 besteht.

2. Rekombinantes adeno-assoziiertes virales Genom nach Anspruch 1, wobei die TERT-codierende Sequenz Codon-optimiert ist, vorzugsweise für die Expression in einer menschlichen Zelle.

3. Rekombinantes adeno-assoziiertes virales Genom nach einem der Ansprüche 1 oder 2, wobei:

a) die TERT-codierende Sequenz eine Nukleotidsequenz umfasst, im Wesentlichen daraus besteht oder daraus besteht, welche ein Polypeptid codiert, dargestellt durch eine Aminosäuresequenz, welche mindestens 60 %, 70 %, 80 %, 90 %, 95 % oder 99 % Identität oder Ähnlichkeit mit einer der SEQ ID NOs: 4, 7 oder 10, vorzugsweise mit SEQ ID NO: 7 oder 10, aufweist, oder;
b) die TERT-codierende Sequenz eine Nukleotidsequenz umfasst, im Wesentlichen daraus besteht oder daraus besteht, welche mindestens 60 %, 70 %, 80 %, 90 %, 95 % oder 99 % Identität mit einer der SEQ ID NOs: 1, 2, 3, 5, 6, 8 und 9, vorzugsweise mit einer der SEQ ID NOs: 6 und 9, aufweist oder;

c) die TERT-codierende Sequenz eine Nukleotidsequenz umfasst, im Wesentlichen daraus besteht oder daraus besteht, deren Sequenz sich von der Sequenz einer Nukleotidsequenz von a) oder b) aufgrund von der Degeneration des genetischen Codes unterscheidet.

4. Rekombinantes adeno-assoziiertes virales Genom nach einem der Ansprüche 1-3, wobei das rekombinante virale Genom zusätzlich eine Kozak-Konsensussequenz umfasst, welche an die Nukleotidsequenz, welche eine TERT codiert, operativ verknüpft ist.

5. Rekombinantes adeno-assoziiertes virales Genom nach einem der Ansprüche 1-4, wobei das rekombinante virale Genom zusätzlich eine polyA-Sequenz umfasst, welche an die Nukleotidsequenz, welche eine TERT codiert, operativ verknüpft ist.

6. Adeno-assoziierter viraler Vektor umfassend ein rekombinantes adeno-assoziiertes virales Genom nach einem der Ansprüche 1 bis 5.

7. Adeno-assoziierter viraler Vektor nach Anspruch 6, wobei der virale Vektor des Serotyps 1, 2, 3, 4, 5, 6, 7, 8, 9, rh10, rh8, Cb4, rh74, DJ, 2/5, 2/1, 1/2 oder Anc80 ist.

8. Adeno-assoziierter viraler Vektor nach Anspruch 7, wobei der virale Vektor des Serotyps 6 oder 9 ist.

9. Adeno-assoziierter viraler Vektor nach Anspruch 8, wobei der virale Vektor des Serotyps 6 ist.

10. Adeno-assoziierter viraler Vektor nach Anspruch 9, wobei der virale Vektor des Serotyps 6 ist und wobei der gewebespezifische Promotor nach Anspruch 1 ist.

11. Pharmazeutische Zusammensetzung umfassend ein rekombinantes adeno-assoziiertes virales Genom nach einem der Ansprüche 1-5 oder einen adeno-assoziierten viralen Vektor nach den Ansprüchen 6 bis 10, wahlweise zusätzlich umfassend einen oder mehrere pharmazeutisch akzeptable Inhaltsstoffe.

12. Rekombinantes adeno-assoziiertes virales Genom nach einem der Ansprüche 1-5, adeno-assoziierter viraler Vektor nach einem der Ansprüche 6-10 oder pharmazeutische Zusammensetzung nach Anspruch 11, für deren Verwendung als Medikament.

13. Rekombinantes adeno-assoziiertes virales Genom nach einem der Ansprüche 1-5, adeno-assoziierter viraler Vektor nach den Ansprüchen 6-10 oder pharmazeutische Zusammensetzung nach Anspruch 11, für deren Verwendung bei der Behandlung und/oder Vorbeugung eines mit verkürzter Telomerlänge assoziierten Zustands.

14. Rekombinantes adeno-assoziiertes virales Genom nach einem der Ansprüche 1-5, adeno-assoziierter viraler Vektor nach den Ansprüchen 6-10 oder pharmazeutische Zusammensetzung nach Anspruch 11, für deren Verwendung bei der Behandlung und/oder Vorbeugung von Lungenfibrose.

15. Rekombinantes adeno-assoziiertes virales Genom nach einem der Ansprüche 1-5, adeno-assoziierter viraler Vektor nach den Ansprüchen 6-10 oder pharmazeutische Zusammensetzung nach Anspruch 11, für deren Verwendung bei der Behandlung und/oder Vorbeugung von Lungenfibrose mittels intratrachealer Verabreichung.

**Revendications**

1. Génome viral recombinant adéno-associé comprenant une séquence de nucléotides codant une télomérase transcriptase inverse (TERT) opérationnellement liée à un promoteur spécifique de tissu, dans lequel la longueur totale du génome viral est inférieure à 4700 nucléotides, et dans lequel le promoteur spécifique de tissu consiste en SEQ ID NO : 12.

2. Génome viral recombinant adéno-associé selon la revendication 1, dans lequel la séquence codant TERT est optimisé pour les codons, de préférence pour l'expression chez une cellule humaine.

3. Génome viral recombinant adéno-associé selon l'une quelconque des revendications 1 ou 2, dans lequel :

a) la séquence codant TERT comprend, consiste essentiellement en, ou consiste en, une séquence de nucléotides codant un polypeptide représenté par une séquence d'acides aminés ayant au moins 60 %, 70 %, 80 %, 90 %, 95 %, ou 99 % d'identité ou de similitude par rapport à l'une quelconque des SEQ ID NO : 4, 7, ou 10, de préférence par rapport à SEQ ID NO : 7 ou 10, ou ;

b) la séquence codant TERT comprend, consiste essentiellement en, ou consiste en, une séquence de nucléotides ayant au moins 60 %, 70 %, 80 %, 90 %, 95 %, ou 99 % d'identité par rapport à l'une quelconque des SEQ ID NO : 1, 2, 3, 5, 6, 8, et 9, de préférence par rapport à l'une quelconque des SEQ ID NO : 6 et 9 ou ;

c) la séquence codant TERT comprend, consiste essentiellement en, ou consiste en, une séquence de nucléotides dont la séquence diffère de la séquence d'une séquence de nucléotides de a) ou b) à cause de la dégénérescence du code génétique.

4. Génome viral recombinant adéno-associé selon l'une quelconque des revendications 1-3, dans lequel le génome viral recombinant comprend en outre une séquence consensus de Kozak opérationnellement liée à la séquence de nucléotides codant une TERT.

5. Génome viral recombinant adéno-associé selon l'une quelconque des revendications 1-4, dans lequel le génome viral recombinant comprend en outre une séquence de polyA opérationnellement liée à la séquence de nucléotides codant une TERT.

6. Vecteur viral adéno-associé comprenant un génome viral recombinant adéno-associé selon l'une quelconque des revendications 1 à 5.

7. Vecteur viral adéno-associé selon la revendication 6, dans lequel le vecteur viral est de sérotype 1, 2, 3, 4, 5, 6, 7, 8, 9, rh10, rh8, Cb4, rh74, DJ, 2/5, 2/1, 1/2 ou Anc80.

8. Vecteur viral adéno-associé selon la revendication 7, dans lequel le vecteur viral est de sérotype 6 ou 9.

9. Vecteur viral adéno-associé selon la revendication 8, dans lequel le vecteur viral est de sérotype 6.

10. Vecteur viral adéno-associé selon la revendication 9, dans lequel le vecteur viral est de sérotype 6 et dans lequel le promoteur spécifique de tissu est tel que défini dans la revendication 1.

11. Composition pharmaceutique comprenant un génome viral recombinant adéno-associé selon l'une quelconque des revendications 1-5 ou un vecteur viral adéno-associé selon les revendications 6 à 10, comprenant optionnellement en outre un ou plusieurs principes pharmaceutiquement acceptables.

12. Génome viral recombinant adéno-associé selon l'une quelconque des revendications 1-5, vecteur viral adéno-associé selon l'une quelconque des revendications 6-10, ou composition pharmaceutique selon la revendication 11, pour son utilisation comme médicament.

13. Génome viral recombinant adéno-associé selon l'une quelconque des revendications 1-5, vecteur viral adéno-associé selon les revendications 6-10, ou composition pharmaceutique selon la revendication 11, pour son utilisation dans le traitement et/ou la prévention d'une affection associée avec une longueur de télomère raccourcie.

14. Génome viral recombinant adéno-associé selon l'une quelconque des revendications 1-5, vecteur viral adéno-associé selon les revendications 6-10, ou composition pharmaceutique selon la revendication 11, pour son utilisation dans le traitement et/ou la prévention de la fibrose pulmonaire.

15. Génome viral recombinant adéno-associé selon l'une quelconque des revendications 1-5, vecteur viral adéno-associé selon les revendications 6-10, ou composition pharmaceutique selon la revendication 11, pour son utilisation dans le traitement et/ou la prévention de la fibrose pulmonaire par administration intratrachéale.

## Fig. 1A

## Fig. 1B

**FIG 1**

LUNG

BAT

HEART

LIVER

**FIG 2**

**FIG 3**

## Fig. 4A

□ AAV9-CMV-GFP 5E11

▨ AAV9-CMV-GFP 2E12

▫ AAV9-CMVenhancer-MLC2v-GFP 5e11

■ AAV9-CMVenhancer-MLC2v-GFP 2e12

## Fig. 4B

□ AAV9-CMV-GFP 5E11

▨ AAV9-CMV-GFP 2E12

▫ AAV9-CMVenhancer-MLC2v-GFP 5e11

■ AAV9-CMVenhancer-MLC2v-GFP 2e12

**FIG 4**

Fig. 5A

FIG 5

Fig. 5B

FIG 5 (CONT.)

EP 4 337 780 B1

**FIG 6**

EP 4 337 780 B1

FIG 7

74

## Fig. 8A    AAV9-CMV-mTERT

## Fig. 8B    AAV9-CMV-mTERT-DN

**FIG 8**

**Fig. 8C**

AAV9-Spb-moTERT

**Fig. 8D**

AAV9-CMVenh-MLC2v-moTERT

**FIG 8 (CONT.)**

Fig. 8E

AAV9-TNT-moTERT

FIG 8 (CONT.)

Fig. 9A

Fig. 9B

FIG 9

Fig. 10A

Fig. 10B

FIG 10

FIG 11

## Fig. 12A

## Fig. 12B

**FIG 12**

FIG 13

FIG 13 (CONT.)

**FIG 14**

BLEO (0.7 mg/Kg) : 48 mice (Deaths 4 instillation+ 11 Bleo)=33 mice
Bleo related deaths: 23%
PF CT-diagnosed: 24 (1 dead) (72%)
Initial affected lung volume by CT
greater than 25% of total lung volume: 7 (29 %)
AAV9 treatment: 24 mice (7 AAV9-null & 8 AAV9-CMV-TERT & 9 AAV9-SpB-TERT)
2 mice died during follow-up at 2 weeks post-AAV9 treatment and were not taken for CT rate calculations

## FIG 15

## FIG 16

**FIG 17**

**FIG 18**

FIG 19

FIG 20

FIG 21

**FIG 22**

Scale bar: 200 µm

FIG 23

AAV6 vs AAV9
CMV vs SpB
Viral doses (D1, D2, D3)
**Intratracheal instillation**
(12th-13th-14th April)

WT
9-10 weeks old males

weeks

1    2    3              9

Sacrifice
**3 weeks post-IT**
4th-7th May

Sacrifice
**9 weeks post-IT**
16th June

30μL of viral suspension intratracheally
administered to wild type mice
anesthetized with isoflurane.

*Viral doses:*
D1: $10^{11}$ vg/mouse
D2: $5 \times 10^{11}$ vg/mouse
D3: $10^{12}$ vg/mouse)

**Paraffin & -80°C samples:**
Lungs
Liver
Brain
Heart

## FIG 24

**FIG 25**

FIG 26

FIG 27

94

FIG 28

FIG 29

**FIG 30**

**FIG 31**

CC10-GFP   9 weeks        AAV6-SpB-GFP targets clara cells

FIG 33

FIG 34

**C**         3 weeks

**D**         9 weeks

**FIG 34 (CONT.)**

FIG 34 (CONT.)

**FIG 35**

C                       3 weeks

D                       9 weeks

**FIG 35 (CONT.)**

E

Brain

3 weeks

F

9 weeks

Brain

FIG 35 (CONT.)

**AAV6-SpB-mmoTERT-bGH**
*(Batch AAV-953)*

*Full vector* ← | Sample dialyzed

## FIG 36

## FIG 37

**FIG 38**

**FIG 39**

**FIG 40**

**FIG 41**

**FIG 42**

FIG 43

TRAP in livers

moTert expression in livers

Undetectable

**FIG 44**

**FIG 45**

**FIG 46**

**FIG 47**

FIG 48

FIG 49

FIG 49 (cont.)

FIG 50

FIG 51

FIG 52

FIG 53

**FIG 54**

| 260 nm | | 280 nm | | Assignment |
|---|---|---|---|---|
| S | % | S | % | |
| 87.3 | 7.9 | 86.8 | 7.3 | Intermediate species |
| 100.9 | 87.7 | 101.2 | 88.7 | Full particles |
| 119.8 | 4.4 | 115.5 | 4.0 | Aggregates |

**FIG 55**

**FIG 56**

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- WO 201620345 A
- WO 2016020346 A
- US 20010016352 A
- US 20140088175 A
- EP 2402038 A
- US 5139941 A
- US 5741683 A, Zhou X
- US 6057152 A, Samulski R
- US 6204059 B, Samulski R
- US 6268213 B, Samulski R
- US 6491907 B, Rabinowitz J
- US 6660514 B, Zolotukhin S
- US 6951753 B, Shenk T
- US 7094604 B, Snyder R
- US 7172893 B, Rabinowitz J
- US 7201898 B, Monahan P
- US 7229823 B, Samulski R
- US 7439065 B, Ferrari F
- WO 2016020345 A
- US 6165782 A
- US 6207455 B
- US 6218181 B
- US 6277633 B
- US 6323031 B
- US 6531456 B

## Non-patent literature cited in the description

- **BLACKBURN et al.** *Cell*, 2001, vol. 106 (6), 661-673
- **GREIDER ; BLACKBURN**. *Cell*, 1985, vol. 43, 405-413
- **BLASCO**. *Nat Chem Biol.*, 2007, vol. 3, 640-649
- **BERNARDES DE JESUS et al.** *EMBO Mol Med*, 2012, vol. 4, 691-704
- **BEIER et al.** *Blood*, 2012, vol. 120 (15), 2990-3000
- **POVEDANO et al.** *eLife*, 2018, vol. 7, e31299
- **BÄR et al.** *Nat Comm*, 2014, vol. 5, 5863
- **CHATTERJEE et al.** *Mol Ther*, 2021, vol. 29 (4), 1-16
- **AUSUBEL et al.** Current Protocols in Molecular Biology. John Wiley & Sons Inc, 2003
- **SAMBROOK ; GREEN**. Molecular Cloning. A Laboratory Manual. Cold Spring Harbor Laboratory Press, 2012
- **KUNKEL**. *Proc. Natl. Acad. Sci.*, 1985, vol. 82, 488
- **ROBERTS et al.** *Nature*, 1987, vol. 328, 731-734
- **WELLS, J.A. et al.** *Gene*, 1985, vol. 34, 315
- **AYUSO E et al.** *Curr. Gene Ther.*, 2010, vol. 10, 423-436
- **OKADA T et al.** *Hum. Gene Ther.*, 2009, vol. 20, 1013-1021
- **ZHANG H et al.** *Hum. Gene Ther.*, 2009, vol. 20, 922-929
- **VIRAG T et al.** *Hum. Gene Ther.*, 2009, vol. 20, 807-817
- **CHIORINI et al.** *J. of Virology*, 1999, vol. 73 (2), 1309-1319
- **METZGER et al.** *Nature*, 1988, vol. 334, 31-36
- **XIAO et al.** *J. Virol.*, 1996, vol. 70, 8098-8108
- Remington: The Science and Practice of Pharmacy. Elsevier, 2020
- **GONZALEZ-SUAREZ et al.** *EMBO J*, 2001, vol. 20 (11), 2619-30
- **CANELA et al.** *Methods Mol Biol*, 2007, vol. 371, 45-72
- **BÄR et al.** *Nat Comm*, 2018, vol. 5, 5863
- **XIA X.** Bioinformatics and the Cell: Modern Computational Approaches in Genomics, Proteomics and transcriptomics. Springer International Publishing, 2018
- **MOUNT D.** Bioinformatics: Sequence and Genome Analysis. Cold Spring Harbor Laboratory Press, 2004
- **HENIKOFF ; HENIKOFF**. *PNAS*, 1992, vol. 89, 915-919
- **ALTSCHUL et al.** *J. Mol. Biol.*, 1990, vol. 215, 403-10
- **ALTSCHUL et al.** *Nucleic Acids Res.*, 1997, vol. 25 (17), 3389-3402
- **ANDERSON**. *Nature*, 1998, vol. 392, 25-30
- **WALTHER ; STEIN**. *Drugs*, 2000, vol. 60, 249-71
- **KAY et al.** *Nat. Med.*, 2001, vol. 7, 33-40
- **RUSSELL**. *J. Gen. Virol.*, 2000, vol. 81, 2573-604
- **AMADO ; CHEN**. *Science*, 1999, vol. 285, 674-6

- **FEDERICO**. *Curr. Opin. Biotechnol.*, 1999, vol. 10, 448-53
- **VIGNA** ; **NALDINI**. *J. Gene Med.*, 2000, vol. 2, 308-16
- **MARIN et al.** *Mol. Med. Today*, 1997, vol. 3, 396-403
- **PENG** ; **RUSSELL**. *Curr. Opin. Biotechnol.*, 1999, vol. 10, 454-7
- **SOMMERFELT**. *J. Gen. Virol.*, 1999, vol. 80, 3049-64
- **REISER**. *Gene Ther.*, 2000, vol. 7, 910-3
- **BÄR et al.** *Nat. Comm.*, 2018, vol. 5, 5863
- **VAN LIESHOUT et al.** *Mol. Ther. Meth. Clin. Dev.*, 2018, 9
- **RUSSELL**. *J. Gen. Virol.*, 2000, vol. 81, 2573-2604
- **GONCALVES**. *Virol J.*, 2005, vol. 2 (1), 43
- **NIEMEYER et al.** *Blood.*, 22 January 2009, vol. 113 (4), 797-806
- **BUCHLIS, G. et al.** *Blood.*, 29 March 2012, vol. 119 (13), 3038-41
- **WANG et al.** *J Gene Med. March*, 2005, 9
- **MANDEL et al.** *Curr Opin Mol Ther.*, 2004, vol. 6 (5), 482-90
- **MARTIN et al.** *Eye*, 2004, vol. 18 (11), 1049-55
- **NATHWANI et al.** *N Engl J Med.*, 22 December 2011, vol. 365 (25), 2357-65
- **APPARAILLY et al.** *Hum Gene Ther.*, April 2005, vol. 16 (4), 426-34
- **FEDERICO**. *Curr Opin Biotechnol*, 1999, vol. 10, 448-53
- **VIGNA et al.** *J Gene Med*, 2000, vol. 2, 308-16
- **AYUSO, E. et al.** *Curr Gene Ther.*, 2010, vol. 10 (6), 423-36
- **BERNARDES DE JESUS, B. et al.** *EMBO Mol Med*, 2012, vol. 4 (8), 691-704
- **PFAFFL, M.** *Nucleic Acids Res.*, 2001, vol. 29 (9), e45
- **BURNHAM, B. et al.** *Hum Gene Ther Methods*, 2015, vol. 26 (6), 228-42
- **POVEDANO, J.M. et al.** *Elife*, 2018, vol. 7, e31299
- **BÄR, C. et al.** *Nat Comm*, 18 December 2014, vol. 5, 5863
- **POVEDANO**. *Cell Rep*, 2015, vol. 12, 286-299
- **HERBERT et al.** *Nat Protoc*, 2006, vol. 1, 583-1590
- **HERBERT et al.** *Nature protocols*, 2006
- **LIMBERIS, MP. et al.** *Proc Natl AcadSci U S A*, 29 August 2006, vol. 103 (35), 12993-8
- **LISA A SANTRY**. *BMC Biotechnol.*, 15 May 2017, vol. 17 (1), 43
- **LAURA P. VAN LIESHOUT**. *Methods Mol Biol.*, 2019, vol. 1950, 361-372
- **KATZ, MG et al.** *J Cardiovasc Dev Dis.*, 15 February 2019, vol. 6 (1), 8
- **KANG, M. et al.** *Nat Commun.*, 06 August 2020, vol. 11 (1), 3929
- **GUGGINO, BW. et al.** *Expert OpinBiolTher.*, October 2017, vol. 17 (10), 1265-1273
- **BLASCO et al.** *Cell*, 1997
- **HERBERT BS** ; **HOCHREITER AE** ; **WRIGHT WE** ; **SHAY JW**. Nonradioactive detection of telomerase activity using the telomeric repeat amplification protocol.. *Nat Protoc.*, 2006, vol. 1 (3), 1583-90
- **BLASCO MA** ; **LEE HW** ; **HANDE MP** ; **SAMPER E** ; **LANSDORP PM** ; **DEPINHO RA et al.** Telomere shortening and tumor formation by mouse cells lacking telomerase RNA.. *Cell*, 23 October 1997, vol. 91 (1), 25-34